# EUROPEAN PATENT SPECIFICATION

(11) **EP 4 271 268 B1**
(45) Date of publication and mention of the grant of the patent: **18.12.2024**
(21) Application number: 21798176.0
(22) Date of filing: 16.09.2021
(51) Int. Cl.: A61B 5/145

(54) **SYSTEMS, DEVICES, AND METHODS FOR ANALYTE MONITORING**
SYSTEME, VORRICHTUNGEN UND VERFAHREN ZUR ANALYTÜBERWACHUNG
SYSTÈMES, DISPOSITIFS ET PROCÉDÉS DE SURVEILLANCE D'ANALYTE

(30) Priority: 31.12.2020 US 202063132651 P
(43) Date of publication of application: 08.11.2023
(73) Proprietor: Abbott Diabetes Care Inc., Alameda, California 94502 (US)
(72) Inventor: RAO, Vivek S., Alameda, California 94502 (US); SIMMONS, Matthew, Pleasanton, California 94588 (US); PACE, Louis G., San Carlos, California 94070 (US); COLE, Jean-Pierre, Tracy, California 95304 (US); ROBINSON, Peter G., Alamo, California 94507 (US); VOIT, Peter, Dublin, California 94568 (US); MITCHELL, Steven T., Pleasant Hill, California 94523 (US)
(74) Representative: Booth, Catherine Louise
(86) International application number: PCT/US2021/050672
(87) International publication number: WO 2022/146505

(56) References cited:
- WO-A1-2019/236850
- WO-A1-2019/236876

## Description

### FIELD

The subject matter described herein relates generally to systems, devices, and methods for in vivo analyte monitoring.

### BACKGROUND

The detection and/or monitoring of analyte levels, such as glucose, ketones, lactate, oxygen, hemoglobin AIC, or the like, can be vitally important to the overall health of a person, particularly for an individual having diabetes. Patients suffering from diabetes mellitus can experience complications including loss of consciousness, cardiovascular disease, retinopathy, neuropathy, and nephropathy. Persons with diabetes are generally required to monitor their glucose levels to ensure that they are being maintained within a clinically safe range, and may also use this information to determine if and/or when insulin is needed to reduce glucose levels in their bodies, or when additional glucose is needed to raise the level of glucose in their bodies.

Growing clinical data demonstrates a strong correlation between the frequency of glucose monitoring and glycemic control. Despite such correlation, however, many individuals diagnosed with a diabetic condition do not monitor their glucose levels as frequently as they should due to a combination of factors including convenience, testing discretion, pain associated with glucose testing, and cost.

To increase patient adherence to a plan of frequent glucose monitoring, in vivo analyte monitoring systems can be utilized, in which a sensor control device may be worn on the body of an individual who requires analyte monitoring. To increase comfort and convenience for the individual, the sensor control device may have a small form-factor, and can be assembled and applied by the individual with a sensor applicator. The application process includes inserting a sensor that senses a user's analyte level in a bodily fluid located in the human body, using an applicator or insertion mechanism, such that the sensor comes into contact with a bodily fluid. The sensor control device may also be configured to transmit analyte data to another device, from which the individual or her health care provider ("HCP") can review the data and make therapy decisions. Furthermore, the benefits of analyte monitoring systems are not limited to persons with diabetes. For instance, analyte monitoring systems can provide useful information and insights to individuals interested in improving their health and wellness. As one example, to improve their sports performance, athletes can utilize a sensor control device worn on the body to collect data relating to one or more analytes such as, for example, glucose and/or lactate. WO 2019/236876 A1 and WO 2019/236850 A1 each disclose systems including a sensor applicator, a sensor control device arranged within the sensor applicator and including an electronics housing and a sensor extending from a bottom of the electronics housing, and a cap coupled to one of the sensor applicator and the sensor control device, wherein the cap is removable prior to deploying the sensor control device from the sensor applicator.

While current sensors can be convenient for users, they are also susceptible to malfunctions due to improper insertion. These malfunctions can be caused by user error, lack of proper training, poor user coordination, overly complicated procedures, and other issues. This can be particularly true for analyte monitoring systems having sensors used to measure an analyte level in an interstitial fluid ("ISF"), and which are inserted using sharps (also known as "introducers" or "needles"). Some prior art systems, for example, may rely too much on the precision assembly and deployment of a sensor control device and an applicator by the individual user. Other prior art systems may utilize sharp insertion and retraction mechanisms that are susceptible to premature withdrawal before the sensor can be properly implanted. In addition, some prior art systems may utilize sharps that are not optimally configured to create an insertion path without creating trauma to surrounding tissue. These challenges and others described herein can lead to improperly inserted or damaged sensors, and consequently, a failure to properly monitor the patient's analyte level.

Thus, a need exists for more reliable sensor insertion devices, systems and methods, that are easy to use by the patient and less prone to error. Additionally, a need exists for sensor insertion deices, systems, and methods that provide moisture control and extended shelf stability. Furthermore, a need exists for sensor control devices housing electronic circuitry to measure and transmit analyte data in a small form-factor.

### SUMMARY

The purpose and advantages of the disclosed subject matter will be set forth in and apparent from the description that follows, as well as will be learned by practice of the disclosed subject matter. Additional advantages of the disclosed subject matter will be realized and attained by the methods and systems particularly pointed out in the written description and claims hereof, as well as from the appended drawings.

To achieve these and other advantages and in accordance with the purpose of the disclosed subject matter, as embodied and broadly described, the disclosed subject matter is directed to an analyte measurement device. The analyte measurement device can include an electronics housing including a shell having an upper surface with a first aperture defined therein; a mount mated to the shell and having an underside with a second aperture defined therein aligned with the first aperture; a collar disposed between the shell and the mount and including: a third aperture aligned with the first aperture and the second aperture, and a plurality of tabs on an outer periphery of the collar; a circuit board disposed within the electronics housing and including a plurality of electronic modules, wherein the circuit board is mounted within the electronics housing on the plurality of tabs on the outer periphery of the collar, wherein the circuit board includes a first portion foldable over a second portion, the second portion mounted on the mount and the first portion mounted on the plurality of tabs on the outer periphery of the collar; and an analyte sensor including a tail portion extending through the first aperture and the second aperture and configured to measure an analyte level and a flag portion including a plurality of electrical contacts coupled with the circuit board, and a neck portion interconnecting the tail portion and the flag portion. The plurality of tabs can include three tabs.

The foldable circuit board can include a first portion foldable over a second portion, the second portion mounted on the mount and the first portion mounted on the plurality of tabs on the outer periphery. The circuit board can include a battery. The circuit board can include battery aperture to position the battery and to eliminate electrical interference between the battery and the circuit board.

The collar can include a collar channel on an upper surface mated with an annular ridge defined on an inner surface of the shell, and an annular lip on a bottom surface received within a channel defined on an inner surface of the mount, and a seal on the upper surface of the collar. The seal can be an elastomeric seal.

The analyte measurement device can include a sharp hub including a sharp extending through the first, second, and third apertures and a mating surface mated with the seal. The mating surface can include a ledge.

In accordance with the disclosed subject matter, an assembly for delivery of an analyte sensor is provided. The assembly can include a sensor control device including: an electronics housing including a shell and a mount mounted to the shell; a circuit board disposed within the electronics housing and including a plurality of electronic modules; an analyte sensor including a tail portion extending through an aperture in the mount and configured to measure an analyte level and a flag portion including a plurality of electrical contacts connected to the circuit board, and a neck portion interconnecting the tail portion and the flag portion; a sensor cap having a first end, a second end, and an inner chamber, wherein the inner chamber receives the tail portion of the analyte sensor and wherein the first end is removably coupled to the mount to seal the inner chamber; and an applicator for delivery of the analyte sensor including: a housing defining an interior space; a sensor carrier within the interior space and releasably securing the sensor control device within an interior of the applicator; an applicator cap removably coupled to the housing and including: a desiccant including a plurality of retention snaps, one or more retention clips engaged to the plurality of retention snaps to retain the desiccant in the cap and to limit rotation of the desiccant, and a cap post engaged with an engagement feature on the second end of the sensor cap.

The desiccant can include an internal bore and the engagement feature on the second end of the sensor cap passes through the internal bore of the desiccant and engages the applicator cap. The desiccant can be substantially cylindrical in shape.

The sensor control device can comprise an elastomeric plug arranged at the second end of the sensor cap. The elastomeric plug can include a seal bead in an interference fit arrangement with the sensor cap. The elastomeric plug can define the engagement feature of the sensor cap.

The applicator for delivery of the analyte sensor can include a sharp carrier coupled to a sharp extending through the aperture in the mount, the sharp configured to penetrate a user's skin to position the analyte sensor into contact with bodily fluid, a sensor carrier to releasably hold the sensor control device within the interior space, and a spring biasing the sharp carrier to move upward within the housing.

The spring can include a spring having a spring constant of about 0.12. The spring can have a wire diameter of about 0.65 millimeters, an inner diameter of about 9.6 millimeter, and an outer diameter of about 11.1 millimeters. The spring can have a maximum solid strength of about 11 millimeters.

### BRIEF DESCRIPTION OF THE FIGURES

The details of the subject matter set forth herein, both as to its structure and operation, may be apparent by study of the accompanying figures, in which like reference numerals refer to like parts. The components in the figures are not necessarily to scale, emphasis instead being placed upon illustrating the principles of the subject matter. Moreover, all illustrations are intended to convey concepts, where relative sizes, shapes and other detailed attributes may be illustrated schematically rather than literally or precisely.
FIG. 1 is a system overview of a sensor applicator, reader device, monitoring system, network, and remote system.
FIG. 2A is a block diagram depicting an example embodiment of a reader device.
FIGS. 2B and 2C are block diagrams depicting example embodiments of sensor control devices.
FIG. 3A is a proximal perspective view depicting an example embodiment of a user preparing a tray for an assembly.
FIG. 3B is a side view depicting an example embodiment of a user preparing an applicator device for an assembly.
FIG. 3C is a proximal perspective view depicting an example embodiment of a user inserting an applicator device into a tray during an assembly.
FIG. 3D is a proximal perspective view depicting an example embodiment of a user removing an applicator device from a tray during an assembly.
FIG. 3E is a proximal perspective view depicting an example embodiment of a patient applying a sensor using an applicator device.
FIG. 3F is a proximal perspective view depicting an example embodiment of a patient with an applied sensor and a used applicator device.
FIG. 4A is a side view depicting an example embodiment of an applicator device coupled with a cap.
FIG. 4B is a side perspective view depicting an example embodiment of an applicator device and cap decoupled.
FIG. 4C is a perspective view depicting an example embodiment of a distal end of an applicator device and electronics housing.
FIG. 4D is a top perspective view of an exemplary applicator device in accordance with the disclosed subject matter.
FIG. 4E is a bottom perspective view of the applicator device of FIG. 4D.
FIG. 4F is an exploded view of the applicator device of FIG. 4D.
FIG. 4G is a side cutaway view of the applicator device of FIG. 4D.
FIG. 5 is a proximal perspective view depicting an example embodiment of a tray with sterilization lid coupled.
FIG. 6A is a proximal perspective cutaway view depicting an example embodiment of a tray with sensor delivery components.
FIG. 6B is a proximal perspective view depicting sensor delivery components.
FIG. 7A is side view depicting an example embodiment of a housing.
FIG. 7B is a perspective view depicting an example embodiment of a distal end of a housing.
FIG. 7C is a side cross-sectional view depicting an example embodiment of a housing.
FIGS. 7D and 7E are side cross-sectional views depicting a locking rib portion of an example embodiment of a housing with a portion of a sheath.
FIGS. 7F and 7G are side cross-sectional views depicting a locking rib portion of another example embodiment of a housing and a portion of a sheath.
FIG. 7H is a side cross-sectional view depicting a locking rib portion of another example embodiment of a housing and a portion of a sheath.
FIG. 7I is a side cross-sectional view depicting a locking rib portion of another example embodiment of a housing and a portion of a sheath.
FIG. 7J is a side view of an exemplary housing in accordance with the disclosed subject matter.
FIG. 7K is a bottom perspective view of the housing of FIG. 7J.
FIG. 7L is a side cutaway view of the housing of FIG. 7J.
FIG. 7M is bottom perspective view of a cap in accordance with the disclosed subject matter.
FIG. 7N is a side cutaway view of the cap of FIG. 7M.
FIG. 7O is a top view of the cap of FIG. 7M.
FIG. 7P is a cutaway view of a desiccant in a cap in accordance with the disclosed subject matter.
FIG. 7Q-R are bottom and top perspective views of an exemplary desiccant in accordance with the disclosed subject matter.
FIGS. 7S-T are enlarged cross-sectional side views of the interface between the housing and cap in accordance with the disclosed subject matter.
FIGS. 7U-V are enlarged cross-sectional side views of the housing and cap, respectively, in accordance with the disclosed subject matter.
FIGS. 7W-X are side cutaway views of the cap of FIG. 7M.
FIG. 7Y is a top perspective view of an exemplary elastomeric plug, according to one or more embodiments.
FIG. 7Z is a partial cross-sectional perspective view of an exemplary sensor cap and elastomeric plug, according to one or more embodiments.
FIG. 8A is a side view depicting an example embodiment of a sheath.
FIG. 8B is a perspective view depicting an example embodiment of a proximal end of a sheath.
FIG. 8C is a close-up perspective view depicting an example embodiment of a distal side of a detent snap of a sheath.
FIG. 8D is a side view depicting an example embodiment of features of a sheath.
FIG. 8E is an end view of an example embodiment of a proximal end of a sheath.
FIGS. 8F to 8H are perspective views depicting another example embodiment of a sheath in various stages of assembly with other applicator components.
FIG. 8I is a side view of a sheath in accordance with the disclosed subject matter.
FIG. 8J is a close-up view of a detent snap of the sheath of FIG. 8I.
FIG. 8K is a top view of the sheath of FIG. 8I.
FIG. 8L is a perspective view of the sheath of FIG. 8I.
FIG. 8M is a side cutaway view of the sheath of FIG. 8I.
FIG. 8N is a close-up view a lock arm of the sheath of FIG. 8I and the lock arm's engagement with a cap and a sensor carrier, in accordance with the disclosed subject matter.
FIG. 8O is a close-up view of a rib of the sheath of FIG. 8I and the rib's engagement with a sensor carrier, in accordance with the disclosed subject matter.
FIG. 9A is a proximal perspective view depicting an example embodiment of a sensor carri er.
FIG. 9B is a distal perspective view depicting an example embodiment of a sensor carri er.
FIG. 9C is a distal perspective view depicting another example embodiment of a sensor carrier.
FIG. 9D is a top perspective view of a sensor carrier in accordance with the disclosed subject matter.
FIG. 9E is a bottom view of the sensor carrier of FIG. 9D.
FIG. 10A is a perspective view of a sharp carrier in accordance with the disclosed subject matter.
FIG. 10B is a side cutaway view of the sharp carrier of FIG. 10A.
FIG. 10C is a perspective view of a sharp carrier in accordance with the disclosed subject matter.
FIG. 10D is a side cutaway view of the sharp carrier of FIG. 10C.
FIGS. 11A to 11B are top and bottom perspective views, respectively, depicting an example embodiment of a sensor module.
FIGS. 12A and 12B are perspective and compressed views, respectively, depicting an example embodiment of a sensor connector.
FIG. 13 is a perspective view depicting an example embodiment of a sensor.
FIGS. 14A and 14B are bottom and top perspective views, respectively, of an example embodiment of a sensor module assembly.
FIGS. 15A and 15B are close-up partial views of an example embodiment of a sensor module assembly.
FIGS. 15C-G are side views of exemplary sensors, according to one or more embodiments of the disclosure.
FIGS. 16A and 16B are isometric and partially exploded isometric views of an example connector assembly, according to one or more embodiments.
FIG. 16C is an isometric bottom view of the connector of FIGS. 16A-16B.
FIGS. 16D and 16E are isometric and partially exploded isometric views of another example connector assembly, according to one or more embodiments.
FIG. 16F is an isometric bottom view of the connector of FIGS. 16D-16E.
FIG. 17A is a perspective view depicting an example embodiment of a sharp module.
FIG. 17B is a perspective view of another example embodiment of a sharp module.
FIGS. 17C and 17D are schematic views depicting the sharp module of FIG. 17B.
FIGS. 17E and 17F are a side schematic view and a top-down schematic view, respectively, of the sharp module of FIG. 17B, as assembled with a sensor module.
FIG. 17G is a perspective view of another example embodiment of a sharp module.
FIG. 17H is a side schematic view depicting the sharp module of FIG. 17G.
FIGS. 17I and 17J are a side cross-sectional view and a side view, respectively, of the sharp module of FIG. 17G, as assembled with a sensor module.
FIGS. 18A and 18B are isometric and side views, respectively, of another example sensor control device.
FIGS. 19A and 19B are exploded isometric top and bottom views, respectively of the sensor control device of FIGS. 18A-18B.
FIG. 19C is a top perspective view of a cell battery in accordance with the disclosed subject matter.
FIG. 20 is a cross-sectional side view of an assembled sealed subassembly, according to one or more embodiments.
FIGS. 21A-21C are progressive cross-sectional side views showing assembly of the sensor applicator with the sensor control device of FIGS. 18A-18B.
FIGS. 22A and 22B are perspective and top views, respectively, of the cap post of FIG. 21C, according to one or more additional embodiments.
FIG. 23 is a cross-sectional side view of the sensor control device of FIGS. 18A- 18B.
FIGS. 24A and 24B are cross-sectional side views of the sensor applicator ready to deploy the sensor control device to a target monitoring location.
FIGS. 25A-25C are progressive cross-sectional side views showing assembly and disassembly of an example embodiment of the sensor applicator with the sensor control device of FIGS. 18A-18B.
FIG. 26A is an isometric bottom view of the housing, according to one or more embodiments.
FIG. 27A is an isometric bottom view of the housing with the sheath and other components at least partially positioned therein.
FIG. 28 is an enlarged cross-sectional side view of the sensor applicator with the sensor control device installed therein, according to one or more embodiments.
FIG. 29A is an isometric top view of the cap, according to one or more embodiments.
FIG. 29B is an enlarged cross-sectional view of the engagement between the cap and the housing, according to one or more embodiments.
FIGS. 30A and 30B are isometric views of the sensor cap and the collar, respectively, according to one or more embodiments.
FIGS. 31A and 31B are side and isometric views, respectively, of an example sensor control device, according to one or more embodiments of the present disclosure.
FIGS. 32A and 32B are exploded, isometric top and bottom views, respectively, of the sensor control device of FIG. 2, according to one or more embodiments.
FIG. 32C is a top perspective view of the collar of FIG. 32A-B, according to one or more embodiments.
FIG. 32D-E are isometric top views of the sensor control device of FIG. 2 according to one or more embodiments.
FIG. 33 is a cross-sectional side view of the sensor control device of FIGS. 31A- 31B and 32A-32B, according to one or more embodiments.
FIG. 33A is an exploded isometric view of a portion of another embodiment of the sensor control device of FIGS. 31A-31B and 32A-32B.
FIG. 33B is a cross-sectional view of the sharp hub and the mount of FIG. 33A, according to one or more embodiments.
FIG. 34A is an isometric bottom view of the mount of FIGS. 31A-31B and 32A-32B.
FIG. 34B is an isometric top view of the sensor cap of FIGS. 31A-31B and 32A-32B.
FIGS. 35A and 35B are side and cross-sectional side views, respectively, of an example sensor applicator, according to one or more embodiments.
FIGS. 36A and 36B are perspective and top views, respectively, of the cap post of FIG. 35B, according to one or more embodiments.
FIG. 37 is a cross-sectional side view of the sensor control device positioned within the applicator cap, according to one or more embodiments.
FIG. 38A is a cross-sectional view of a sensor control device showing example interaction between the sensor and the sharp.
FIG. 38B is a side cross-sectional view of a sharp hub, sharp, and sensor, with the sensor in an unbiased position, in accordance with the disclosed subject matter.
FIG. 38C is a side cross-sectional view of a sharp hub, sharp, and sensor, with the sensor in a biased position, in accordance with the disclosed subject matter.
FIG. 38D is a closeup of a portion of a sharp in accordance with the disclosed subject matter.
FIGS. 39A-39F illustrate cross-sectional views depicting an example embodiment of an applicator during a stage of deployment.

### DETAILED DESCRIPTION

Before the present subject matter is described in detail, it is to be understood that this disclosure is not limited to the particular embodiments described, as such may, of course, vary. It is also to be understood that the terminology used herein is for the purpose of describing particular embodiments only, and is not intended to be limiting, since the scope of the present disclosure will be limited only by the appended claims.

As used herein and in the appended claims, the singular forms "a," "an," and "the" include plural referents unless the context clearly dictates otherwise.

The publications discussed herein are provided solely for their disclosure prior to the filing date of the present application. Nothing herein is to be construed as an admission that the present disclosure is not entitled to antedate such publication by virtue of prior disclosure. Further, the dates of publication provided may be different from the actual publication dates which may need to be independently confirmed.

Generally, embodiments of the present disclosure include systems, devices, and methods for the use of analyte sensor insertion applicators for use with in vivo analyte monitoring systems. An applicator can be provided to the user in a sterile package with an electronics housing of the sensor control device contained therein. According to some embodiments, a structure separate from the applicator, such as a container, can also be provided to the user as a sterile package with a sensor module and a sharp module contained therein. The user can couple the sensor module to the electronics housing, and can couple the sharp to the applicator with an assembly process that involves the insertion of the applicator into the container in a specified manner. In other embodiments, the applicator, sensor control device, sensor module, and sharp module can be provided in a single package. The applicator can be used to position the sensor control device on a human body with a sensor in contact with the wearer's bodily fluid. The embodiments provided herein are improvements to reduce the likelihood that a sensor is improperly inserted or damaged, or elicits an adverse physiological response. Other improvements and advantages are provided as well. The various configurations of these devices are described in detail by way of the embodiments which are only examples.

Furthermore, many embodiments include in vivo analyte sensors structurally configured so that at least a portion of the sensor is, or can be, positioned in the body of a user to obtain information about at least one analyte of the body. It should be noted, however, that the embodiments disclosed herein can be used with in vivo analyte monitoring systems that incorporate in vitro capability, as well as purely in vitro or ex vivo analyte monitoring systems, including systems that are entirely non-invasive.

Furthermore, for each and every embodiment of a method disclosed herein, systems and devices capable of performing each of those embodiments are covered within the scope of the present disclosure. For example, embodiments of sensor control devices are disclosed and these devices can have one or more sensors, analyte monitoring circuits (e.g., an analog circuit), memories (e.g., for storing instructions), power sources, communication circuits, transmitters, receivers, processors and/or controllers (e.g., for executing instructions) that can perform any and all method steps or facilitate the execution of any and all method steps. These sensor control device embodiments can be used and can be capable of use to implement those steps performed by a sensor control device from any and all of the methods described herein.

As mentioned, a number of embodiments of systems, devices, and methods are described herein that provide for the improved assembly and use of sensor insertion devices for use with in vivo analyte monitoring systems. In particular, several embodiments of the present disclosure are designed to improve the method of sensor insertion with respect to in vivo analyte monitoring systems and, in particular, to prevent the premature retraction of an insertion sharp during a sensor insertion process. Some embodiments, for example, include a sensor insertion mechanism with an increased firing velocity and a delayed sharp retraction. In other embodiments, the sharp retraction mechanism can be motion-actuated such that the sharp is not retracted until the user pulls the applicator away from the skin. Consequently, these embodiments can reduce the likelihood of prematurely withdrawing an insertion sharp during a sensor insertion process; decrease the likelihood of improper sensor insertion; and decrease the likelihood of damaging a sensor during the sensor insertion process, to name a few advantages. Several embodiments of the present disclosure also provide for improved insertion sharp modules. In addition, several embodiments of the present disclosure are designed to prevent undesirable axial and/or rotational movement of applicator components during sensor insertion. Accordingly, these embodiments can reduce the likelihood of instability of a positioned sensor, irritation at the insertion site, and damage to surrounding tissue, to name a few advantages. In addition, to mitigate inaccurate sensor readings which can be caused by trauma at the insertion site, several embodiments of the present disclosure can reduce the end-depth penetration of the needle relative to the sensor tip during insertion.

Before describing these aspects of the embodiments in detail, however, it is first desirable to describe examples of devices that can be present within, for example, an in vivo analyte monitoring system, as well as examples of their operation, all of which can be used with the embodiments described herein.

There are various types of in vivo analyte monitoring systems. "Continuous Analyte Monitoring" systems (or "Continuous Glucose Monitoring" systems), for example, can transmit data from a sensor control device to a reader device continuously without prompting, e.g., automatically according to a schedule. "Flash Analyte Monitoring" systems (or "Flash Glucose Monitoring" systems or simply "Flash" systems), as another example, can transfer data from a sensor control device in response to a scan or request for data by a reader device, such as with a Near Field Communication (NFC) or Radio Frequency Identification (RFID) protocol. In vivo analyte monitoring systems can also operate without the need for finger stick calibration.

In vivo analyte monitoring systems can be differentiated from "in vitro" systems that contact a biological sample outside of the body (or "ex vivo") and that typically include a meter device that has a port for receiving an analyte test strip carrying bodily fluid of the user, which can be analyzed to determine the user's blood sugar level.

In vivo monitoring systems can include a sensor that, while positioned in vivo, makes contact with the bodily fluid of the user and senses the analyte levels contained therein. The sensor can be part of the sensor control device that resides on the body of the user and contains the electronics and power supply that enable and control the analyte sensing. The sensor control device, and variations thereof, can also be referred to as a "sensor control unit," an "on-body electronics" device or unit, an "on-body" device or unit, or a "sensor data communication" device or unit, to name a few.

In vivo monitoring systems can also include a device that receives sensed analyte data from the sensor control device and processes and/or displays that sensed analyte data, in any number of forms, to the user. This device, and variations thereof, can be referred to as a "handheld reader device," "reader device" (or simply a "reader"), "handheld electronics" (or simply a "handheld"), a "portable data processing" device or unit, a "data receiver," a "receiver" device or unit (or simply a "receiver"), or a "remote" device or unit, to name a few. Other devices such as personal computers have also been utilized with or incorporated into in vivo and in vitro monitoring systems.

### Exemplary In Vivo Analyte Monitoring System

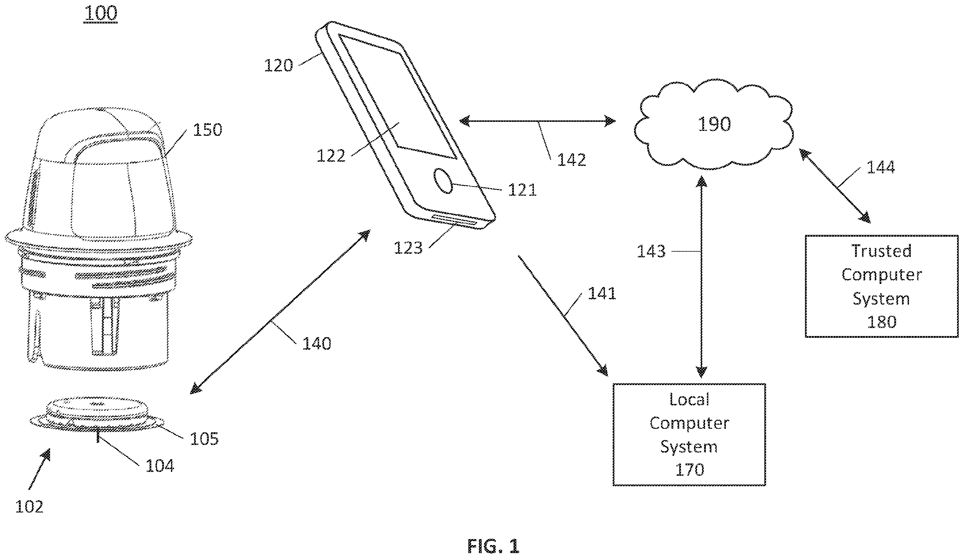

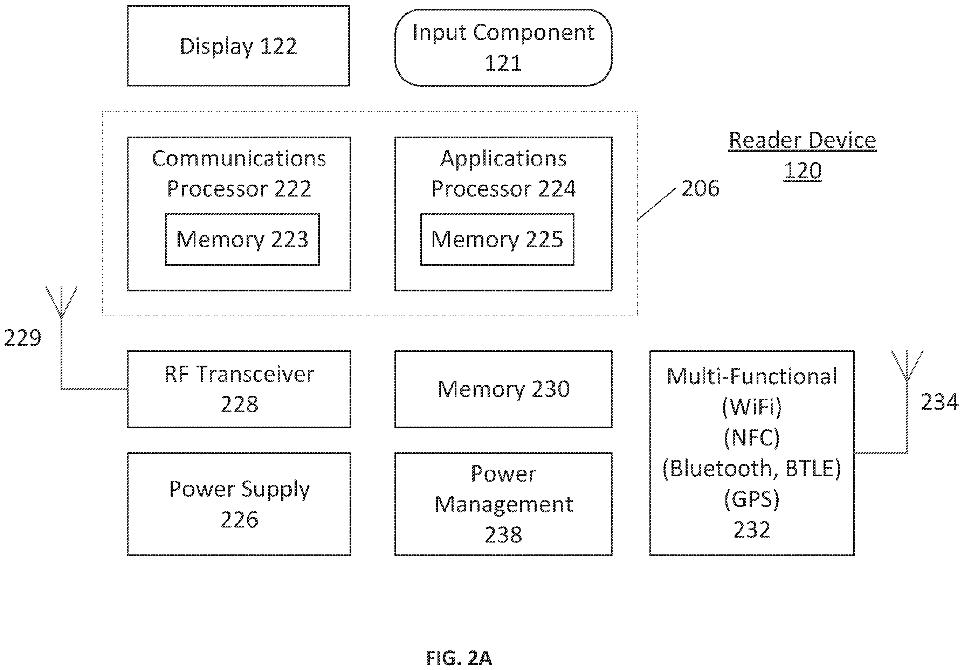

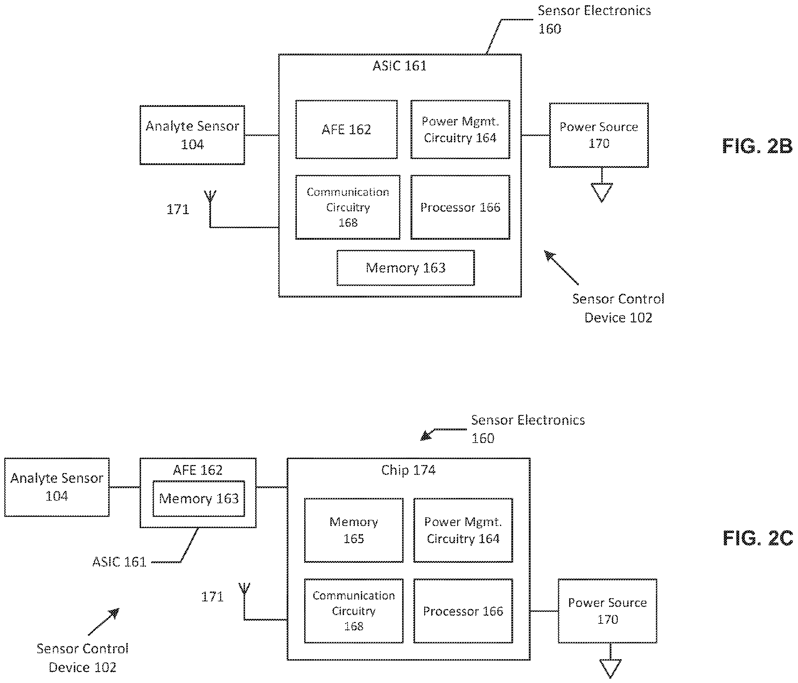

FIG. 1 is a conceptual diagram depicting an example embodiment of an analyte monitoring system 100 that includes a sensor applicator 150, a sensor control device 102, and a reader device 120. Here, sensor applicator 150 can be used to deliver sensor control device 102 to a monitoring location on a user's skin where a sensor 104 is maintained in position for a period of time by an adhesive patch 105. Sensor control device 102 is further described in FIGS. 2B and 2C, and can communicate with reader device 120 via a communication path 140 using a wired or wireless technique. Example wireless protocols include Bluetooth, Bluetooth Low Energy (BLE, BTLE, Bluetooth SMART, etc.), Near Field Communication (NFC) and others. Users can monitor applications installed in memory on reader device 120 using screen 122 and input 121 and the device battery can be recharged using power port 123. More detail about reader device 120 is set forth with respect to FIG. 2A below. Reader device 120 can communicate with local computer system 170 via a communication path 141 using a wired or wireless technique. Local computer system 170 can include one or more of a laptop, desktop, tablet, phablet, smartphone, set-top box, video game console, or other computing device and wireless communication can include any of a number of applicable wireless networking protocols including Bluetooth, Bluetooth Low Energy (BTLE), Wi-Fi or others. Local computer system 170 can communicate via communications path 143 with a network 190 similar to how reader device 120 can communicate via a communications path 142 with network 190, by wired or wireless technique as described previously. Network 190 can be any of a number of networks, such as private networks and public networks, local area or wide area networks, and so forth. A trusted computer system 180 can include a server and can provide authentication services and secured data storage and can communicate via communications path 144 with network 190 by wired or wireless technique.

### Exemplary Reader Device

FIG. 2A is a block diagram depicting an example embodiment of a reader device configured as a smartphone. Here, reader device 120 can include a display 122, input component 121, and a processing core 206 including a communications processor 222 coupled with memory 223 and an applications processor 224 coupled with memory 225. Also included can be separate memory 230, RF transceiver 228 with antenna 229, and power supply 226 with power management module 238. Further included can be a multi-functional transceiver 232 which can communicate over Wi-Fi, NFC, Bluetooth, BTLE, and GPS with an antenna 234. As understood by one of skill in the art, these components are electrically and communicatively coupled in a manner to make a functional device.

### Exemplary Sensor Control Devices

FIGS. 2B and 2C are block diagrams depicting example embodiments of sensor control device 102 having analyte sensor 104 and sensor electronics 160 (including analyte monitoring circuitry) that can have the majority of the processing capability for rendering end-result data suitable for display to the user. In FIG. 2B, a single semiconductor chip 161 is depicted that can be a custom application specific integrated circuit (ASIC). Shown within ASIC 161 are certain high-level functional units, including an analog front end (AFE) 162, power management (or control) circuitry 164, processor 166, and communication circuitry 168 (which can be implemented as a transmitter, receiver, transceiver, passive circuit, or otherwise according to the communication protocol). In this embodiment, both AFE 162 and processor 166 are used as analyte monitoring circuitry, but in other embodiments either circuit can perform the analyte monitoring function. Processor 166 can include one or more processors, microprocessors, controllers, and/or microcontrollers, each of which can be a discrete chip or distributed amongst (and a portion of) a number of different chips.

A memory 163 is also included within ASIC 161 and can be shared by the various functional units present within ASIC 161, or can be distributed amongst two or more of them. Memory 163 can also be a separate chip. Memory 163 can be volatile and/or non-volatile memory. In this embodiment, ASIC 161 is coupled with power source 170, which can be a coin cell battery, or the like. AFE 162 interfaces with in vivo analyte sensor 104 and receives measurement data therefrom and outputs the data to processor 166 in digital form, which in turn processes the data to arrive at the end-result glucose discrete and trend values, etc. This data can then be provided to communication circuitry 168 for sending, by way of antenna 171, to reader device 120 (not shown), for example, where minimal further processing is needed by the resident software application to display the data.

FIG. 2C is similar to FIG. 2B but instead includes two discrete semiconductor chips 162 and 174, which can be packaged together or separately. Here, AFE 162 is resident on ASIC 161. Processor 166 is integrated with power management circuitry 164 and communication circuitry 168 on chip 174. AFE 162 includes memory 163 and chip 174 includes memory 165, which can be isolated or distributed within. In one example embodiment, AFE 162 is combined with power management circuitry 164 and processor 166 on one chip, while communication circuitry 168 is on a separate chip. In another example embodiment, both AFE 162 and communication circuitry 168 are on one chip, and processor 166 and power management circuitry 164 are on another chip. It should be noted that other chip combinations are possible, including three or more chips, each bearing responsibility for the separate functions described, or sharing one or more functions for fail-safe redundancy.

### Exemplary Assembly Processes for Sensor Control Devices

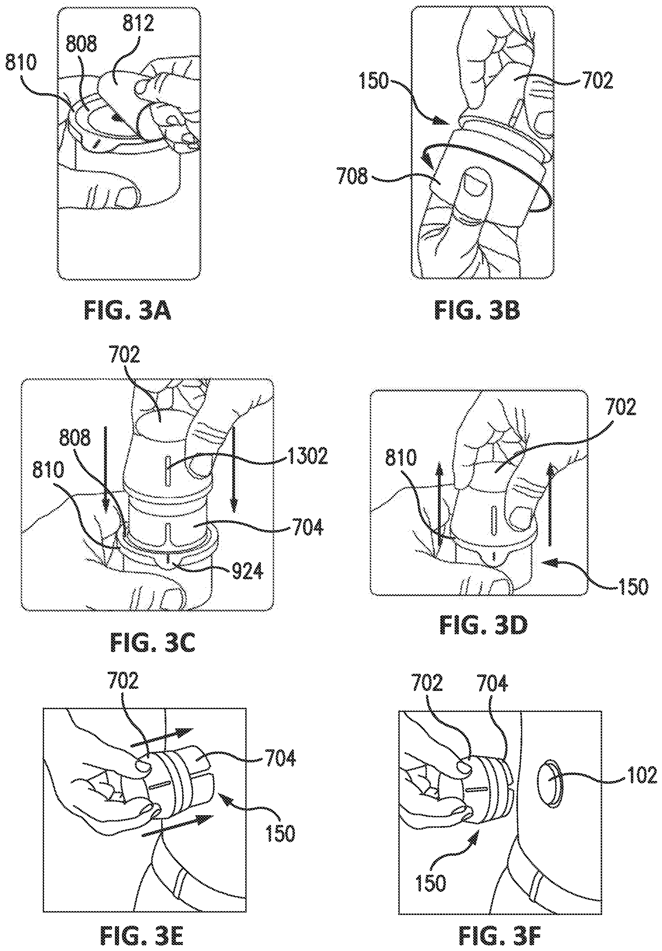

The components of sensor control device 102 can be acquired by a user in multiple packages requiring final assembly by the user before delivery to an appropriate user location. FIGS. 3A-3D depict an example embodiment of an assembly process for sensor control device 102 by a user, including preparation of separate components before coupling the components in order to ready the sensor for delivery. FIGS. 3E-3F depict an example embodiment of delivery of sensor control device 102 to an appropriate user location by selecting the appropriate delivery location and applying device 102 to the location.

FIG. 3A is a proximal perspective view depicting an example embodiment of a user preparing a container 810, configured here as a tray (although other packages can be used), for an assembly process. The user can accomplish this preparation by removing lid 812 from tray 810 to expose platform 808, for instance by peeling a non-adhered portion of lid 812 away from tray 810 such that adhered portions of lid 812 are removed. Removal of lid 812 can be appropriate in various embodiments so long as platform 808 is adequately exposed within tray 810. Lid 812 can then be placed aside.

FIG. 3B is a side view depicting an example embodiment of a user preparing an applicator device 150 for assembly. Applicator device 150 can be provided in a sterile package sealed by a cap 708. Preparation of applicator device 150 can include uncoupling housing 702 from cap 708 to expose sheath 704 (FIG. 3C). This can be accomplished by unscrewing (or otherwise uncoupling) cap 708 from housing 702. Cap 708 can then be placed aside.

FIG. 3C is a proximal perspective view depicting an example embodiment of a user inserting an applicator device 150 into a tray 810 during an assembly. Initially, the user can insert sheath 704 into platform 808 inside tray 810 after aligning housing orienting feature 1302 (or slot or recess) and tray orienting feature 924 (an abutment or detent). Inserting sheath 704 into platform 808 temporarily unlocks sheath 704 relative to housing 702 and also temporarily unlocks platform 808 relative to tray 810. At this stage, removal of applicator device 150 from tray 810 will result in the same state prior to initial insertion of applicator device 150 into tray 810 (i.e., the process can be reversed or aborted at this point and then repeated without consequence).

Sheath 704 can maintain position within platform 808 with respect to housing 702 while housing 702 is distally advanced, coupling with platform 808 to distally advance platform 808 with respect to tray 810. This step unlocks and collapses platform 808 within tray 810. Sheath 704 can contact and disengage locking features (not shown) within tray 810 that unlock sheath 704 with respect to housing 702 and prevent sheath 704 from moving (relatively) while housing 702 continues to distally advance platform 808. At the end of advancement of housing 702 and platform 808, sheath 704 is permanently unlocked relative to housing 702. A sharp and sensor (not shown) within tray 810 can be coupled with an electronics housing (not shown) within housing 702 at the end of the distal advancement of housing 702. Operation and interaction of the applicator device 150 and tray 810 are further described below.

FIG. 3D is a proximal perspective view depicting an example embodiment of a user removing an applicator device 150 from a tray 810 during an assembly. A user can remove applicator 150 from tray 810 by proximally advancing housing 702 with respect to tray 810 or other motions having the same end effect of uncoupling applicator 150 and tray 810. The applicator device 150 is removed with sensor control device 102 (not shown) fully assembled (sharp, sensor, electronics) therein and positioned for delivery.

FIG. 3E is a proximal perspective view depicting an example embodiment of a patient applying sensor control device 102 using applicator device 150 to a target area of skin, for instance, on an abdomen or other appropriate location. Advancing housing 702 distally collapses sheath 704 within housing 702 and applies the sensor to the target location such that an adhesive layer on the bottom side of sensor control device 102 adheres to the skin. The sharp is automatically retracted when housing 702 is fully advanced, while the sensor (not shown) is left in position to measure analyte levels.

FIG. 3F is a proximal perspective view depicting an example embodiment of a patient with sensor control device 102 in an applied position. The user can then remove applicator 150 from the application site.

System 100, described with respect to FIGS. 3A-3F and elsewhere herein, can provide a reduced or eliminated chance of accidental breakage, permanent deformation, or incorrect assembly of applicator components compared to prior art systems. Since applicator housing 702 directly engages platform 808 while sheath 704 unlocks, rather than indirect engagement via sheath 704, relative angularity between sheath 704 and housing 702 will not result in breakage or permanent deformation of the arms or other components. The potential for relatively high forces (such as in conventional devices) during assembly will be reduced, which in turn reduces the chance of unsuccessful user assembly.

### Exemplary Sensor Applicator Devices

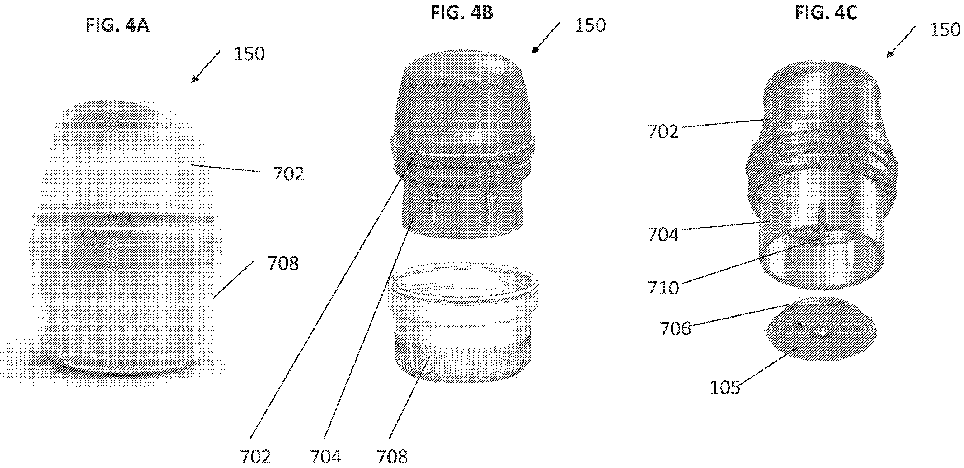

FIG. 4A is a side view depicting an example embodiment of an applicator device 150 coupled with screw cap 708. This is an example of how applicator 150 is shipped to and received by a user, prior to assembly by the user with a sensor. FIG. 4B is a side perspective view depicting applicator 150 and cap 708 after being decoupled. FIG. 4C is a perspective view depicting an example embodiment of a distal end of an applicator device 150 with electronics housing 706 and adhesive patch 105 removed from the position they would have retained within sensor carrier 710 of sheath 704, when cap 708 is in place.

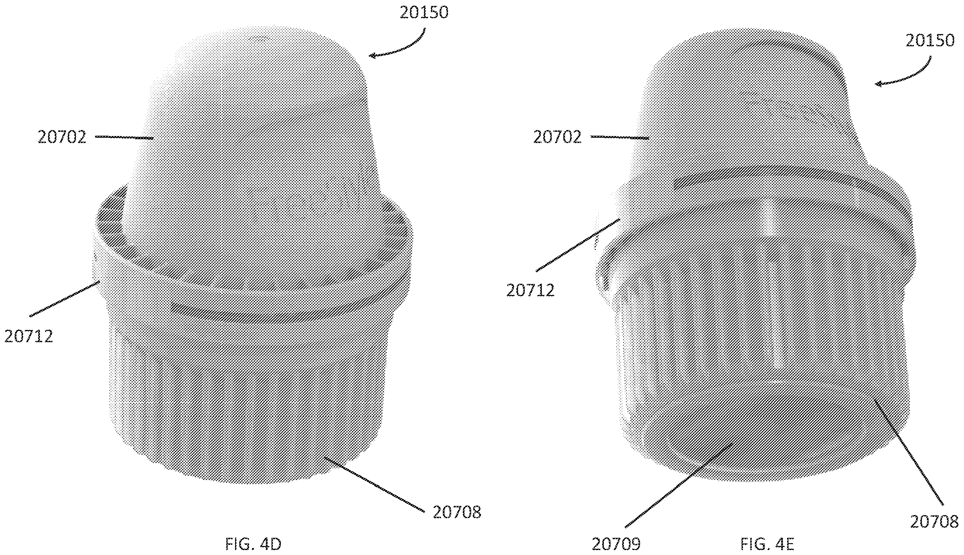

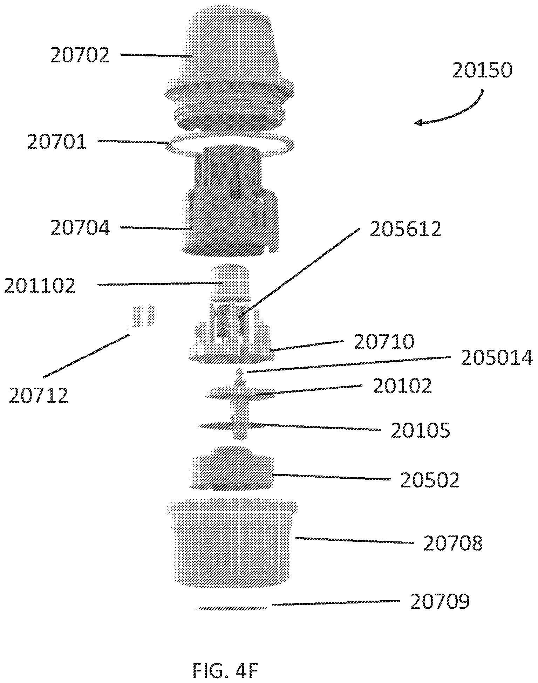

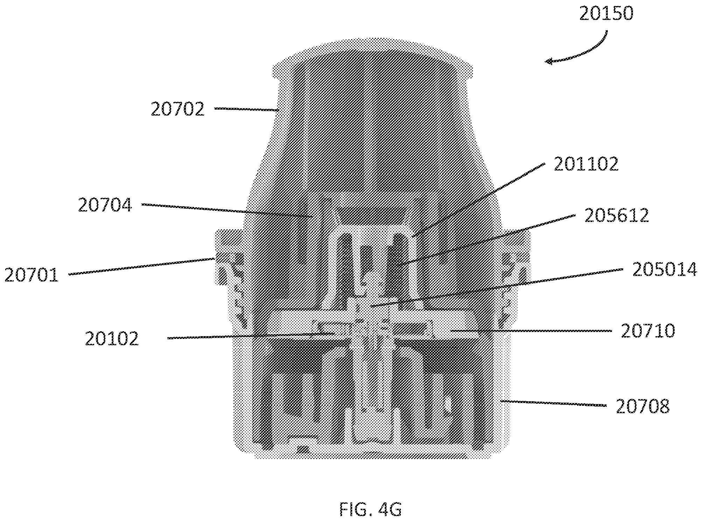

Referring to FIG. 4D-G for purpose of illustration and not limitation, the applicator device 20150 can be provided to a user as a single integrated assembly. FIGs. 4D and 4E provide perspective top and bottom views, respectively, of the applicator device 20150, FIG. 4F provides an exploded view of the applicator device 20150 and FIG. 4G provides a side cut-away view. The perspective views illustrate how applicator 20150 is shipped to and received by a user. The exploded and cut-away views illustrate the components of the applicator device 20150. The applicator device 20150 can include a housing 20702, gasket 20701, sheath 20704, sharp carrier 201102, spring 205612, sensor carrier 20710 (also referred to as a "puck carrier"), sharp hub 205014, sensor control device (also referred to as a "puck") 20102, adhesive patch 20105, desiccant 20502, cap 20708, serial label 20709, and tamper evidence feature 20712. As received by a user, only the housing 20702, cap 20708, tamper evidence feature 20712, and label 20709 are visible. The tamper evidence feature 20712 can be, for example, a sticker coupled to each of the housing 20702 and the cap 20708, and tamper evidence feature 20712 can be damaged, for example, irreparably, by uncoupling housing 20702 and cap 20708, thereby indicating to a user that the housing 20702 and cap 20708 have been previously uncoupled. These features are described in greater detail below.

### Exemplary Tray and Sensor Module Assembly

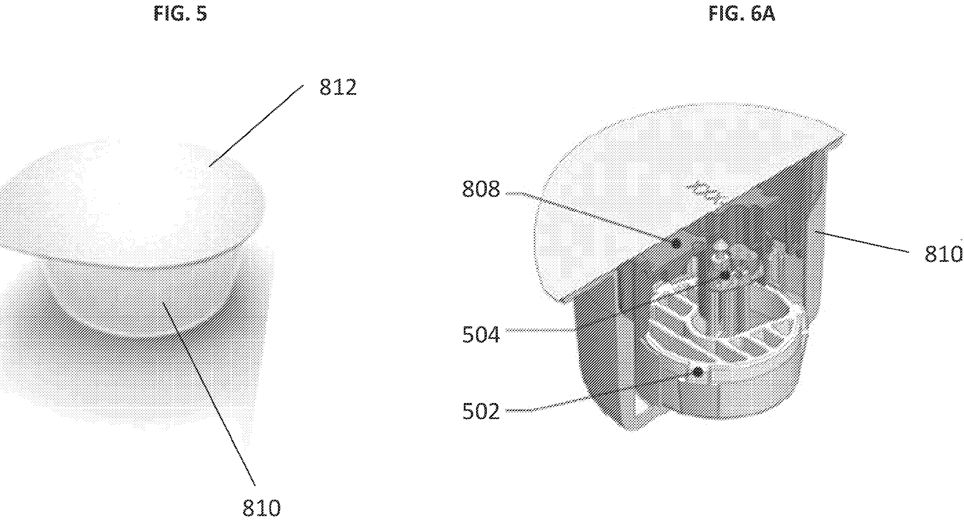

FIG. 5 is a proximal perspective view depicting an example embodiment of a tray 810 with sterilization lid 812 removably coupled thereto, which may be representative of how the package is shipped to and received by a user prior to assembly.

FIG. 6A is a proximal perspective cutaway view depicting sensor delivery components within tray 810. Platform 808 is slidably coupled within tray 810. Desiccant 502 is stationary with respect to tray 810. Sensor module 504 is mounted within tray 810.

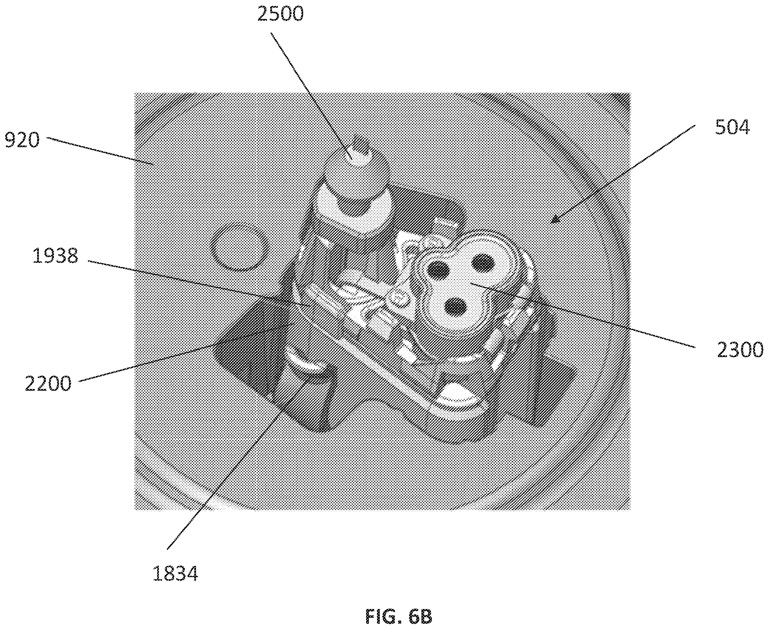

FIG. 6B is a proximal perspective view depicting sensor module 504 in greater detail. Here, retention arm extensions 1834 of platform 808 releasably secure sensor module 504 in position. Module 2200 is coupled with connector 2300, sharp module 2500 and sensor (not shown) such that during assembly they can be removed together as sensor module 504.

### Exemplary Applicator Housings and Caps

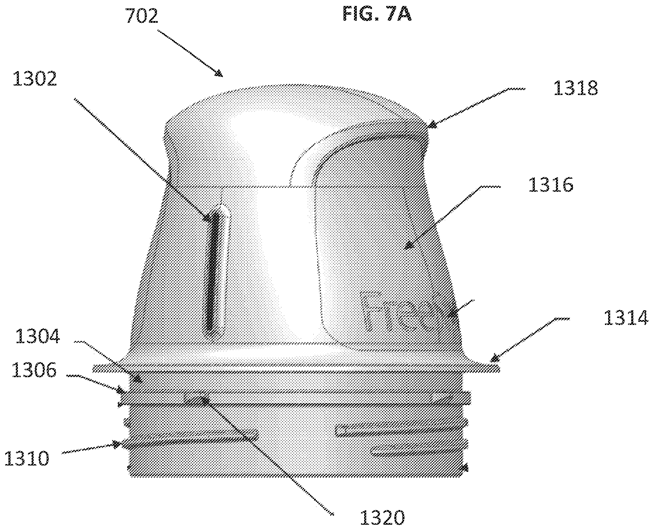

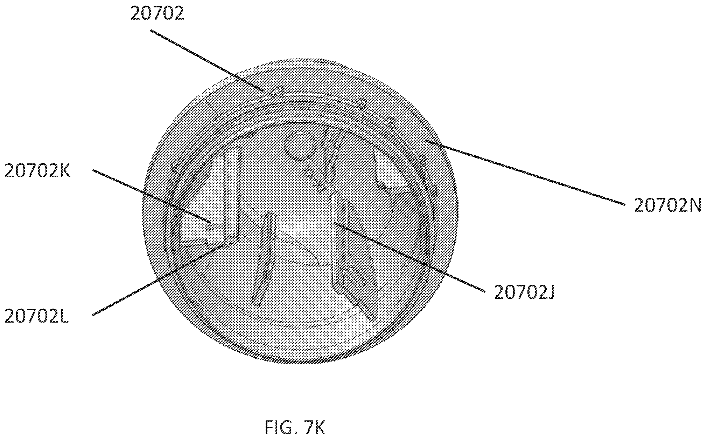

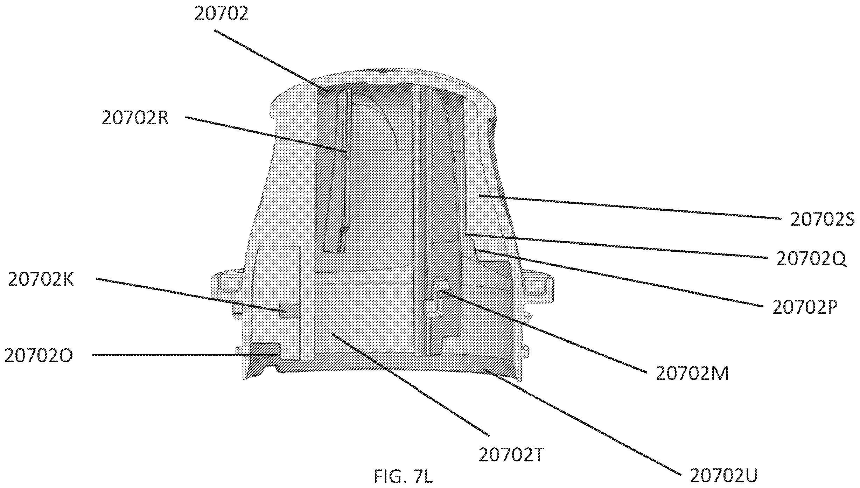

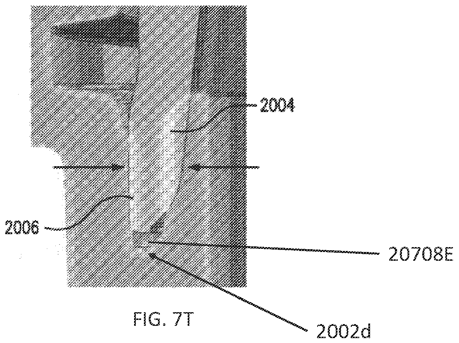

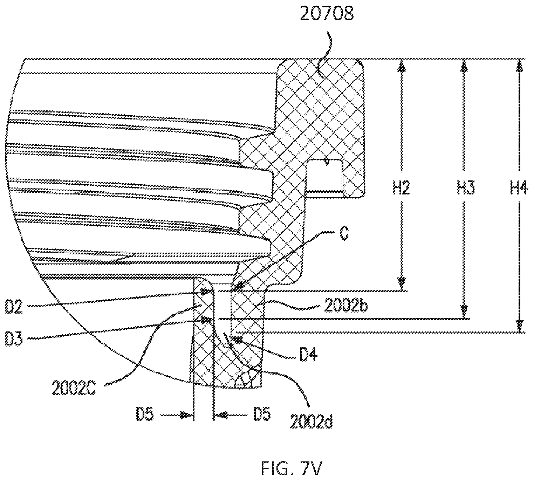

FIG. 7A is side view depicting an example embodiment of the applicator housing 702 that can include an internal cavity with support structures for applicator function. A user can push housing 702 in a distal direction to activate the applicator assembly process and then also to cause delivery of sensor control device 102, after which the cavity of housing 702 can act as a receptacle for a sharp. In the example embodiment, various features are shown including housing orienting feature 1302 for orienting the device during assembly and use. Tamper ring groove 1304 can be a recess located around an outer circumference of housing 702, distal to a tamper ring protector 1314 and proximal to a tamper ring retainer 1306. Tamper ring groove 1304 can retain a tamper ring so users can identify whether the device has been tampered with or otherwise used. Housing threads 1310 can secure housing 702 to complimentary threads on cap 708 (FIGS. 4A and 4B) by aligning with complimentary cap threads and rotating in a clockwise or counterclockwise direction. A side grip zone 1316 of housing 702 can provide an exterior surface location where a user can grip housing 702 in order to use it. Grip overhang 1318 is a slightly raised ridge with respect to side grip zone 1316 which can aid in ease of removal of housing 702 from cap 708. A shark tooth 1320 can be a raised section with a flat side located on a clockwise edge to shear off a tamper ring (not shown), and hold tamper ring in place after a user has unscrewed cap 708 and housing 702. In the example embodiment four shark teeth 1320 are used, although more or less can be used as desired.

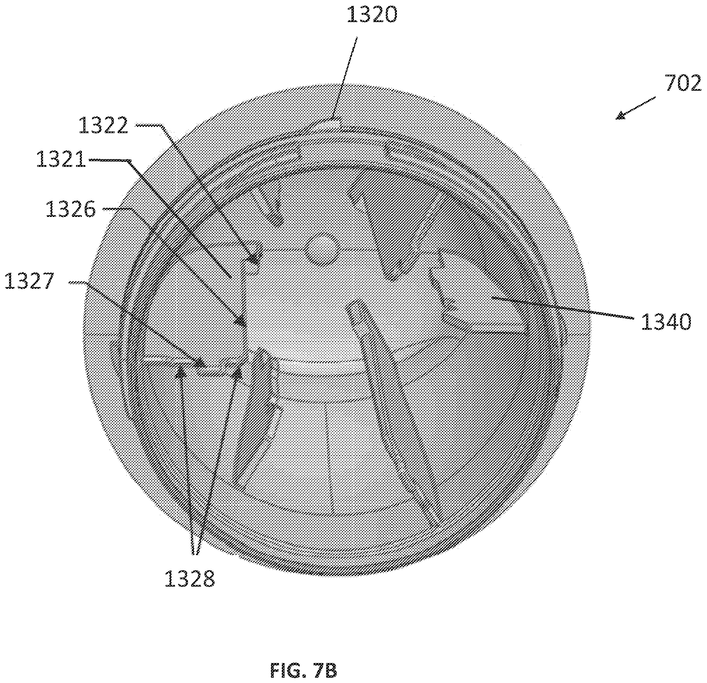

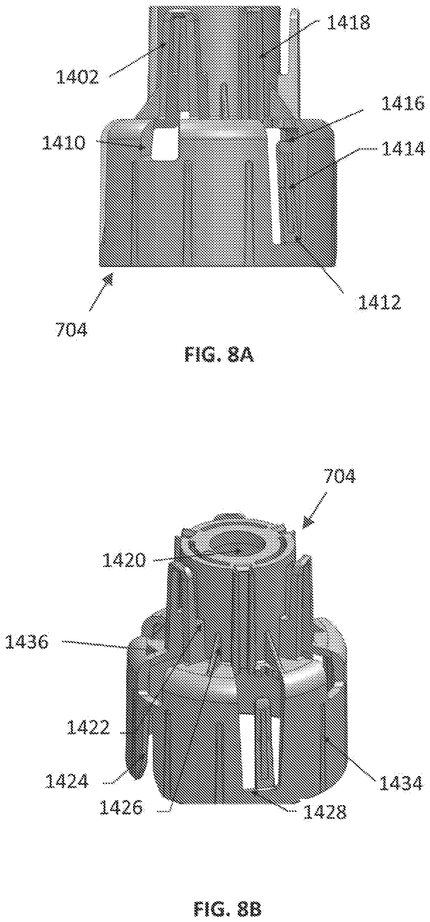

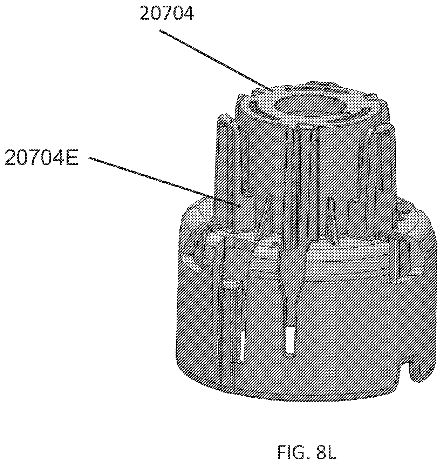

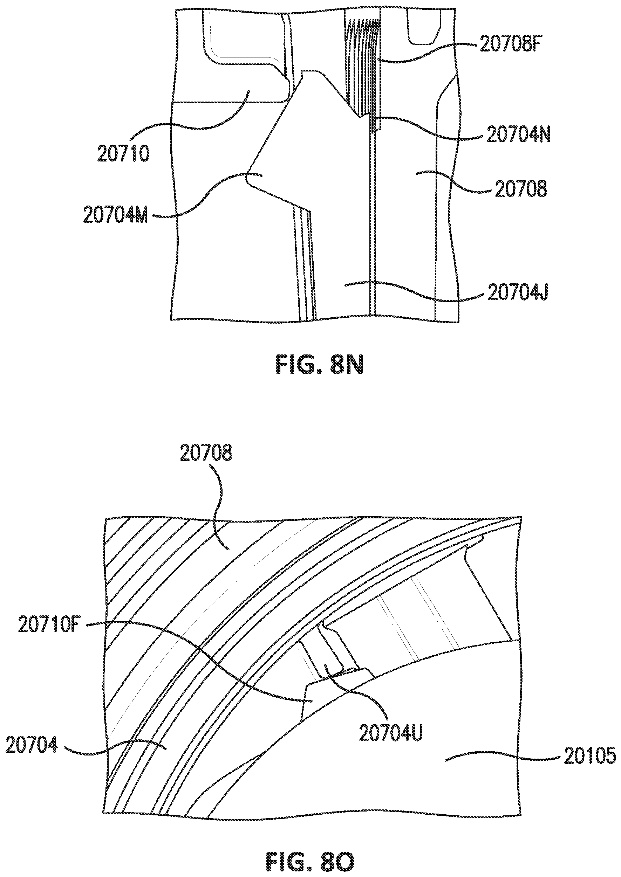

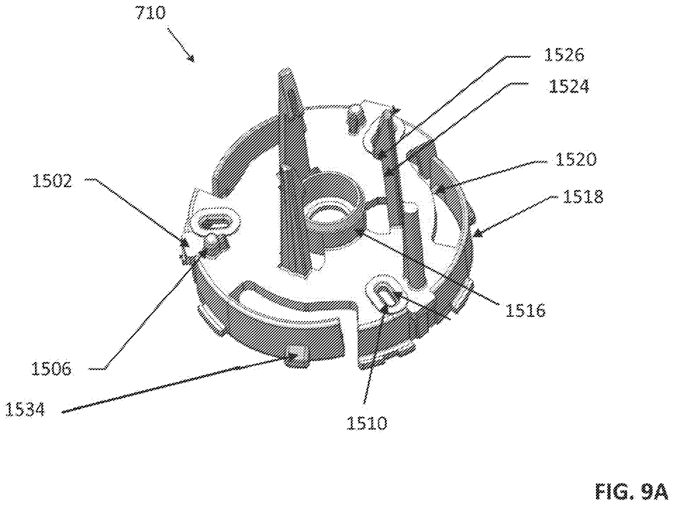

FIG. 7B is a perspective view depicting a distal end of housing 702. Here, three housing guide structures (or "guide ribs") 1321 are located at 120 degree angles with respect to each other and at 60 degree angles with respect to locking structures (or "locking ribs") 1340, of which there are also three at 120 degree angles with respect to each other. Other angular orientations, either symmetric or asymmetric, can be used, as well as any number of one or more structures 1321 and 1340. Here, each structure 1321 and 1340 is configured as a planar rib, although other shapes can be used. Each guide rib 1321 includes a guide edge (also called a "sheath guide rail") 1326 that can pass along a surface of sheath 704 (e.g., guide rail 1418 described with respect to FIG. 8A). An insertion hard stop 1322 can be a flat, distally facing surface of housing guide rib 1321 located near a proximal end of housing guide rib 1321. Insertion hard stop 1322 provides a surface for a sensor carrier travel limiter face 1420 of a sheath 704 (FIG. 8B) to abut during use, preventing sensor carrier travel limiter face 1420 from moving any further in a proximal direction. A carrier interface post 1327 passes through an aperture 1510 (FIG. 9A) of sensor carrier 710 during an assembly. A sensor carrier interface 1328 can be a rounded, distally facing surface of housing guide ribs 1321 which interfaces with sensor carrier 710.

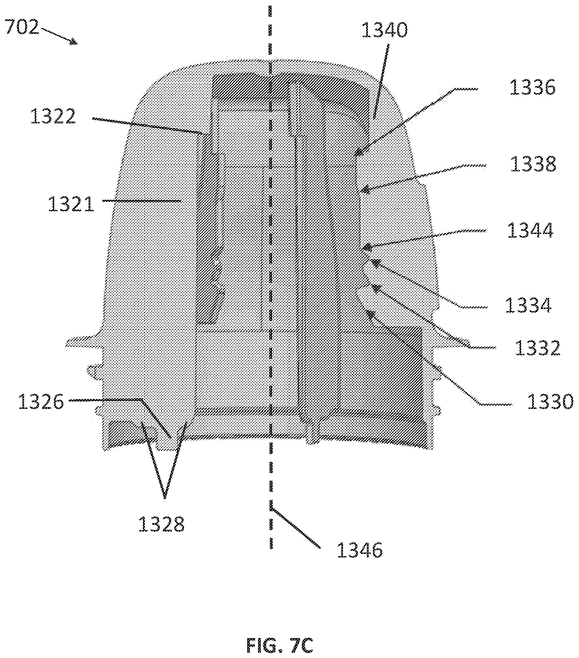

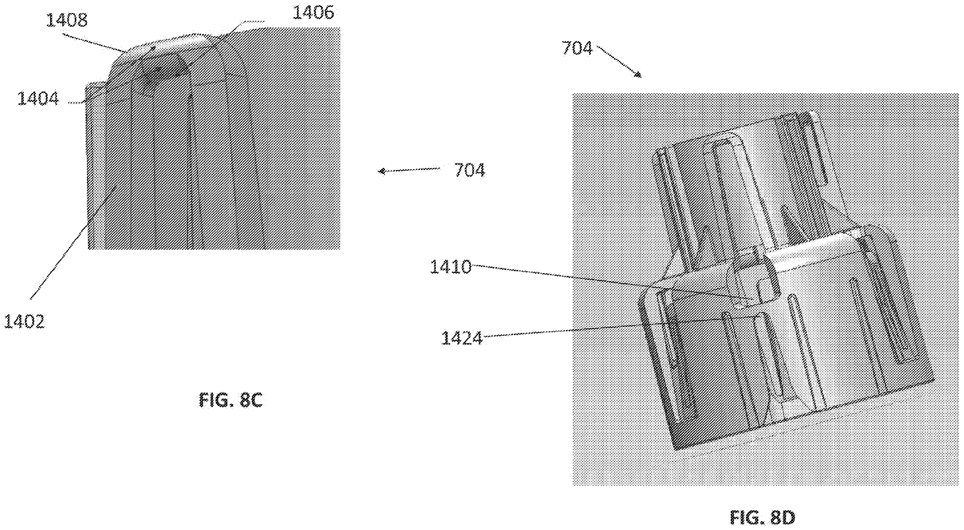

FIG. 7C is a side cross-section depicting an example embodiment of a housing. In the example embodiment, side cross-sectional profiles of housing guide rib 1321 and locking rib 1340 are shown. Locking rib 1340 includes sheath snap lead-in feature 1330 near a distal end of locking rib 1340 which flares outward from central axis 1346 of housing 702 distally. Each sheath snap lead-in feature 1330 causes detent snap round 1404 of detent snap 1402 of sheath 704 as shown in FIG. 8C to bend inward toward central axis 1346 as sheath 704 moves towards the proximal end of housing 702. Once past a distal point of sheath snap lead-in feature 1330, detent snap 1402 of sheath 704 is locked into place in locked groove 1332. As such, detent snap 1402 cannot be easily moved in a distal direction due to a surface with a near perpendicular plane to central axis 1346, shown as detent snap flat 1406 in FIG. 8C.

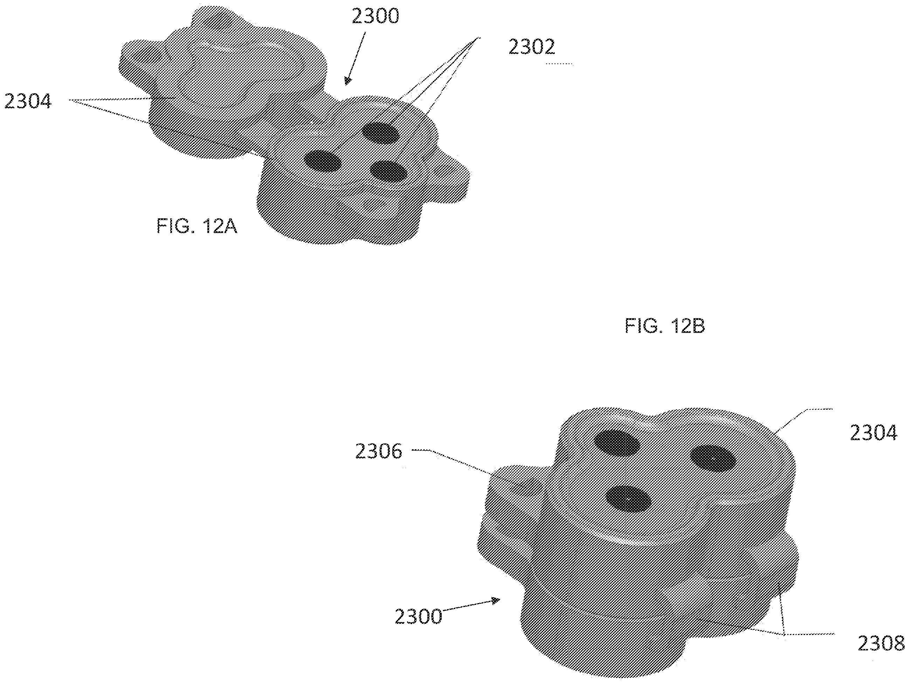

As housing 702 moves further in a distal direction toward the skin surface, and as sheath 704 advances toward the proximal end of housing 702, detent snaps 1402 shift into the unlocked grooves 1334, and applicator 150 is in an "armed" position, ready for use. When the user further applies force to the proximal end of housing 702, while sheath 704 is pressed against the skin, detent snap 1402 passes over firing detent 1344. This begins a firing sequence (as described, for example, with respect to FIGS. 12A-12D) due to release of stored energy in the deflected detent snaps 1402, which travel in a proximal direction relative to the skin surface, toward sheath stopping ramp 1338 which is slightly flared outward with respect to central axis 1346 and slows sheath 704 movement during the firing sequence. The next groove encountered by detent snap 1402 after unlocked groove 1334 is final lockout groove 1336 which detent snap 1402 enters at the end of the stroke or pushing sequence performed by the user. Final lockout recess 1336 can be a proximally-facing surface that is perpendicular to central axis 1346 which, after detent snap 1402 passes, engages a detent snap flat 1406 and prevents reuse of the device by securely holding sheath 704 in place with respect to housing 702. Insertion hard stop 1322 of housing guide rib 1321 prevents sheath 704 from advancing proximally with respect to housing 702 by engaging sensor carrier travel limiter face 1420.

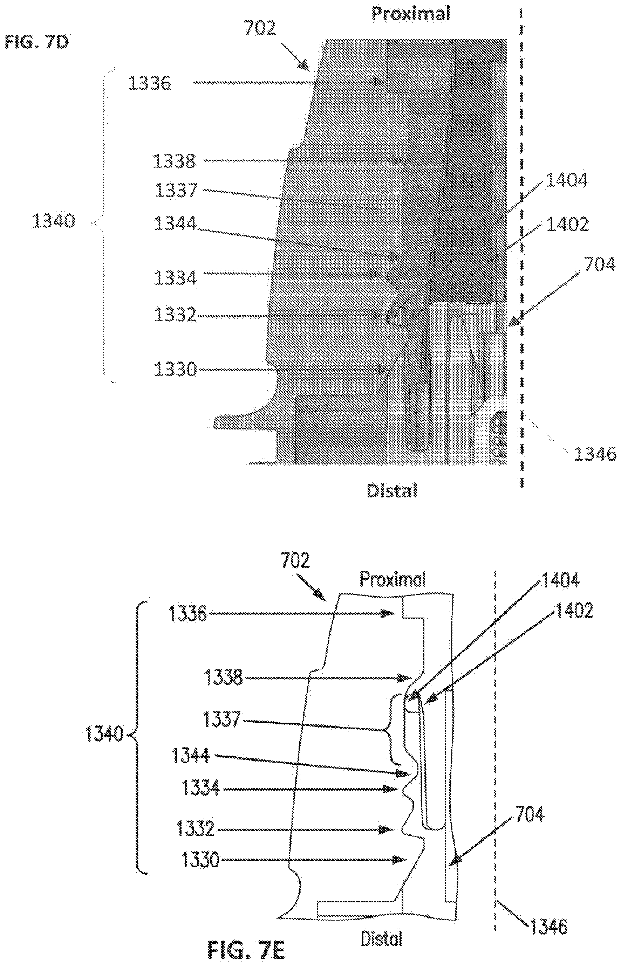

FIGS. 7D and 7E are close-up side views of an example embodiment of locking rib 1340 of applicator housing 702, as detent snap 1402 of sheath 704 moves toward the proximal end of housing 702. FIG. 7D shows sheath 704 in a "locked" state, in which detent round 1404 of detent snap 1402 has already passed over sheath snap lead-in feature 1330 and is positioned in locked groove 1332 of locking rib 1340. As force is applied to the proximal end of housing 702, detent round 1404 is advanced proximally into unlocked groove 1334, placing applicator 150 into an "armed" position. When force is further applied to the proximal end of housing 702, applicator 150 is "fired," as detent round 1404 is advanced proximally from the unlocked groove 1334 and passes over firing detent 1344. Thereafter, sheath 704 is further advanced proximally such that detent round 1404 is slidably advanced over firing surface 1337, as shown in FIG. 7E. In this embodiment, firing surface 1337 is substantially parallel to central axis 1346. As sheath 704 continues to advance proximally, detent round 1404 reaches sheath stopping ramp 1338 which slows the movement of sheath 704. Upon detent round 1404 reaching final lockout recess 1336, detent snap flat 1406 (not shown) is engaged and securely holds sheath 704 in place with respect to housing 702.

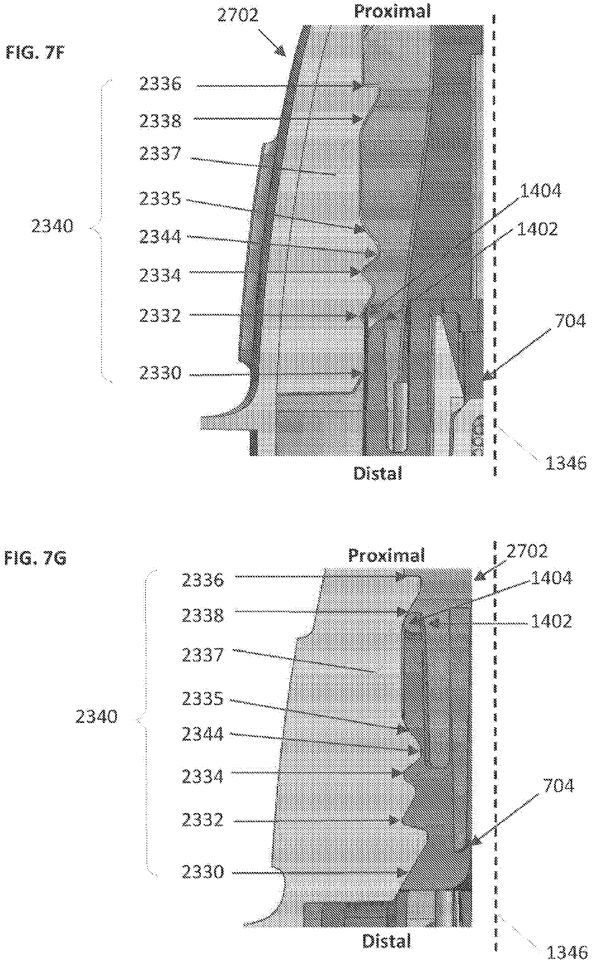

FIGS. 7F and 7G are close-up side views of an alternative embodiment of locking rib 2340 that is designed to improve the firing velocity of the sharp from the sensor applicator. Here, locking rib 2340 includes an inward detent ramp 2335 to reduce friction between sheath 704 and housing 2702 during firing. Locking rib 2340 also includes a sheath stopping ramp 2338 at the proximal end of firing surface 2337. In FIG. 7F, sheath 704 is initially shown in a "locked" state, in which detent round 1404 of detent snap 1402 has already passed over sheath snap lead-in feature 2330, and is positioned in locked groove 2332. As force is applied to the proximal end of housing 2702, detent round 1404 is advanced into unlocked groove 2334, placing applicator 150 into the "armed" position. When force is further applied to the proximal end of housing 2702, applicator 150 is "fired," as detent round 1404 passes over firing detent 2344.

As shown in FIG. 7G, detent round 1404 then advances toward the proximal end of housing 2702 in a "free flight" state, in which detent round 1404 passes over inward detent ramp 2335. While advancing proximally in the "free flight" state, detent round 1404 can be in noncontinuous, or have no contact with, inward detent ramp 2335 and firing surface 2337. In this regard, detent round 1404 can be easily and quickly advanced, as there is little to no frictional force between detent round 1404 and inward detent ramp 2335 and firing surface 2337, and as such, improves upon the firing velocity of the sharp from the applicator. Sheath stopping ramp 2338, which is positioned proximally further along the locking rib 2340 relative to the embodiment shown in FIGS. 7D and 7E, provides an edge portion to frictionally engage the detent round 1404 and slow the movement of sheath 704. The sheath stopping ramp 2338 can have a sloped shape and provide for increasing frictional contact as the detent round 1404 advances in a proximal direction. Finally, upon detent round 1404 reaching final lockout recess 2336, detent snap flat 1406 (not shown) is engaged and securely holds sheath 704 in place with respect to housing 2702. Lockout recess 2336 prevents detent round 1404 and sheath 704 from backwards, or distal movement. This embodiment reflects a higher firing velocity relative to the embodiment depicted in FIGS. 7D and 7E, which also assists in prevention of a premature withdrawal of sharp.

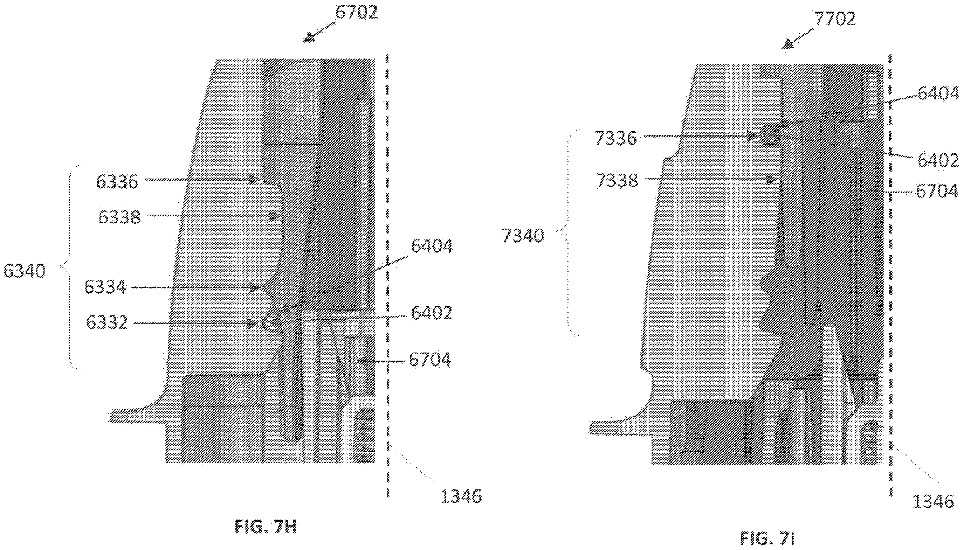

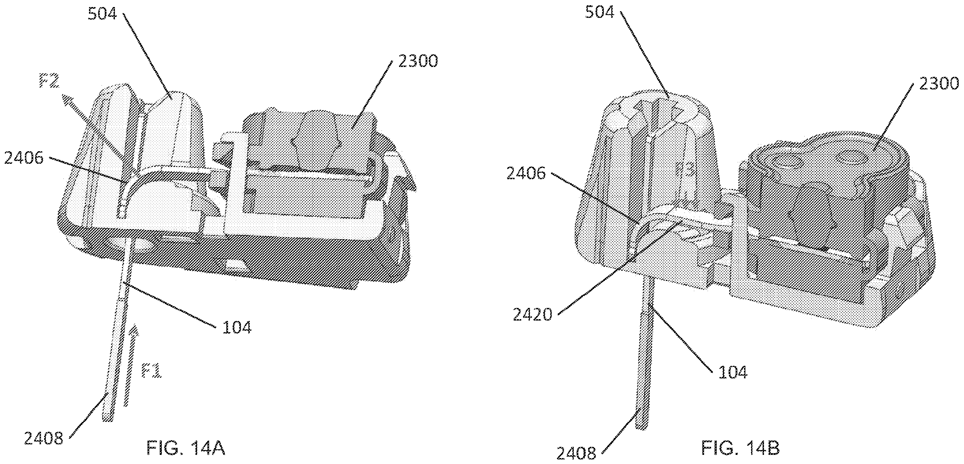

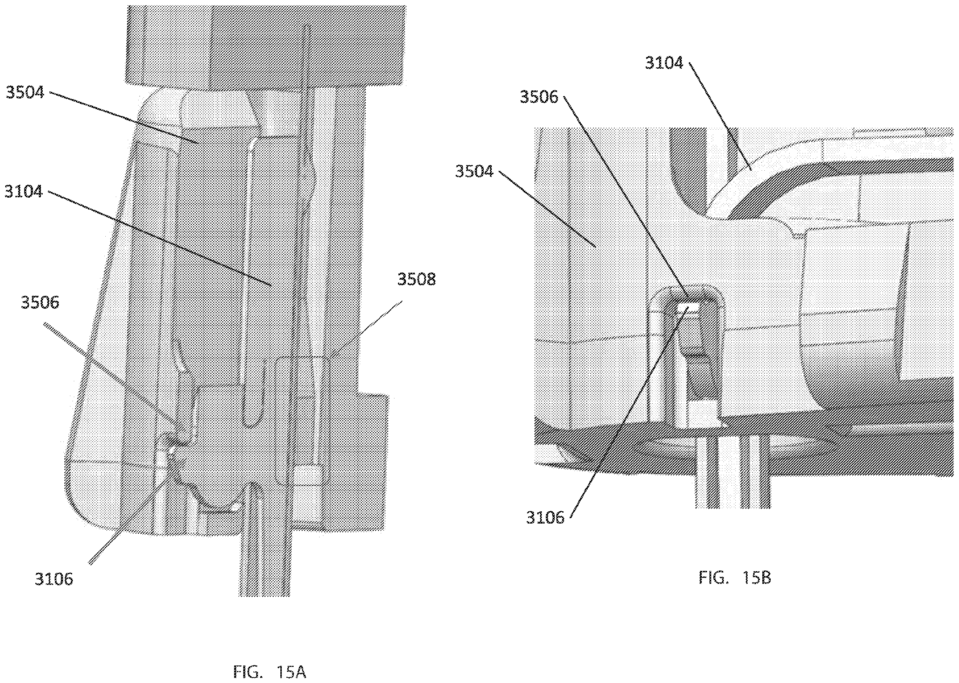

FIG. 7H is a close-up side view of an alternative embodiment of locking rib 6340 designed to maintain a downward force on sheath 6704 during firing which, in turn, can prevent sheath 6704 from unwanted movement during the sensor insertion process. Here, sheath 6704 is shown in a "locked" state, in which detent round 6404 of detent snap 6402 is positioned in locked groove 6332. As force is applied to the proximal end of housing 6702, detent round 6404 is advanced into unlocked groove 6334, placing applicator in the "armed" position. When force is further applied to the proximal end of housing 6702, applicator is "fired," and detent round 6404 advances over sloped firing surface 6338 toward the proximal end of housing 6702. Sloped firing surface 6338 can be angled toward central axis 1346 such that the resulting downward force upon sheath 6704 increases as detent round 6404 advances in a proximal direction. In the depicted embodiment, detent round 6404 is in continuous contact with sloped firing surface 6338. Lockout recess 6336 prevents detent round 6404 and sheath 6704 from backwards, or distal movement. This embodiment reflects a slower firing velocity relative to the previously described embodiments, and can be used, for example, with the motion-actuated sharp retraction process that is described with respect to FIGS. 14A-14C and 15A-15B.

FIG. 7I is a close-up side view of still another alternative embodiment of locking rib 7340, also designed to maintain a downward force on sheath 6704 during firing which, in turn, can prevent sheath 6704 from unwanted movement during a sensor insertion process. Here, sheath 6704 is shown in a "fired" state, in which detent round 6404 of detent snap 6402 is positioned in a two-way lockout recess 7336. Upon detent round 6404 advancing into two-way lockout recess 7336, sheath 6704 can be prevented from further movement in either a proximal or distal direction. This can reduce unwanted movement of sheath 6704 during the sensor insertion process. Furthermore, in some embodiments, as described with respect to FIGS. 14A-14C and 15A-15B, two-way lockout recess 7336 can provide for the immobilization of sheath 6704 during a motion-actuated sharp retraction process. As can be seen in FIG. 7I, sloped firing surface 7338 is angled toward central axis 1346 such that a resulting downward force upon sheath 6704 increases as detent round 6404 advances in a proximal direction. In the depicted embodiment, detent round 6404 is in continuous contact with sloped firing surface 7338. This embodiment reflects a slower firing velocity and can be used, for example, with the motion-actuated sharp retraction process that is described with respect to FIGS. 14A-14C and 15A-15B.

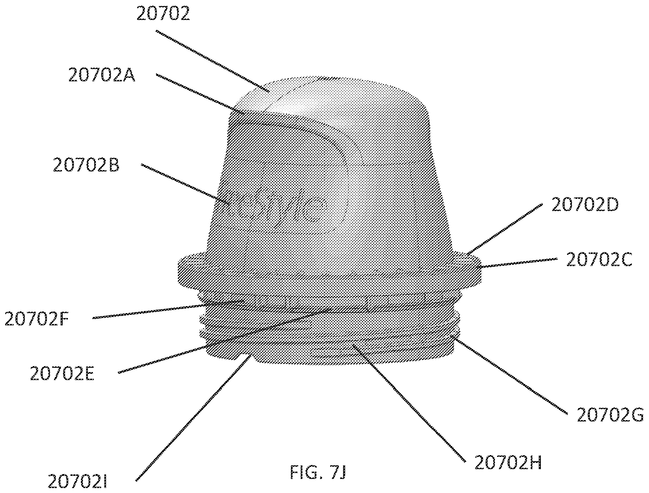

Referring to FIGs. 7J-7L, for purpose of illustration and not limitation, a housing 20702 in accordance with the disclosed subject matter is provided. Housing 20702 can be made of cyclic olefin copolymer, or other suitable materials, such as Polycarbonate or high density poly ethylene (HDPE). Housing 20702 can include one or more of the features described herein with regard to housings, wherein similar features can operate as described herein. For example, housing 20702 can include a grip overhang 20702A that can enable a user to securely grip housing 20702. The housing 20702 can have additional grip overhangs 20702A, for example, two grip overhands 20702A on opposite sides of the housing 20702. The housing 20702 can include a side grip zone 20702B disposed below the grip overhand 20702A. The side grip zone 20702B can be textured for improved gripping by a user. The housing 20702 can have additional side grip zones 20702B, for example, two side grip zones 20702B on opposite sides of the housing 20702, each disposed below a grip overhang 20702A.

The housing 20702 can include a housing skirt 20702C, which can provide a surface for tamper evidence feature 20712. The housing skirt 20702C can be supported by a plurality of skirt stiffening ribs 20702D. The skirt stiffening ribs 20702D can provide support for the housing skirt 20702C and can help protect the applicator device 20150 during a shock event, such as a drop. Additionally, the skirt stiffening ribs 20702D can be used to support the housing 20702 during manufacturing. The housing skirt 20702C and skirt stiffening ribs 20702D can provide stiffness against forces due to gasket compression, and can help maintain gasket 20701 compression through shelf life. The housing 20702 can include a gasket retention ring 20702E and a plurality of gasket retention pockets 20702F, which can hold the gasket 20701 relative the housing 20702. For example, the gasket retention ring 20702E can prevent lateral movement of the gasket 20701 and the gasket retention pockets 20702E can prevent rotation of the gasket 20701. The housing 20702 can include a plurality of gasket retention pockets, for example, 14 gasket retention pockets 20702E. Gasket sealing face 20702N that can seal against the gasket 20701. Housing 20702 can additionally or alternatively have an applicator cap sealing lip 20702U that can interface with the cap 20708, as described in greater detail below. Housing 20702 can have inner surface 20702T that can receive the sheath 20704.

Housing 20702 can include threads 20702G configure to engage with threads 20708D disposed on cap 20708. The threads can include radial limiting features 20702H, which can limit radial deformation of the cap 20702G during a shock event, such as a drop. Housing 20702 can include a plurality of radial limiting features 20702H, for example, 6 radial limiting features 20702H. The radial limiting features 20702H can be protrusions from the housing and can close a gap with the threads 20708D disposed on cap 20708. This can limit oval deformation of the cap 20702H during a shock event, such as a drop. Preventing oval deformation of cap 20702H can, in turn, ensure that lock arms 20704J of sheath 20704 stay locked between the cap 20702 and the sensor carrier 20710 to limit movement of the sheath 20704 prior to removing cap 20702H (as described in greater detail below). Housing 20702 can further include a clearance notch 20702I for clearance of the sheath arms during firing.

The interior of housing 20702 can include a plurality of sensor carrier attachment features for receiving, aligning, and limiting movement of the sensor carrier 20710. For example, housing 20703 can include sheath guide rails 20702J, which can help to align and guide sheath 20704 as the sheath 20704 moves relative the housing 20702. Housing 20702 can include sensor carrier attach slots 20702K, which can engage and hold the sensor carrier 20710, and sensor carrier hard stops 20702L, that can limit axial movement of the sensor carrier 20710 relative the housing 20702. Housing 20702 can include sensor carrier biasing feature 20702M that can remove slop between the sensor carrier 20710 and the housing 20702 after assembly and sensor carrier radial limiting feature 20702O that can keep the sensor carrier radially aligned relative the housing 20702. Flat horizontal faces between sensor carrier attach slots 20702K and sensor carrier radial limiting feature 20702O can be used to stop the sheath 20704 at the end of a stroke. Corresponding features on the sheath 20704 can interact with these faces. The sensor carrier biasing feature 20702M can further limit rotation of the sensor carrier 20710 relative the housing 20702. Housing 20702 can include one or more of each of the sheath guide rails 20702J, sensor carrier attach slots 20702K, sensor carrier hard stops 20702L, sensor carrier radial limiting feature 20702O, and sensor carrier biasing feature 20702M, for example, three of each.

The interior of housing 20702 can further include a plurality sheath ribs 20702S for engaging the sheath 20704 for insertion, as described herein. Housing 20702 can include one or more of sheath ribs 20702S, for example, three. Each sheath rib 20702S can include a sheath snap lead in feature 20702P configured to initially lead in the detent snap 20704A of sheath 20704 into the correct location. The housing 20702 can include a firing detent 20702Q. After the detent snap 20704A of sheath 20704 passes the firing detent 20702Q, the firing sequence can be initiated, and the sheath 20704 can travel toward the sheath stopping ramp 20702R. The sheath stopping ramp 20702 can slow the sheath 20704 at the end of firing.

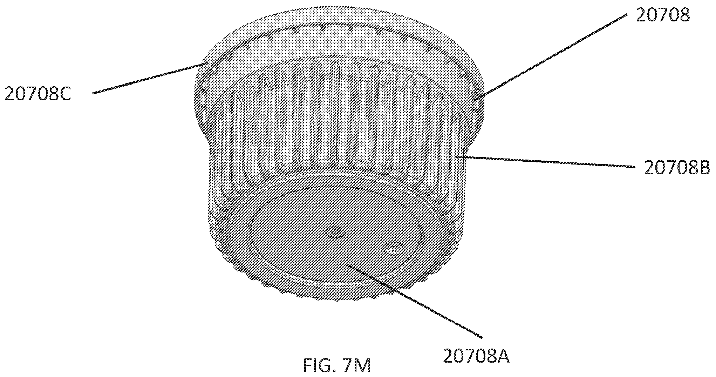

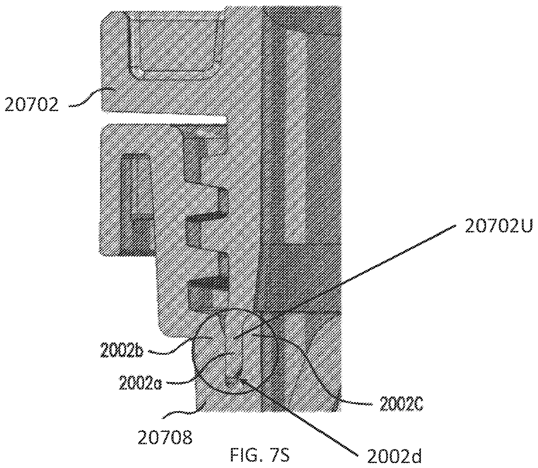

Referring to FIGs. 7M-7Q and 7S-X for purpose of illustration, an exemplary cap 20708 is provided. Cap 20708 can include one or more of the features described herein with regard to caps, wherein similar features can operate as described herein. Cap 20708 can be made of high density polyethylene (HDPE) or any other suitable materials, such as Polypropylene or low-density polyethylene (LDPE). Cap 20708 can include a label surface 20708A configured to receive label 20709. Cap 20708 can include ribs 20708B which can provide strength and provide an improved gripping surface for a user. The cap 20708 can include tamper label ring 20708C, which can receive the tamper evidence feature 20712. The cap 20708 can also include a gasket sealing surface 20708G, configured to engage gasket 20701.

Internally, cap 20708 can include threads 20708D, which can engage threads 20702G disposed on the housing 20702. Cap 20708 can also include seal interface 20708E which can be configured to receive the applicator cap sealing lip 20702U to create a seal between the housing 20702 and the cap 20708.

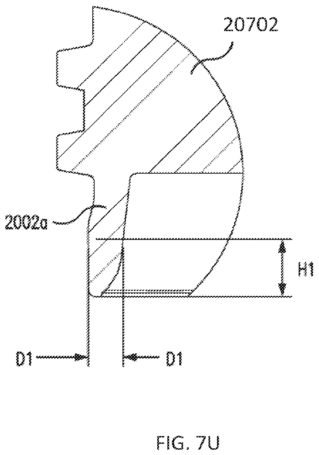

FIGS. 7S-V show an enlarged cross-sectional side view of the interface between housing 20702 and cap 20708. As illustrated, applicator cap sealing lip 20702U of housing 20702 includes a first axial extension 2002a and seal interface 20708E of cap 20708 provides a cavity 2002d matable with the first axial extension 2002a. In the illustrated embodiment, the diameter of cavity 2002d formed from second axial extension 2002b and third axial extension 2002c of the cap 20708 is sized to receive the diameter of first axial extension 2002a of housing 20702 within cavity 2002d. For example, as shown in FIG. 7U, axial extension 2002a can have thickness D1 at height H1, as measured from distal edge of axial extension 2002a. Similarly, second axial extension 2002c can have a thickness D5 at height H3, as measured from proximal edge of cap 20708; cavity 2002d can have a thickness D2, D3, and D4 at heights H2, H3, and H4, respectively, as measured from proximal edge of cap 20708. In certain embodiments, D1 can measure 1mm with a tolerance of +/- 0.03mm, D2, D3, D4 can have any suitable dimensions, H1 can measure 1.66mm with a tolerance of +/- 0.1mm, H2 can measure 8.25mm with a tolerance of +/- 0.1mm, H3 can measure 9.25mm with a tolerance of +/- 0.1mm, H4 can measure 9.75mm with a tolerance of +/- 0.1mm. In other embodiments, however, the reverse can be employed, where the diameter of first axial extension 2002a can be sized to receive the diameter of the second axial extension 2002b

In each embodiment, two radial seals 2004, 2006 can be defined or otherwise provided at the interface between first and second axial extensions 2002a, b and radial seals 2004 and 2006 can help prevent migration of fluids or contaminants across the interface in either axial direction. Moreover, the dual radial seals described herein can accommodate tolerance and thermal variations combined with stress relaxation via a redundant sealing strategy. In the illustrated embodiment, dual radial seals 2004, 2006 utilize a "wedge" effect for effective sealing between first axial extension 2002a and second axial extension 2002b.

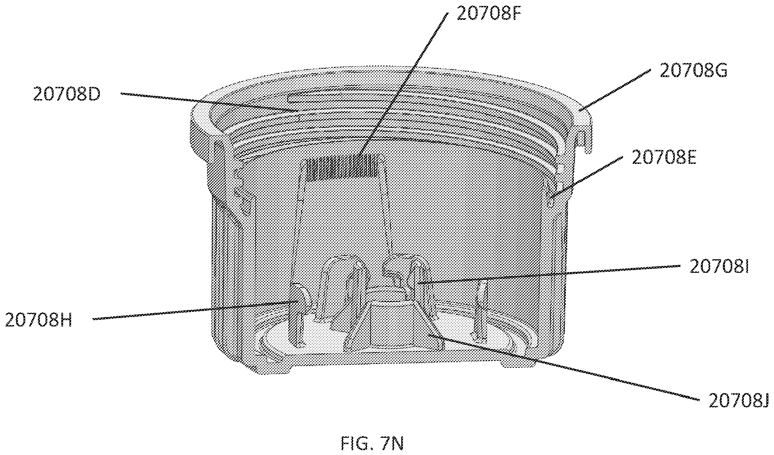

Cap 20708 can include one or more sets of crush ribs 20708F (see FIG. 7N), for example, two sets of crush ribs 20708F. The crush ribs 20708F can be configured to engage the sharp edge 20704N of lock arm 20704J during a shock event, for example a drop, as described in greater detail below (see e.g., FIG. 8N).

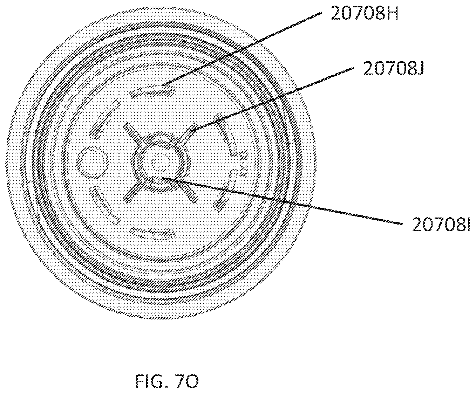

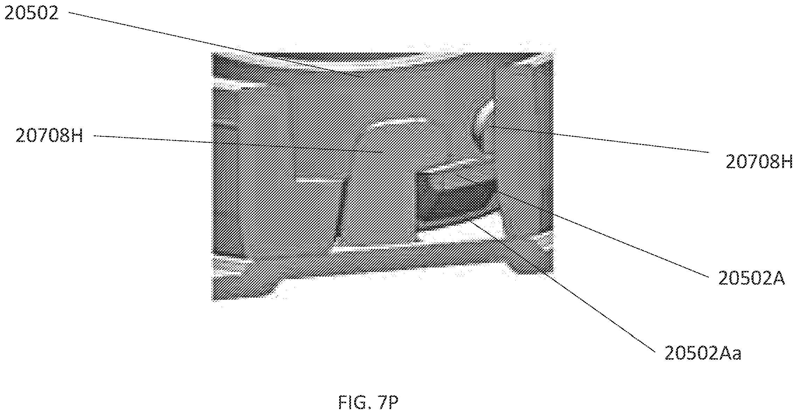

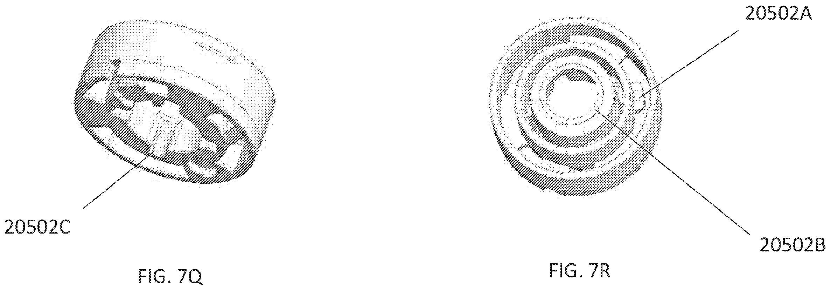

In accordance with the disclosed subject matter, cap 20708 can include one or more desiccant retention clips 20708H to retain the desiccant 20502 in the cap 20708 and limit rotation of the desiccant 20502 (see e.g., FIG. 7P). Cap 20708 can include any number of desiccant retention clips 20708H. For example, cap 20708 can include one, two, three, four, five, six, or more desiccant retention clips 20708H. As can be seen in FIG. 7O, cap 20708 can include three pairs of two desiccant retention clips 20708H each. Each pair of desiccant retention clips 20708H may correspond to a desiccant retention snap 20502A (see e.g., FIG. 7R) on desiccant 20502 (see e.g., FIG. 7P). As can be seen in FIG. 7P, desiccant 20502 can include a plurality of desiccant retention snaps 20502A and each desiccant retention snap 20502A may snap down into a pair of desiccant retention clip 20708H. For example, desiccant retention snap 20502A can include angled surface 20502Aa to radially expand clips 20708H when the desiccant is pressed down onto the cap until desiccant retention snap 20502A locks in place within desiccant retention clips 20708H. Desiccant retention clips 20708H can hold desiccant 20502 within cap 20708 to limit distal and rotational movement of desiccant 20502. As can be seen in FIG. 7Q, desiccant 20502 can include catch pockets (not shown) to angularly position desiccant 20502 relative to desiccant retention clips 20708H. In some embodiments, as can be seen in FIG. 7Q desiccant 20502 can be cylindrical in shape.

In accordance with disclosed subject matter, cap 20708 can include a ratchet 20708I to engage the sensor cap and remove the sensor cap when the cap 20708 is removed from the housing 20702, as described in greater detail below. According to some embodiments, ratchet 20708I can be provided in a cap 20708 without the use or presence of the desiccant retention clips 20708H described earlier. In other embodiments, ratchet 20708I and desiccant retention clips 20708H can be provided together with cap 20708. Cap 20708 can include a plurality of ribs 20708J to provide strength. Correspondingly, as can be seen in FIGS. 4G and 7Q-R, internal bore 20502B of desiccant 20502 can be sized and dimensioned to allow sufficient clearance for sensor cap to pass through and engage with ratchet 20708I of cap 20708; desiccant 20502 can include a plurality of rib pockets 20502C to provide clearance for plurality of ribs 20708J of cap 20708.

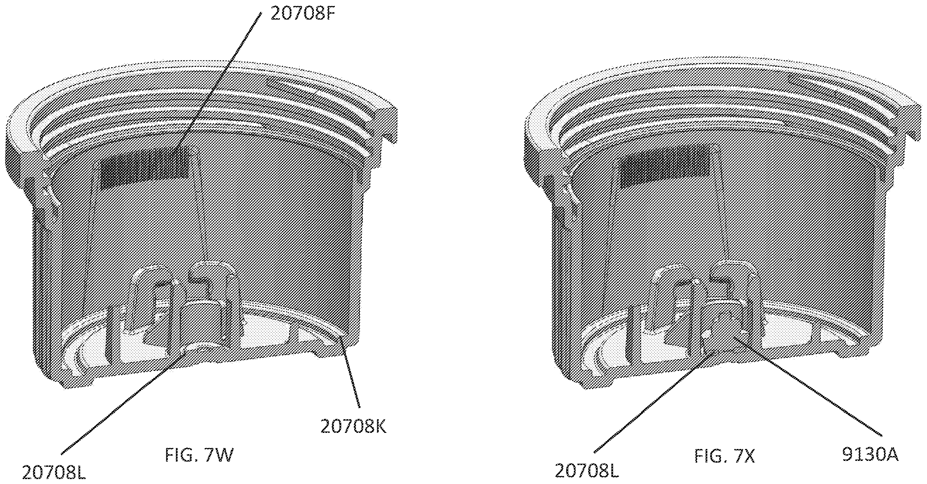

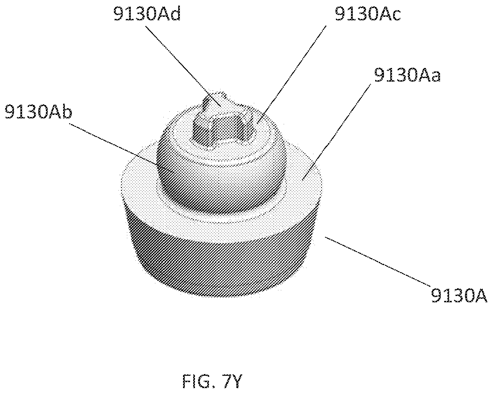

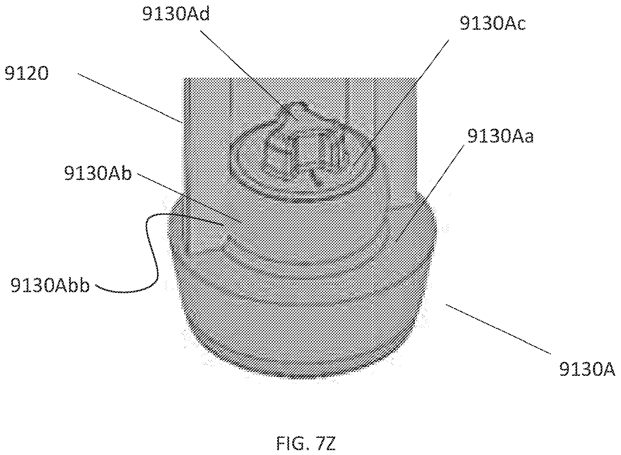

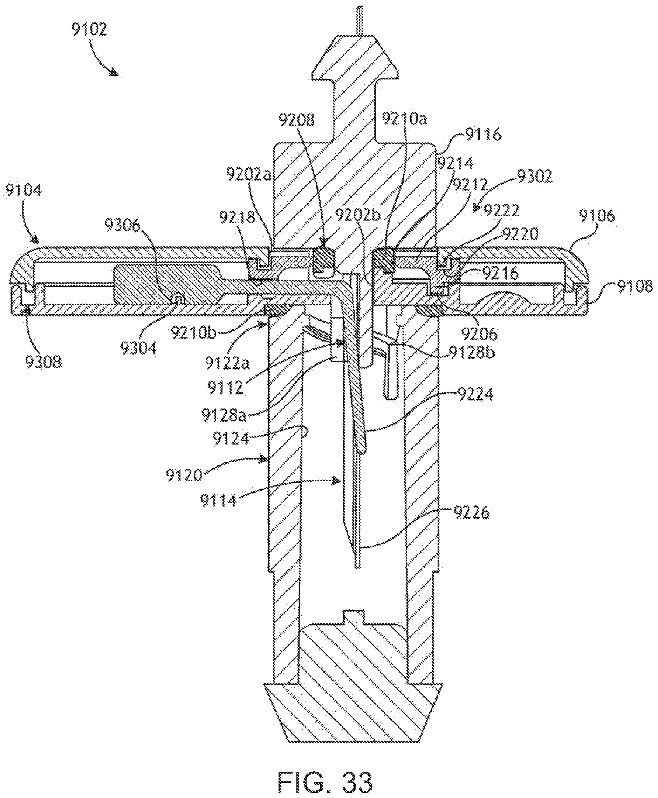

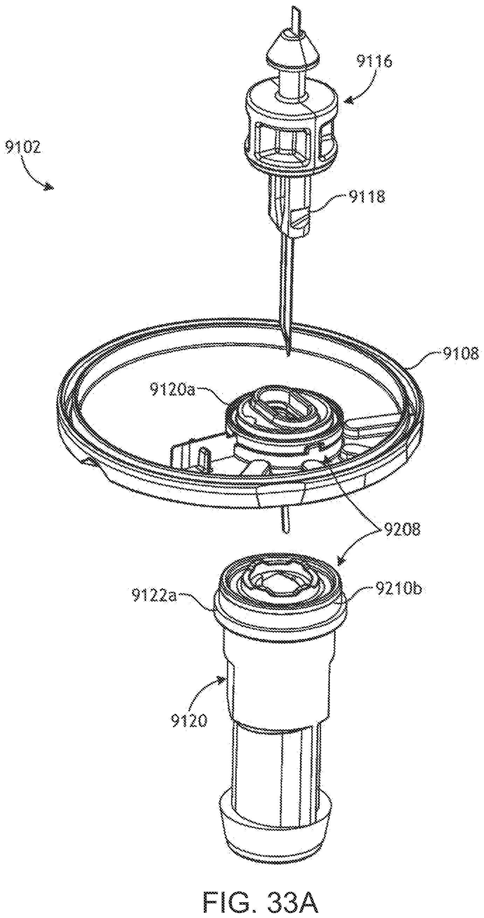

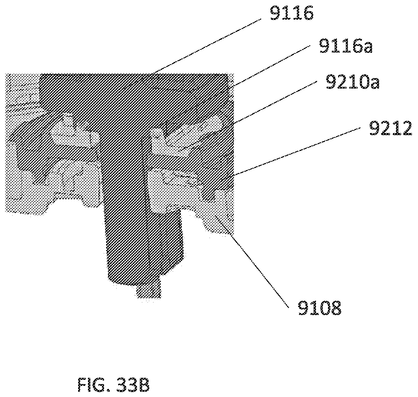

Referring to FIGs. 7W and 7X for purpose of illustration and not limitation, in accordance with the disclosed subject matter, cap 20708 can include one or more surfaces to engage other elements in the applicator device 20150 to provide support or limit movement in the case of a shock event, for example, a drop. For example, the cap can include a sheath support surface 20708K, configured to support the sheath 20704 during a shock event. The sheath support surface 20708K can limit distal movement of the sheath 20704 during a shock event. This can lead to less stress on the sensor carrier 20710 and the sensor control device 20102 and can reduce the risk of the sensor control device 20102 dislodging form the sensor carrier 20710. Additionally, or alternatively, the cap 20708 can include a raised ridge 20708L. The raised ridge 20708L can interface with a plug, such as an elastomeric plug 9130A (which can, in some embodiments, be coupled to a desiccant cap 9130 and in other embodiments, as can be seen in FIG. 7Z, couple to sensor cap 9120). In some embodiments, the elastomeric plug can include liquid silicone rubber. The raised ridge 20708L can thereby also support the sharp carrier 201102, sensor carrier 20710, sensor control device 20102, and accordingly, can prevent dislodging of the sensor control device 20102 from the sensor carrier 20710 during a shock event. Furthermore, additional support on the elastomeric plug 9130A and other features can increase the stress on various seals in the applicator device 20150, and thereby improve the seals prior to removing the cap 20708 from the housing. For example, as can be seen in FIG. 7Y, elastomeric plug 9130A may include stop surface 9130Aa, seal bead 9130Ab, intermediate surface 9130Ac, and top surface 9130Ad. As can be seen in FIG. 7Y, top surface 9130Ad can include a triangular surface with squares or rectangular corners instead of pointed vertices. As can be seen in FIG. 7Z, seal bead 9130Ab may be inserted in sensor cap 9120 until stop surface 9130Aa (as shown in cross-sectional view of FIG. 33) mates to the bottom of surface of sensor cap 9120. Seal bead 9130Ab may generally be barrel shaped (i.e., cylindrical shape with a thicker middle portion that tapers towards the top and bottom of seal bead 9130Ab) and configured to create an interference fit 9130Abb between the seal bead 9130Ab and the inner diameter of sensor cap 9120 which creates a sterile barrier and to seal inner chamber 9124.

### Exemplary Applicator Sheaths

FIGS. 8A and 8B are a side view and perspective view, respectively, depicting an example embodiment of sheath 704. In this example embodiment, sheath 704 can stage sensor control device 102 above a user's skin surface prior to application. Sheath 704 can also contain features that help retain a sharp in a position for proper application of a sensor, determine the force required for sensor application, and guide sheath 704 relative to housing 702 during application. Detent snaps 1402 are near a proximal end of sheath 704, described further with respect to FIG. 8C below. Sheath 704 can have a generally cylindrical cross section with a first radius in a proximal section (closer to top of figure) that is shorter than a second radius in a distal section (closer to bottom of figure). Also shown are a plurality of detent clearances 1410, three in the example embodiment. Sheath 704 can include one or more detent clearances 1410, each of which can be a cutout with room for sheath snap lead-in feature 1330 to pass distally into until a distal surface of locking rib 1340 contacts a proximal surface of detent clearance 1410.

Guide rails 1418 are disposed between sensor carrier traveler limiter face 1420 at a proximal end of sheath 704 and a cutout around lock arms 1412. Each guide rail 1418 can be a channel between two ridges where the guide edge 1326 of housing guide rib 1321 can slide distally with respect to sheath 704.

Lock arms 1412 are disposed near a distal end of sheath 704 and can include an attached distal end and a free proximal end, which can include lock arm interface 1416. Lock arms 1412 can lock sensor carrier 710 to sheath 704 when lock arm interface 1416 of lock arms 1412 engage lock interface 1502 of sensor carrier 710. Lock arm strengthening ribs 1414 can be disposed near a central location of each lock arm 1412 and can act as a strengthening point for an otherwise weak point of each lock arm 1412 to prevent lock arm 1412 from bending excessively or breaking.

Detent snap stiffening features 1422 can be located along the distal section of detent snaps 1402 and can provide reinforcement to detent snaps 1402. Alignment notch 1424 can be a cutout near the distal end of sheath 704, which provides an opening for user alignment with sheath orientation feature of platform 808. Stiffening ribs 1426 can include buttresses, that are triangularly shaped here, which provide support for detent base 1436. Housing guide rail clearance 1428 can be a cutout for a distal surface of housing guide rib 1321 to slide during use.

FIG. 8C is a close-up perspective view depicting an example embodiment of detent snap 1402 of sheath 704. Detent snap 1402 can include a detent snap bridge 1408 located near or at its proximal end. Detent snap 1402 can also include a detent snap flat 1406 on a distal side of detent snap bridge 1408. An outer surface of detent snap bridge 1408 can include detent snap rounds 1404 which are rounded surfaces that allow for easier movement of detent snap bridge 1408 across interior surfaces of housing 702 such as, for example, locking rib 1340.

FIG. 8D is a side view depicting an example embodiment of sheath 704. Here, alignment notch 1424 can be relatively close to detent clearance 1410. Detent clearance 1410 is in a relatively proximal location on distal portion of sheath 704.

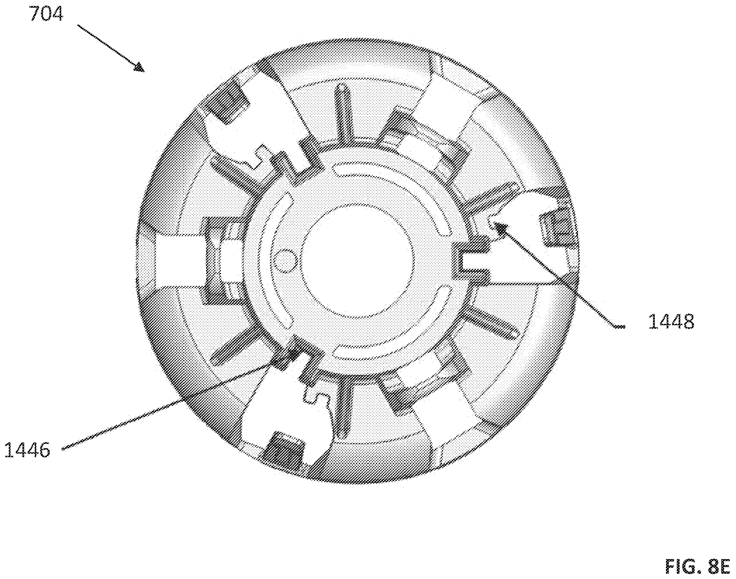

FIG. 8E is an end view depicting an example embodiment of a proximal end of sheath 704. Here, a back wall for guide rails 1446 can provide a channel to slidably couple with housing guide rib 1321 of housing 702. Sheath rotation limiter 1448 can be notches which reduce or prevent rotation of the sheath 704.

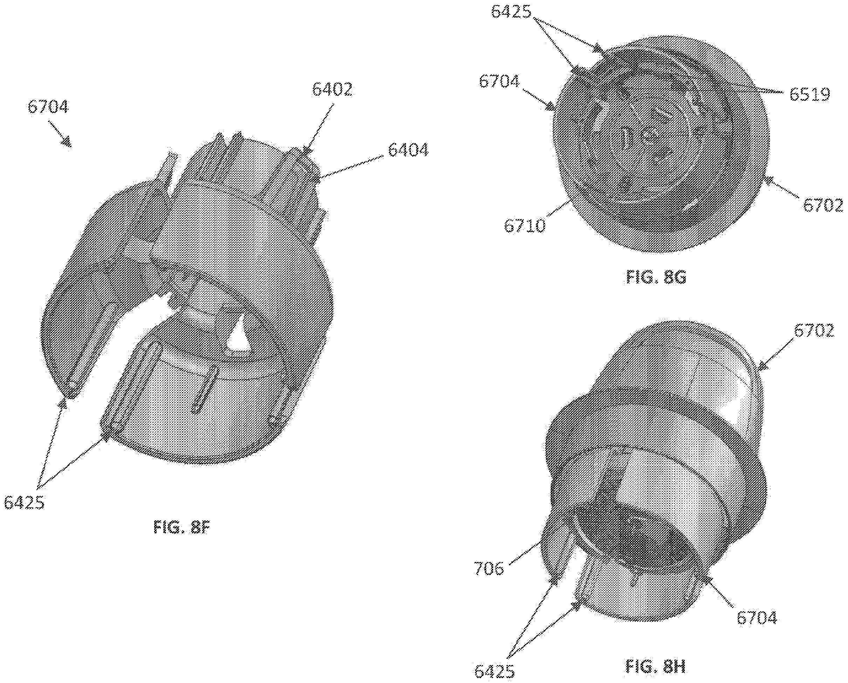

FIGS. 8F-8H are perspective views of an alternative example embodiment of sheath 6704 in various stages of assembly with other components of the applicator. As shown in FIG. 8F, sheath 6704 can have many of the same features as sheath 704, previously described with respect to FIGS. 8A-8C. Sheath 6704, for example, can include one or more detent snaps 6404 having one or more detent rounds 6402 attached thereto. Sheath 6704, however, can be shorter in overall length as compared to sheath 702. In addition, sheath 6704 can include one or more inner sheath ribs 6425 disposed on the inner surface of sheath 6704, and which protrude in an inward direction towards the central axis of sheath 6704.

Turning to FIG. 8G, sheath 6704 is shown in perspective view in a stage of assembly with applicator housing 6702 and sensor carrier 6710. One or more inner sheath ribs 6425 of sheath 6704 can interface with one or more corresponding rib notches 6519 in sensor carrier 6710. The fitted interface between corresponding ribs 6425 and notches 6519 can help maintain axial alignment of the sheath 6704 and sensor carrier 6710 during the sensor insertion process. Furthermore, the interface between ribs 6425 and notches 6519 can reduce lateral and rotational movement between the applicator components, which can, in turn, reduce the chance of improper sensor insertion.

Turning to FIG. 8H, sheath 6704 is shown in perspective view in a stage of assembly with applicator housing 6702 and sensor electronics housing 706, which has been inserted into sensor carrier 6710. Inner sheath ribs 6425 are also shown.

It should be noted that although six inner sheath ribs 6425 and six corresponding rib notches 6519 are depicted, any number of ribs and notches are fully within the scope of the present disclosure. Moreover, while ribs 6425 are depicted with a rounded surface edge, in other embodiments, ribs 6425 can have a rectangular or triangular shape, and rib notches 6519 can have a corresponding receiving shape for interfacing with ribs 6425. In addition, although ribs 6425 are depicted as being disposed on an inner circumferential surface of sheath 6704, ribs 6425 can also be disposed on any other surface of sheath 6704, or portion thereof, that comes into contact with sensor carrier 6710.

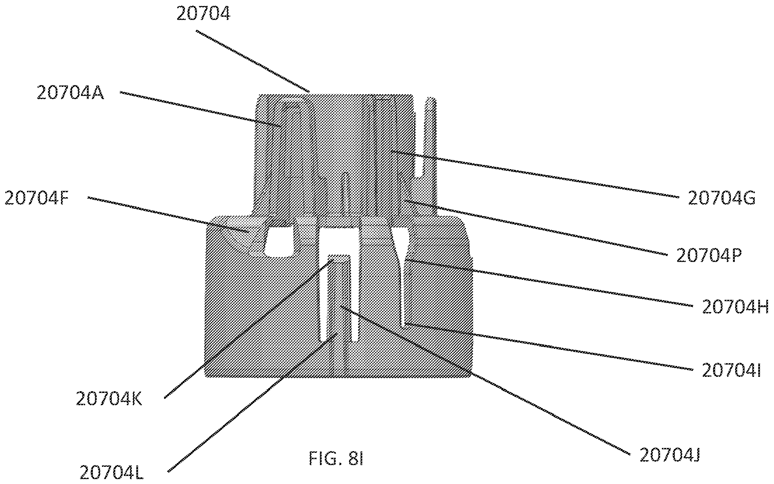

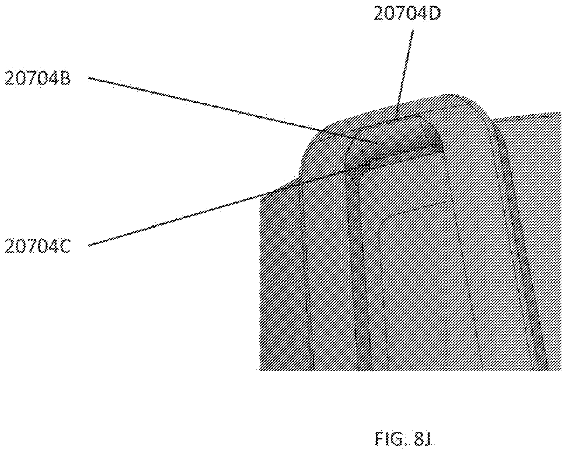

Referring to FIGs. 8I-8O, for purpose of illustration and not limitation, a sheath 20704 is provide, in accordance with the disclosed subject matter. Sheath 20704 can be made of Delrin or other suitable materials, for example, other low friction polymers. Sheath 20704 can include one or more of the features described herein with regard to sheaths, wherein similar features can operate as described herein. For example, sheath 20704 can include detent snaps 20704A having a free proximal end, configured to engage the sheath ribs 20702S during firing. Fig. 8J shows a close up of the free proximal end of detent snap 20704A. The detent snaps 20704A can include a round portion 20704B, for engagement with the sheath ribs 20702S and a flat portion 20704C for final lockout on housing 20704 after use. The round portion 20704B can include a parting line mismatch 20704D that can prevent a force spike during firing. The detent snaps 20704A can be coupled to the sheath 20704 at an enlarged distal portion 20704E, which can provide support to the detent snap 20704. Sheath 20704 can include a plurality of detent snaps 20704A, for example, three. The sheath 20704 can include one or more, for example, three, housing clearances 20704F, which can allow the sheath 20704 to clear the housing 20702 at the end of firing. In accordance with the disclosed subject matter, sheath 20704 can further include a plurality of stiffening ribs 20704P (e.g., six) which can stiffen the sheath 20704.

Sheath 20704 can include a plurality of guides 20704G for engaging the sheath guide rails 20702J of the housing 20702. Sheath 20704 can further include a slot 20704H including a stop 20704I at a distal end of the slot 20704H configured to engage the sheath guide rails 20702J of the sheath 20702 to limit further proximal movement of the sheath 20704 relative the housing 20702 at the end of firing. Sheath 20704 can also include a clearance 20704T for clearing the sensor carrier biasing feature 20702I disposed on the sheath guide rails 20702J of the housing 20702.

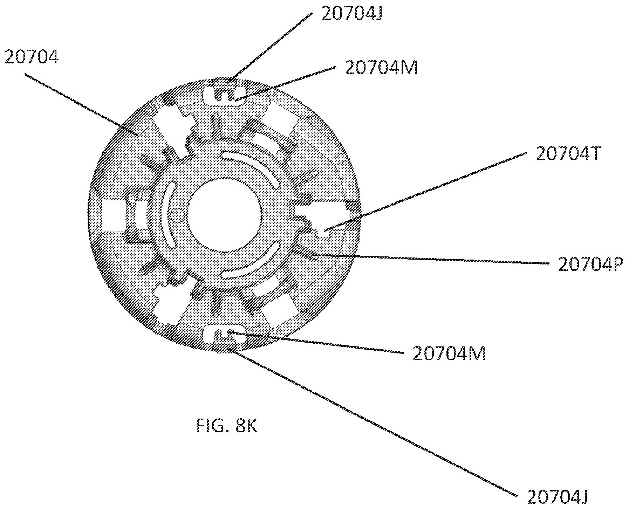

In accordance with the disclosed subject matter, sheath 20704 can include lock arms 20704J. Lock arms 20704J can be configured to engage the sensor carrier 20710 and limit movement of the sensor carrier 20710 or sheath 20704 prior to firing. The lock arms 20704J can include a free proximal end 20704K and an attached distal end 20704L. The free proximal end 20704K can include a lock arm interface 20704M disposed on an inner surface of the lock arm 20704J. The lock arm interface 20704M can engage a lock ledge 20710N on the sensor carrier 20710. For example, when cap 20708 is coupled to housing 20702, the cap 20708 can urge the lock arm 20704J inwardly, and can cause the lock arm interface 20704M to engage the sensor carrier 20710. That is, the lock arms 20704J can wedge between the cap 20708 and the sensor carrier 20710. Accordingly, the lock arm 20704J can limit proximal movement of the sheath 20704 when the cap 20708 is coupled to the housing 20702. Such engagement can limit movement of the sheath 20704 during a shock event, such as a drop. The lock arm interface 20704M can have a triangle shape when viewed in side view (e.g., FIG. 8N) and a "U" shape when viewed in top view (e.g., FIG. 8K). The shape of the lock arm interface 20704M can provide benefits during manufacturing. For example, the shape of the lock arm interface 20704M can allow the sheath 20704 to be force ejected from a mold during manufacturing of the sheath 20704. Force ejecting the sheath 20704 can allow for a more a simplified manufacture process, for example, using a one-piece mold, and can eliminate parting lines created from two-piece molds. Parting lines can result in an unsmooth surface which can catch on the sensor carrier 20710 during firing and can result in potential spikes in firing force. Accordingly, using force ejection and a one-piece mold can create a smoother lock arm interface 20704M and prevent potential spikes in firing force due to parting lines.

The proximal free end of the lock arm 20704J can further include a sharp edge 20704N on an outer surface. The sharp edge 20704N can be configured to engage crush ribs 20708F disposed on the cap 20708 during a shock event. The sharp edge 20704N can dig into the crush ribs 20708F and permanently deform the crush ribs 20708F, which can absorb energy during a shock event, and prevent sheath 20704 collapse. The shape lock arm interface 20704M can also be beneficial for drop protection. The ramp can force the lock arm 20704J to move radially as the sheath 20704 collapsed during a drop. This can force the sharp edge 20704N to dig into the crush ribs 20708F and can help to stop the sheath 20704 from collapsing. Sheath 20704 can include a plurality of lock arms 20704J, for example, two lock arms 20704J.

Additionally or alternatively, sheath 20704 can include rib 20704U configured to engage a lock interface 20710F on a sensor retention arm 20710B on the sensor carrier 20710. The rib 20704U can prevent the sensor retention arm 20710B from flexing outwardly, for example, during a shock event, and therefore can prevent movement of the sensor control device 20102 during a shock event. Rib 20704U can have a height (i.e., in the longitudinal direction) selected such that even if the sheath 20704 moves proximally or distally during a shock event, the rib 20704U will continue to engage lock interface 20710F on a sensor retention arm 20710B on the sensor carrier 20710 and prevent the sensor control device 20102 from dislodging from the sensor carrier 20710.

The sheath 20704 can include a noise damper 20704O. The noise damper 20704O can be configured to engage the sharp carrier 201102 as the sharp carrier 201102 is retracted to slow movement of the sharp carrier 201102 and can thereby reduce noise produce by the sharp carrier 201102 engaging the sheath 20704. In exemplary embodiments, the noise damper 20704O includes an angled ramp extending from the inner surface of sheath 20704, but other suitable configurations can be used.

In accordance with the disclosed subject matter, sheath 20704 can include a slot 20704Q configured to receive sharp carrier retention feature 20710L disposed on the sensor carrier 20710 and to thereby permit partial retraction of the sharp carrier 201102 during deployment (as described in greater detail below). The sheath 20704 can also include cap lead-in 20704R, alignment notch 20704S and skin interface 20704T.

### Exemplary Sensor Carriers

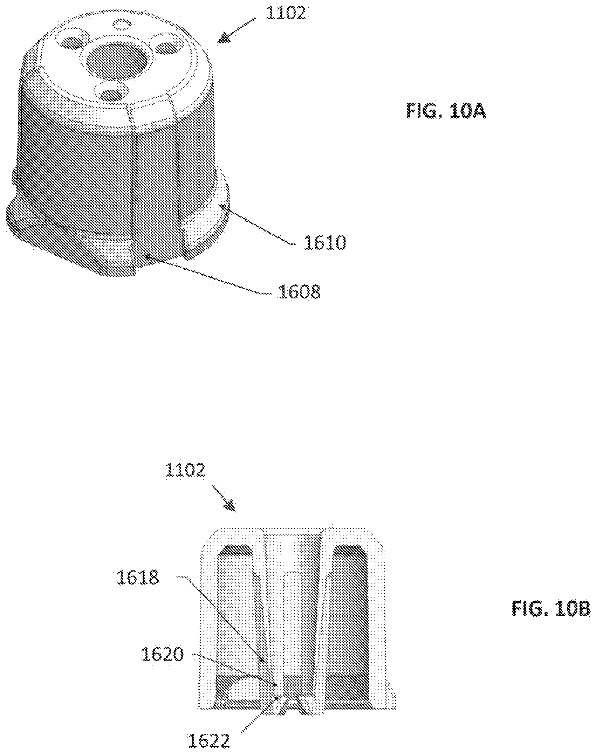

FIG. 9A is a proximal perspective view depicting an example embodiment of sensor carrier 710 that can retain sensor electronics within applicator 150. It can also retain sharp carrier 1102 with sharp module 2500. In this example embodiment, sensor carrier 710 generally has a hollow round flat cylindrical shape, and can include one or more deflectable sharp carrier lock arms 1524 (e.g., three) extending proximally from a proximal surface surrounding a centrally located spring alignment ridge 1516 for maintaining alignment of spring 1104. Each lock arm 1524 has a detent or retention feature 1526 located at or near its proximal end. Shock lock 1534 can be a tab located on an outer circumference of sensor carrier 710 extending outward and can lock sensor carrier 710 for added safety prior to firing. Rotation limiter 1506 can be a proximally extending relatively short protrusion on a proximal surface of sensor carrier 710 which limits rotation of carrier 710. Sharp carrier lock arms 1524 can interface with sharp carrier 1102 as described with reference to FIGS. 10A-10E below.

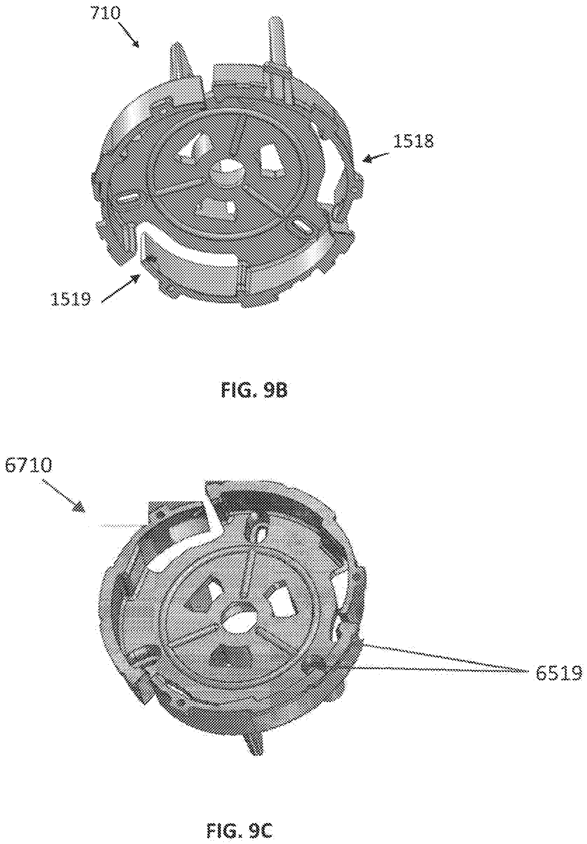

FIG. 9B is a distal perspective view of sensor carrier 710. Here, one or more sensor electronics retention spring arms 1518 (e.g., three) are normally biased towards the position shown and include a detent 1519 that can pass over the distal surface of electronics housing 706 of device 102 when housed within recess or cavity 1521. In certain embodiments, after sensor control device 102 has been adhered to the skin with applicator 150, the user pulls applicator 150 in a proximal direction, i.e., away from the skin. The adhesive force retains sensor control device 102 on the skin and overcomes the lateral force applied by spring arms 1518. As a result, spring arms 1518 deflect radially outwardly and disengage detents 1519 from sensor control device 102 thereby releasing sensor control device 102 from applicator 150.

FIG. 9C is a perspective view of an alternative example embodiment of sensor carrier 6710. As shown in FIG. 9C, sensor carrier 6710 can have many of the same features as sensor carrier 710, previously described with respect to FIGS. 9A-9B. In addition, sensor carrier 6710 also includes one or more notch ribs 6519 disposed along an outer circumferential surface. As best seen in FIGS. 8F-8H, notch ribs 6519 are configured to interface with inner sheath ribs 6425 in order to maintain axial alignment of the sheath and sensor carrier, and reduce lateral and rotational movement between applicator components during the sensor insertion process.

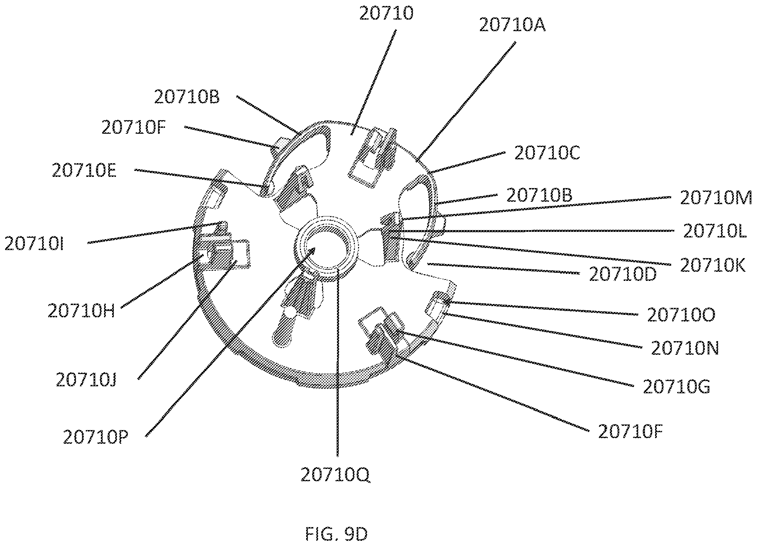

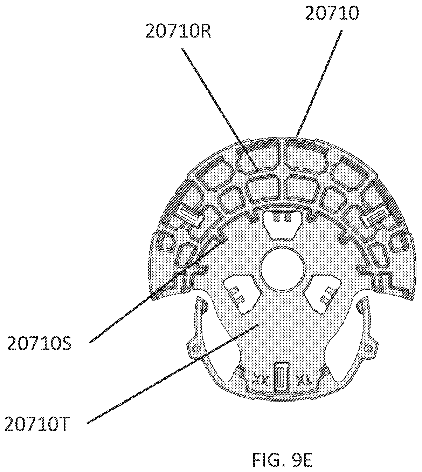

Referring to FIGS. 9D and 9E, for purpose of illustration and not limitation, an exemplary sensor carrier 20710 is provided. Sensor carrier 20710 can include one or more of the features described herein with regard to sensor carriers, wherein similar features can operate as described herein. For example, sensor carrier 20710 can include a base 20710A and first and second retention arms 20710B. Each retention arm 20710B can include a first end portion 20710C coupled to the base 20710A and a free end portion 20710D. For example, each retention arm 20710B can be coupled to the base 20710A at a first half of the base 20710A and the free end portion 20710D can extend toward a second half of the base 20710A. Each retention arm 20710B can include a sensor retention feature 20710E disposed on an inner surface of the sensor retention arm 20710B. The sensor retention feature 20710E can be disposed on the free end portion 20710D. The sensor retention feature 20710E can be configured to retain the sensor control device 20102 within the housing 20702. The retention feature 20710E can include a conical surface and angular parting line, which can allow for release of the sensor control device 20102 upon delivery. Each retention arm 20710 can include a lock interface 20710F disposed on an outer surface of the retention arm 20710B. The lock interface 20710F can engage rib 20704U on the sheath 20704. As described hereinabove, the rib 20704U can prevent the sensor retention arm 20710B from flexing outwardly, for example, during a shock event, and therefore can keep retention feature 20710E engaged with the sensor control device 20102, and thereby prevent movement of the sensor control device 20102 during a shock event.

Sensor carrier 20710 can include a plurality of housing attachment features 20710F, for example three housing attachment features 20710F. The housing attachment features 20710F can be equally spaced on the sensor carrier 20710 and can extend upwardly from a top surface of the sensor carrier 20710. Each sensor housing attachment feature 20710F can include a housing snap 20710G, housing locator feature 20710H, biasing feature 20710I, and housing stop 20710J. The housing locator feature 20710H can locate the sensor carrier 20710 relative the housing 20702 when the two are to be coupled together. The housing snap 20710G can engage the sensor carrier attach slots 20702K on the housing 20702 to couple the sensor carrier 20710 to the housing 20702. The biasing feature 20710I can engage sensor carrier biasing feature 20702M on housing 20702 configured to remove slop between the sensor carrier 20710 and the housing 20702. Housing stop 20710J can engage sensor carrier hard stop 20702L on sheath guide rails 20702J on housing 20702 to locate the sensor carrier 20710 axially relative to the housing 20702.

Sensor carrier 20710 can further include a plurality of sharp carrier lock arms 20710K, for example three sharp carrier lock arms 20710K. The sharp carrier lock arms 20710K can be equally spaced on the sensor carrier 20710 and can extend upwardly form a top surface of the sensor carrier 20710. Each sharp carrier lock arm 20710K can include a sharp carrier retention feature 20710L and a rib 20710M. Rib 20710M can engage an inner surface of the sheath 20704, which can urge the sharp carrier lock arm 20710K inwardly and cause sharp carrier retention feature 20710L to retain sharp carrier 201102, as described in greater detail below. The carrier retention feature 20710L can have a triangle shape when viewed in side view and a "U" shape when viewed in top view.

In accordance with the disclosed subject matter, the sensor carrier 20710 can include a plurality of lock ledges 20710N configured to engage lock arm interface 20704M of the sheath 20704 as described herein above. For example, the sensor carrier 20710 can include two lock ledges 20710N. Sensor carrier 20710 can include recesses 207100 disposed proximate each lock ledge 20710N and configured to receive the lock arm interface 20704M during firing, to prevent the lock arm 20704J from engaging with housing 20702 during firing. Sensor carrier 20710 can include a hole 20710P extending through a middle of the base 20710A. The hole 20710P can guide and limit movement of sharp hub 205014 during insertion. Additionally, or alternatively , sensor carrier 20710 can include spring locator 20710Q.

A bottom surface of the sensor carrier 20710 can include stiffening ribs 20710R and sensor locator ribs 20710S, which can limit planar motion of the sensor control device 20102 relative the sensor carrier 20710. The bottom surface of the sensor carrier 20710 can include a sensor support surface 20710T configure to support the sensor control device 20102.

### Exemplary Sharp Carriers

FIGS. 10A and 10B are a proximal perspective view and a side cross-sectional view, respectively, depicting an example embodiment of sharp carrier 1102. Sharp carrier 1102 can grasp and retain sharp module 2500 within applicator 150. It can also automatically retract as a result of one or more springs changing from a preloaded, compressed state to an expanded state during an insertion process, as described with respect to FIGS. 40A-40F. Near a distal end of sharp carrier 1102 can be anti-rotation slots 1608 which prevent sharp carrier 1102 from rotating when located within a central area of sharp carrier lock arms 1524 (as shown in FIG. 9A). Anti-rotation slots 1608 can be located between sections of sharp carrier base chamfer 1610, which can ensure full retraction of sharp carrier 1102 through sheath 704 upon retraction of sharp carrier 1102 at the end of the deployment procedure.

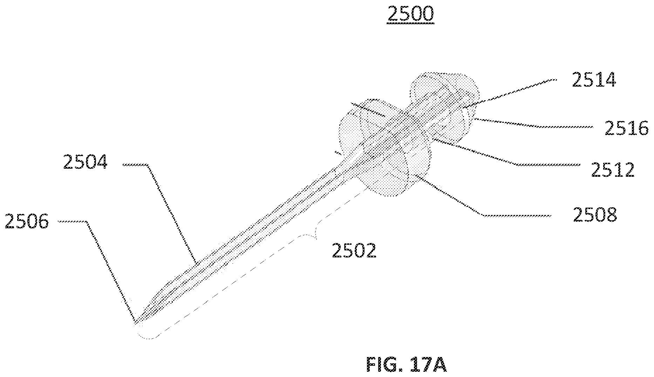

As shown in FIG. 10B, sharp retention arms 1618 can be located in an interior of sharp carrier 1102 about a central axis and can include a sharp retention clip 1620 at a distal end of each arm 1618. Sharp retention clip 1620 can have a proximal surface which can be nearly perpendicular to the central axis and can abut a distally facing surface of sharp hub 2516 (FIG. 17A).

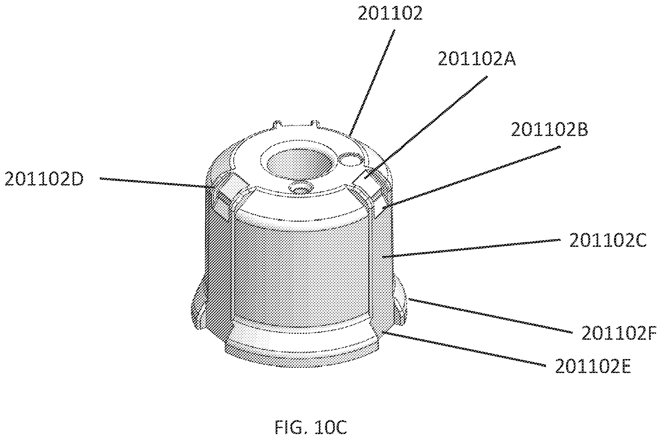

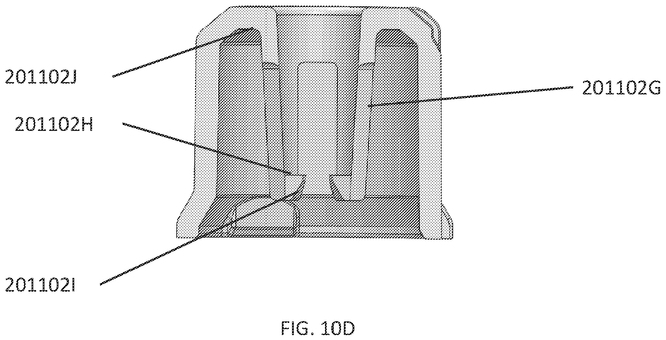

Referring to FIGs. 10C and 10D, for purpose of illustration and not limitation, an exemplary sharp carrier 201102 is provided. Sharp carrier 201102 can include one or more features described herein with regard to sharp carriers, wherein similar features can operate as describe herein. For example, sharp carrier 201102 can include a series of features for engaging with the three sharp carrier lock arms 20710K of the sensor carrier 20710. The features can include a pre-partial-retraction retention face 201102A and a post-partial-retraction retention face 201102B. The pre-partial retraction retention face 201102A can engage the sharp carrier retention feature 20710L prior to partial retraction, for example, during shipping and storage. Post-partial-retraction retention face 201102B can engage the sharp carrier retention feature 20710L after partial retraction. For example, as the sheath 20704 initially move proximally relative to the sensor carrier 20710, the rib 20710M of the retention arm 20710L can engage slot 20704Q of sheath 20704, which can allow the retention arm 20710L to move radially outward and allow sharp carrier retention feature 20710L to clear the pre-partial retraction retention face 201102A and engage the post-partial retraction retention face 201102B. A height between the end of the pre-partial-retraction face 201102A and the start of the post-partial-retraction face 201102B can be the distance of the partial retraction. A running face 201102C can be disposed below the post-partial-retraction retention face 201102B and can slide against the retention arm 20710L as the sharp carrier 201102 is retracted. Alignment walls 201102D can help to keep the sharp carrier 201102 aligned with the sensor carrier 20704 during partial retraction. Sharp carrier 201102 can include a chamfer 201102F, which can include anti-rotation slots 201102E to engage the retention arms 20710L on the sensor carrier 20710.

Internally, sharp carrier 201102 can include sharp retention arms 201102G including lead-in face 201102I and sharp hub contact face 201102H. The retention arms 201102G can receive and hold sharp hub 205014. Spring stop 201102J can engage retraction spring 205612.

Spring 205612 can include any type of spring known in the art, such as a helical spring. For example, according to certain embodiments, spring 205612 may include a helical spring constructed of stainless steel. Spring 205612 may include a spring constant of any suitable range, and a wire diameter, an inner diameter, an outer diameter, and a maximum solid strength of any suitable dimension. For example, the spring constant can be about 0.12, the wire diameter can be about 0.65 millimeters, the inner diameter can be about 9.6 millimeter, the outer diameter can be about 11.1 millimeters, and the maximum solid strength can be 11 millimeters. It is noted that each of the parameter values listed above may be provided independently without requiring a particular embodiment of the spring to fulfill all of the listed parameter values. For example, a spring 205612 may be provided having a spring constant of about 0.12 while having different dimensions to those listed above, or a spring 205612 may have a different wire diameter while having the other highlighted dimensions.

### Exemplary Sensor Modules

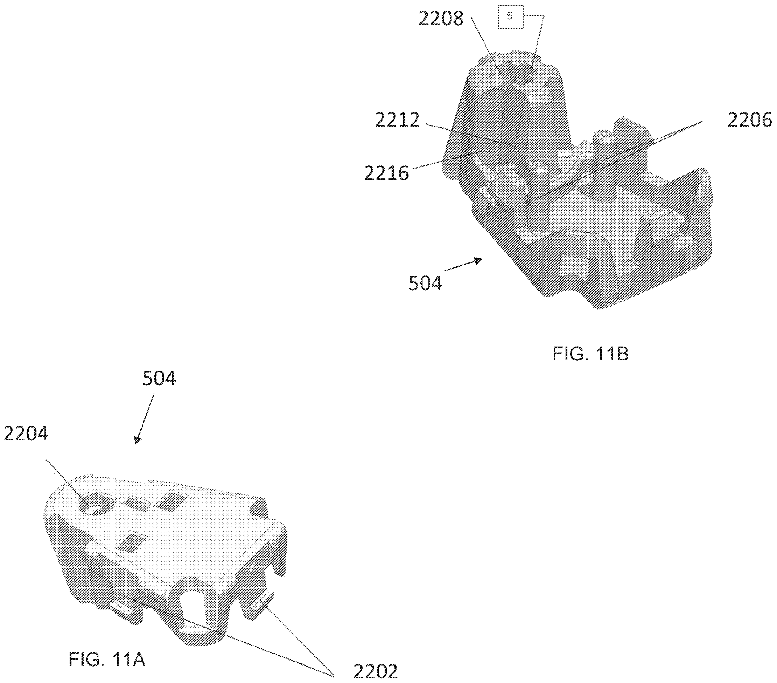

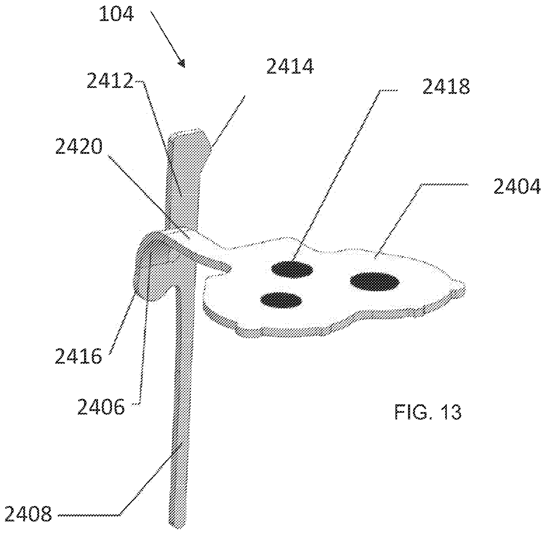

FIGS. 11A and 11B are a top perspective view and a bottom perspective view, respectively, depicting an example embodiment of sensor module 504. Module 504 can hold a connector 2300 (FIGS. 12A and 12B) and a sensor 104 (FIG. 13). Module 504 is capable of being securely coupled with electronics housing 706. One or more deflectable arms or module snaps 2202 can snap into the corresponding features 2010 of housing 706. A sharp slot 2208 can provide a location for sharp tip 2502 to pass through and sharp shaft 2504 to temporarily reside. A sensor ledge 2212 can define a sensor position in a horizontal plane, prevent a sensor from lifting connector 2300 off of posts and maintain sensor 104 parallel to a plane of connector seals. It can also define sensor bend geometry and minimum bend radius. It can limit sensor travel in a vertical direction and prevent a tower from protruding above an electronics housing surface and define a sensor tail length below a patch surface. A sensor wall 2216 can constrain a sensor and define a sensor bend geometry and minimum bend radius.

FIGS. 12A and 12B are perspective views depicting an example embodiment of connector 2300 in an open state and a closed state, respectively. Connector 2300 can be made of silicone rubber that encapsulates compliant carbon impregnated polymer modules that serve as electrical conductive contacts 2302 between sensor 104 and electrical circuitry contacts for the electronics within housing 706. The connector can also serve as a moisture barrier for sensor 104 when assembled in a compressed state after transfer from a container to an applicator and after application to a user's skin. A plurality of seal surfaces 2304 can provide a watertight seal for electrical contacts and sensor contacts. One or more hinges 2208 can connect two distal and proximal portions of connector 2300.

FIG. 13 is a perspective view depicting an example embodiment of sensor 104. A neck 2406 can be a zone which allows folding of the sensor, for example ninety degrees. A membrane on tail 2408 can cover an active analyte sensing element of the sensor 104. Tail 2408 can be the portion of sensor 104 that resides under a user's skin after insertion. A flag 2404 can contain contacts and a sealing surface. A biasing tower 2412 can be a tab that biases the tail 2408 into sharp slot 2208. A bias fulcrum 2414 can be an offshoot of biasing tower 2412 that contacts an inner surface of a needle to bias a tail into a slot. A bias adjuster 2416 can reduce a localized bending of a tail connection and prevent sensor trace damage. Contacts 2418 can electrically couple the active portion of the sensor to connector 2300. A service loop 2420 can translate an electrical path from a vertical direction ninety degrees and engage with sensor ledge 2212 (FIG. 11B).

FIGS. 14A and 14B are bottom and top perspective views, respectively, depicting an example embodiment of a sensor module assembly comprising sensor module 504, connector 2300, and sensor 104. According to one aspect of the aforementioned embodiments, during or after insertion, sensor 104 can be subject to axial forces pushing up in a proximal direction against sensor 104 and into the sensor module 504, as shown by force, F1, of FIG. 14A. According to some embodiments, this can result in an adverse force, F2, being applied to neck 2406 of sensor 104 and, consequently, result in adverse forces, F3, being translated to service loop 2420 of sensor 104. In some embodiments, for example, axial forces, F1, can occur as a result of a sensor insertion mechanism in which the sensor is designed to push itself through the tissue, a sharp retraction mechanism during insertion, or due to a physiological reaction created by tissue surrounding sensor 104 (e.g., after insertion).

FIGS. 15A and 15B are close-up partial views of an example embodiment of a sensor module assembly having certain axial stiffening features. In a general sense, the embodiments described herein are directed to mitigating the effects of axial forces on the sensor as a result of insertion and/or retraction mechanisms, or from a physiological reaction to the sensor in the body. As can be seen in FIGS. 15A and 15B, according to one aspect of the embodiments, sensor 3104 comprises a proximal portion having a hook feature 3106 configured to engage a catch feature 3506 of the sensor module 3504. In some embodiments, sensor module 3504 can also include a clearance area 3508 to allow a distal portion of sensor 3104 to swing backwards during assembly to allow for the assembly of the hook feature 3106 of sensor 3104 over and into the catch feature 3506 of sensor module 3504.

According to another aspect of the embodiments, the hook and catch features 3106, 3506 operate in the following manner. Sensor 3104 includes a proximal sensor portion, coupled to sensor module 3504, as described above, and a distal sensor portion that is positioned beneath a skin surface in contact with a bodily fluid. As seen in FIGS. 15A and 15B, the proximal sensor portion includes a hook feature 3106 adjacent to the catch feature 3506 of sensor module 3504. During or after sensor insertion, one or more forces are exerted in a proximal direction along a longitudinal axis of sensor 3104. In response to the one or more forces, hook feature 3106 engages catch feature 3506 to prevent displacement of sensor 3104 in a proximal direction along the longitudinal axis.

According to another aspect of the embodiments, sensor 3104 can be assembled with sensor module 3504 in the following manner. Sensor 3104 is loaded into sensor module 3504 by displacing the proximal sensor portion in a lateral direction to bring the hook feature 3106 in proximity to the catch feature 3506 of sensor module 3504. More specifically, displacing the proximal sensor portion in a lateral direction causes the proximal sensor portion to move into clearance area 3508 of sensor module 3504.

Although FIGS. 15A and 15B depict hook feature 3106 as a part of sensor 3104, and catch feature 3506 as a part of sensor module 3504, those of skill in the art will appreciate that hook feature 3106 can instead be a part of sensor module 3504, and, likewise, catch feature 3506 can instead be a part of sensor 3106. Similarly, those of skill in the art will also recognize that other mechanisms (e.g., detent, latch, fastener, screw, etc.) implemented on sensor 3104 and sensor module 3504 to prevent axial displacement of sensor 3104 are possible and within the scope of the present disclosure.

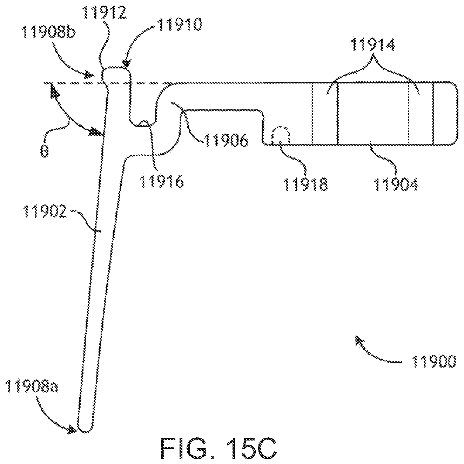

FIG. 15C is a side view of an example sensor 11900, according to one or more embodiments of the disclosure. The sensor 11900 may be similar in some respects to any of the sensors described herein and, therefore, may be used in an analyte monitoring system to detect specific analyte concentrations. As illustrated, the sensor 11900 includes a tail 11902, a flag 11904, and a neck 11906 that interconnects the tail 11902 and the flag 11904. The tail 11902 includes an enzyme or other chemistry or biologic and, in some embodiments, a membrane may cover the chemistry. In use, the tail 11902 is transcutaneously received beneath a user's skin, and the chemistry included thereon helps facilitate analyte monitoring in the presence of bodily fluids.

The tail 11902 may be received within a hollow or recessed portion of a sharp (not shown) to at least partially circumscribe the tail 11902 of the sensor 11900. As illustrated, the tail 11902 may extend at an angle Q offset from horizontal. In some embodiments, the angle Q may be about 85°. Accordingly, in contrast to other sensor tails, the tail 11902 may not extend perpendicularly from the flag 11904, but instead at an angle offset from perpendicular. This may prove advantageous in helping maintain the tail 11902 within the recessed portion of the sharp.

The tail 11902 includes a first or bottom end 11908a and a second or top end 11908b opposite the bottom end 11908a. A tower 11910 may be provided at or near the top end 11908b and may extend vertically upward from the location where the neck 11906 interconnects the tail 11902 to the flag 11904. During operation, if the sharp moves laterally, the tower 11910 will help pivot the tail 11902 toward the sharp and otherwise stay within the recessed portion of the sharp. Moreover, in some embodiments, the tower 11910 may provide or otherwise define a protrusion 11912 that extends laterally therefrom. When the sensor 11900 is mated with the sharp and the tail 11902 extends within the recessed portion of the sharp, the protrusion 11912 may engage the inner surface of the recessed portion. In operation, the protrusion 11912 may help keep the tail 11902 within the recessed portion.

The flag 11904 may comprise a generally planar surface having one or more sensor contacts 11914 arranged thereon. The sensor contact(s) 11914 may be configured to align with a corresponding number of compliant carbon impregnated polymer modules encapsulated within a connector.

In some embodiments, as illustrated, the neck 11906 may provide or otherwise define a dip or bend 11916 extending between the flag 11904 and the tail 11902. The bend 11916 may prove advantageous in adding flexibility to the sensor 11900 and helping prevent bending of the neck 11906.

In some embodiments, a notch 11918 (shown in dashed lines) may optionally be defined in the flag near the neck 11906. The notch 11918 may add flexibility and tolerance to the sensor 11900 as the sensor 11900 is mounted to the mount. More specifically, the notch 11918 may help take up interference forces that may occur as the sensor 11900 is mounted within the mount.

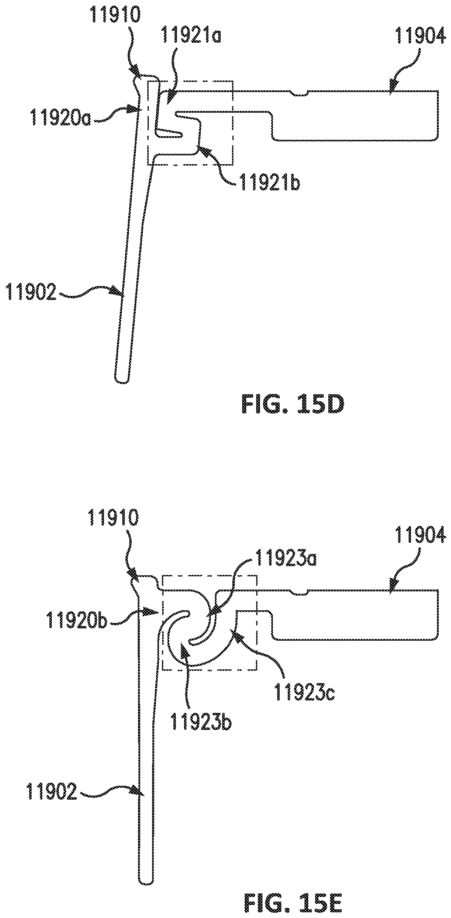

In some embodiments, as illustrated in FIGs. 15D-15G, the neck can comprise or otherwise define a non-linear configuration such as a dip or bend 11920a-11920d with a plurality of turns, e.g., 11921a, 11921b, extending between the flag 11904 and the tail 11902. The bend 11920a-11920d can be advantageous in reducing in-place stiffness of the sensor 11900 by adding flexibility to the sensor 11900 in both a vertically-oriented and horizontally-oriented direction. The added flexibility can provide a multi-directional spring-like structure in the sensor 11900 that helps to limit deformation of the neck 11906 while ensuring that the tail 11902 and the flag 11904 can remain in their expected or fixed positions. The spring-like structure also increases compliance of the sensor 11900 while reducing stress on the overall structure.

Generally, the sensor can be understood as including a tail, a flag, and a neck aligned along a planar surface having a vertical axis and a horizontal axis. The spring-like structure can be created by various orientations of turns in the bend of the neck of a sensor. Between the tail and the flag, the neck can include at least two turns in relation to the vertical axis providing a spring-like structure. The at least two turns can provide, in relation to an axis of the planar surface shared by the tail, the flag, and the neck, overlapping layers of the structure of the neck, where the neck itself remains unbroken. These overlapping turns make up the spring-like structure. In some embodiments, the overlapping layers of the neck are vertically-oriented. In some embodiments, the overlapping layers of the neck are horizontally-oriented.

FIG. 15D illustrates one embodiment of a sensor 11900 including a neck between the flag 11904 and tail 11902 with a bend 11920a including turns 11921a and 11921b. In the illustrated embodiment, at least one turn 11921a abuts the top end of the tail or possibly the tower 11910 of the sensor 11900. This orientation can be advantageous in that it reduces the overall footprint of the sensor, even considering the additional material used to generate the bend 11920a. The arrangement can provide multiple overlapping, vertically-aligned horizontal layers between the turns.

FIG. 15E illustrates another embodiment of a sensor 11900 including a neck between the flag 11904 and tail 11902 with a bend 11920b that generally forms a swirl pattern including at least turn turns 11923a, 11923b, and 11923c. In this embodiment, the turns again abut the top end of the tail or the tower 11910 of the sensor 11900. In addition to maintaining the overall footprint of the sensor, this orientation may provide for additional balancing of the horizontally-oriented and vertically-oriented stresses. The overlapping layers in this arrangement of turns are substantially balanced in along both the horizontal and vertical axes.

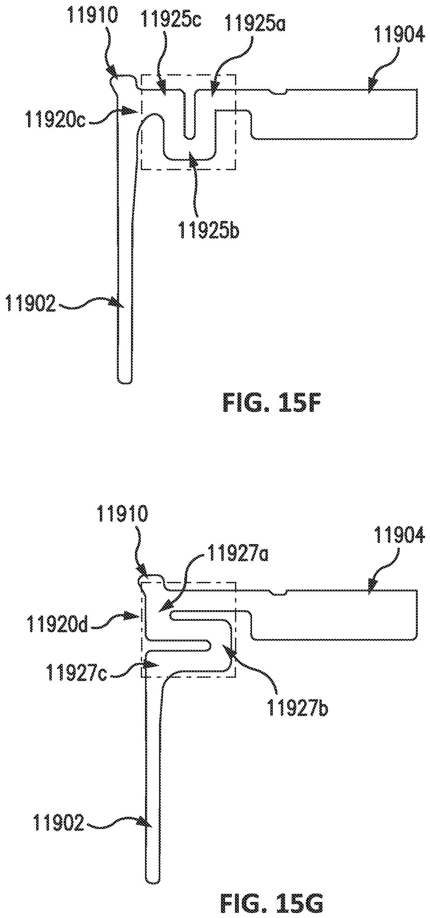

FIG. 15F illustrates another embodiment of a sensor 11900 including a neck between the flag 11904 and tail 11902 with a bend 11920c including turns 11925a, 11925b, and 11925c. In the illustrated embodiment, the turn 11925c connects a region of the tail 11902 near the top end of the tail or the tower 11910 of the sensor to the rest of the bend 11920c. In addition to reducing the overall footprint of the sensor, this orientation can be considered to provide additional flexibility in the horizontally-oriented axis. The arrangement can provide multiple overlapping, horizontally-aligned vertical layers between the turns.

FIG. 15G illustrates another embodiment of a sensor 11900 including a neck between the flag 11904 and tail 11902 with a bend 11920d including turn 11927a, 11927b, and 11927c. In the illustrated embodiment, the bend 11920d occurs primarily in the tail 11902 of the sensor, connecting the tail 11902 and the tower 11910, while the stretch of the sensor between the tower 11910 and the flag 11904 is generally uninterrupted. The turn 11927a generally connects the tower 11910 to the rest of the bend 11920d, while the turn 11927c connects the tail 11902 to the rest of the bend 11920d. This orientation can be considered to provide additional flexibility in the vertically-oriented axis. The arrangement can provide multiple overlapping, horizontally-aligned vertical layers between the turns.

The turns of the neck can be created by folding the neck of the sensor from a larger neck structure, laser cutting the sensor from a sheet of the material comprising the sensor, printing the sensor having the configuration with turns, stamping the sensor from a sheet of material of which the sensor is composed, or other suitable manufacturing processes for providing precision bends in the neck.

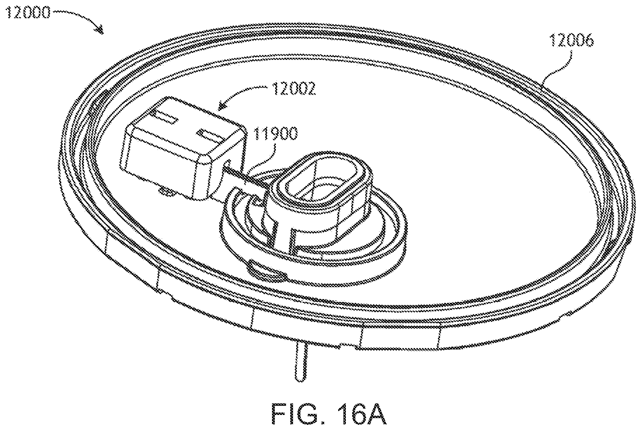

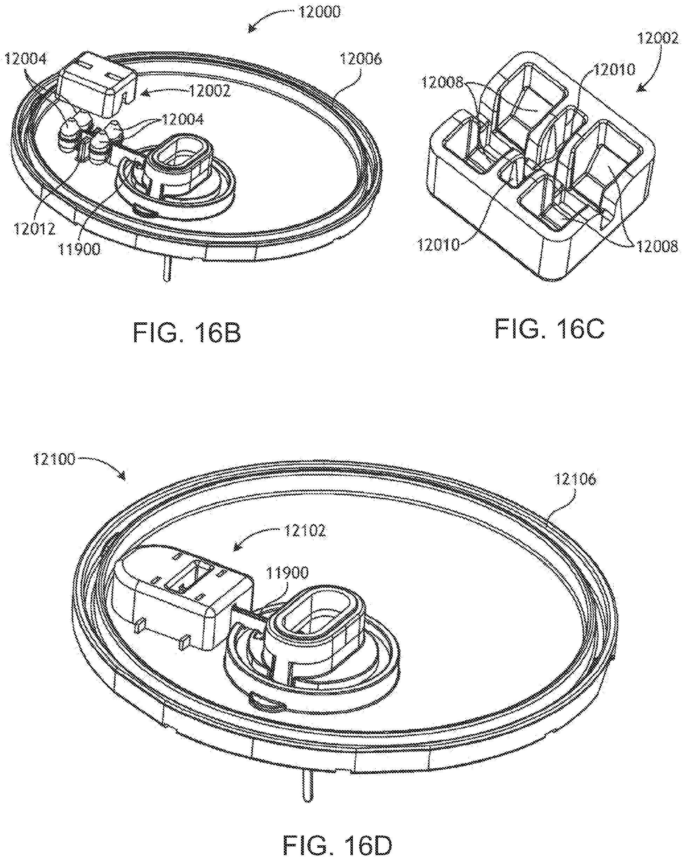

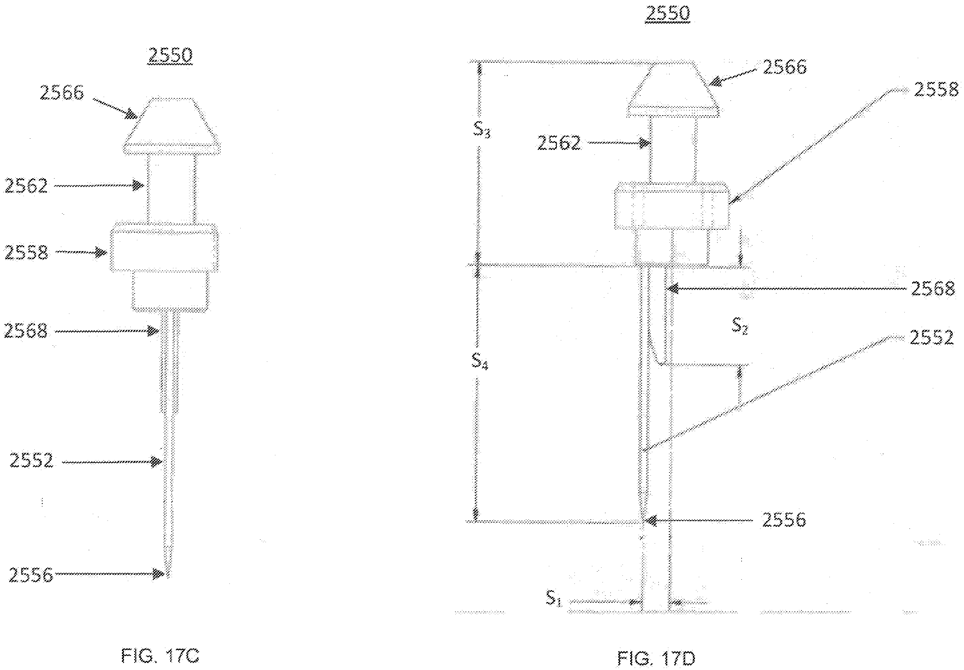

FIGS. 16A and 16B are isometric and partially exploded isometric views of an example connector assembly 12000, according to one or more embodiments. As illustrated, the connector assembly 12000 may include a connector 12002, and FIG. 17C is an isometric bottom view of the connector 12002. The connector 12002 may comprise an injection molded part used to help secure one or more compliant carbon impregnated polymer modules 12004 (four shown in FIG. 16B) to a mount 12006. More specifically, the connector 12002 may help secure the modules 12004 in place adjacent the sensor 11900 and in contact with the sensor contacts 11914 (FIG. 15C) provided on the flag 11904 (FIG. 15C). The modules 12004 may be made of a conductive material to provide conductive communication between the sensor 11900 and corresponding circuitry contacts (not shown) provided within the mount 12006.

As best seen in FIG. 16C, the connector 12002 may define pockets 12008 sized to receive the modules 12004. Moreover, in some embodiments, the connector 12002 may further define one or more depressions 12010 configured to mate with one or more corresponding flanges 12012 (FIG. 16B) on the mount 12006. Mating the depressions 12010 with the flanges 12012 may secure the connector 12002 to the mount 12006 via an interference fit or the like. In other embodiments, the connector 12002 may be secured to the mount 12006 using an adhesive or via sonic welding.

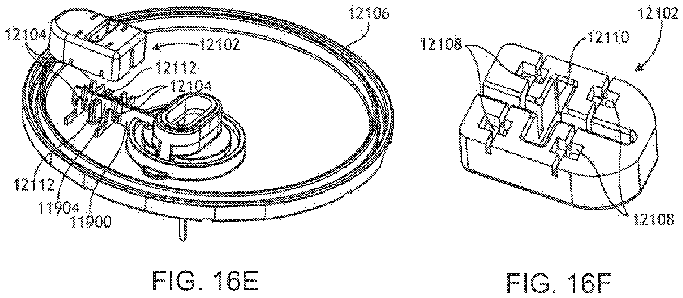

FIGS. 16D and 16E are isometric and partially exploded isometric views of another example connector assembly 12100, according to one or more embodiments. As illustrated, the connector assembly 12100 may include a connector 12102, and FIG. 16F is an isometric bottom view of the connector 12102. The connector 12102 may comprise an injection molded part used to help keep one or more compliant metal contacts 12104 (four shown in FIG. 16E) secured against the sensor 11900 on a mount 12106. More specifically, the connector 12102 may help secure the contacts 12104 in place adjacent the sensor 11900 and in contact with the sensor contacts 11914 (FIG. 15C) provided on the flag 11904. The contacts 12104 may be made of a stamped conductive material that provides conductive communication between the sensor 11900 and corresponding circuitry contacts (not shown) provided within the mount 12106. In some embodiments, for example, the contacts 12104 may be soldered to a PCB (not shown) arranged within the mount 12106.

As best seen in FIG. 16F, the connector 12102 may define pockets 12108 sized to receive the contacts 12104. Moreover, in some embodiments, the connector 12102 may further define one or more depressions 12110 configured to mate with one or more corresponding flanges 12112 (FIG. 120B) on the mount 12006. Mating the depressions 12110 with the flanges 12112 may help secure the connector 12102 to the mount 12106 via an interference fit or the like. In other embodiments, the connector 12102 may be secured to the mount 12106 using an adhesive or via sonic welding.

### Exemplary Sharp Modules

FIG. 17A is a perspective view depicting an example embodiment of sharp module 2500 prior to assembly within sensor module 504 (FIG. 6B). Sharp 2502 can include a distal tip 2506 which can penetrate the skin while carrying sensor tail in a hollow or recess of sharp shaft 2504 to put the active surface of the sensor tail into contact with bodily fluid. A hub push cylinder 2508 can provide a surface for a sharp carrier to push during insertion. A hub small cylinder 2512 can provide a space for the extension of sharp hub contact faces 1622 (FIG. 10B). A hub snap pawl locating cylinder 2514 can provide a distal-facing surface of hub snap pawl 2516 for sharp hub contact faces 1622 to abut. A hub snap pawl 2516 can include a conical surface that opens clip 1620 during installation of sharp module 2500.

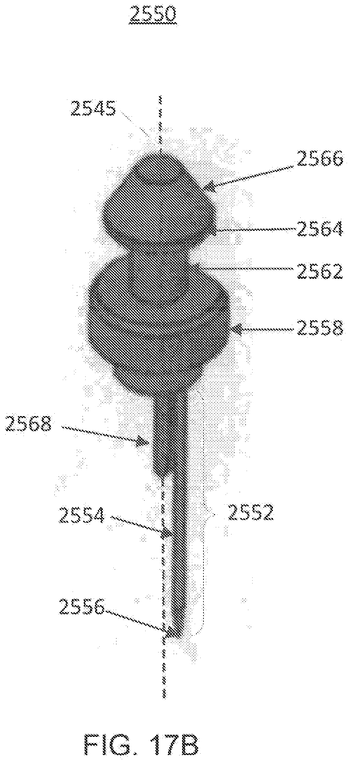

FIGS. 17B to 17H show example embodiments of sharp modules, in various stages of assembly, for use in the insertion of dermal analyte sensors. According to one aspect of the embodiments, angling the sensor and/or insertion sharp relative to a reference point can enable co-localization of the tip of the insertion needle and the tip of the sensor, and furthermore, can create a single contact point at the surface of the skin. As such, the sharp can create a leading edge at the surface of the skin to form an insertion path into the dermal layer for the sensor, as the sensor is inserted into a subject. In some embodiments, for example, the sharp and/or dermal sensor may be angled relative to a reference point (e.g., each other, surface of the skin, or the base of the applicator) for insertion, where the angle of the sharp differs from the angle of the sensor. For example, the reference point may be the skin surface to be breached for dermal insertion, or may be a reference or component of the sensor applicator set. In some embodiments, the sharp may be disposed at an angle relative to the sensor. For example, when designed so that that the sharp is angled relative to the sensor, the needle creates a leading edge for the sensor during operation of the applicator set. Furthermore, the needle design itself, and the positioning of the needle with respect to the sensor can be implemented in any desired configuration, including all of those configurations disclosed in U.S. Patent Publication No. 2014/0171771.

Furthermore, although many of the example embodiments described with respect to FIGS. 17B to 17J make reference to dermal analyte sensors and dermal insertion, it will be understood by those of skill in the art that any of the embodiments can be dimensioned and configured for use with analyte sensors that can be positioned beyond the dermal space, such as into (or even fully through) subcutaneous tissue (e.g., 3 mm to 10 mm beneath the surface of the skin depending on the location of the skin on the body).

FIG. 17B is a perspective view depicting an example embodiment of a sharp module 2550 that can be used for the insertion of a dermal sensor. Sharp module 2550 is shown here prior to assembly with sensor module 504 (FIG. 6B), and can include components similar to those of the embodiment described with respect to FIG. 17A, including sharp 2552, sharp shaft 2554, sharp distal tip 2556, hub push cylinder 2558, hub small cylinder 2562, hub snap pawl 2566 and hub snap pawl locating cylinder 2564. Sharp 2552 can be positioned within sharp module 2550 at an off-center location relative to a longitudinal axis 2545 that extends through center of hub snap pawl 2566, hub small cylinder 2562 and hub push cylinder 2558. In addition, sharp module 2550 can include a sharp spacer 2568 that is parallel to and adjacent with a portion of sharp 2552. Sharp spacer 2568 can be positioned in between sensor 104 (not shown) and sharp 2552 along a proximal portion of sharp 2552, and can ensure that sensor 104 and sharp 2552 remain spaced apart at a proximal portion of sharp 2552. Sharp 2552 can be positioned in an off-center location during a molding process with hub components 2558, 2562, 2566, each of which may consist of a rigid plastic material.

FIGS. 17C and 17D are two side views depicting sharp module 2550 prior to assembly with sensor module 504 (FIG. 6B), and include sharp 2552, spacer 2568, hub push cylinder 2558, hub small cylinder 2562 and hub snap pawl 2566. In some embodiments, the relative distances between the sharp 2552 and hub components can be positioned as follows. For example, distance, Si, between the sharp 2552 and the radial center of hub can range from 0.50 mm to 1 mm (e.g., 0.89 mm). Height, S₂, of sharp spacer 2568 can range from 3 to 5 mm (e.g., 3.26 mm). Height, S₃, of hub can range from 5 to 10 mm (e.g., 6.77 mm). Length, S₄, of sharp 2552 can range from 1.5 mm to 25 mm (e.g., 8.55 mm), and may depend on the location of the insertion site on the subject.

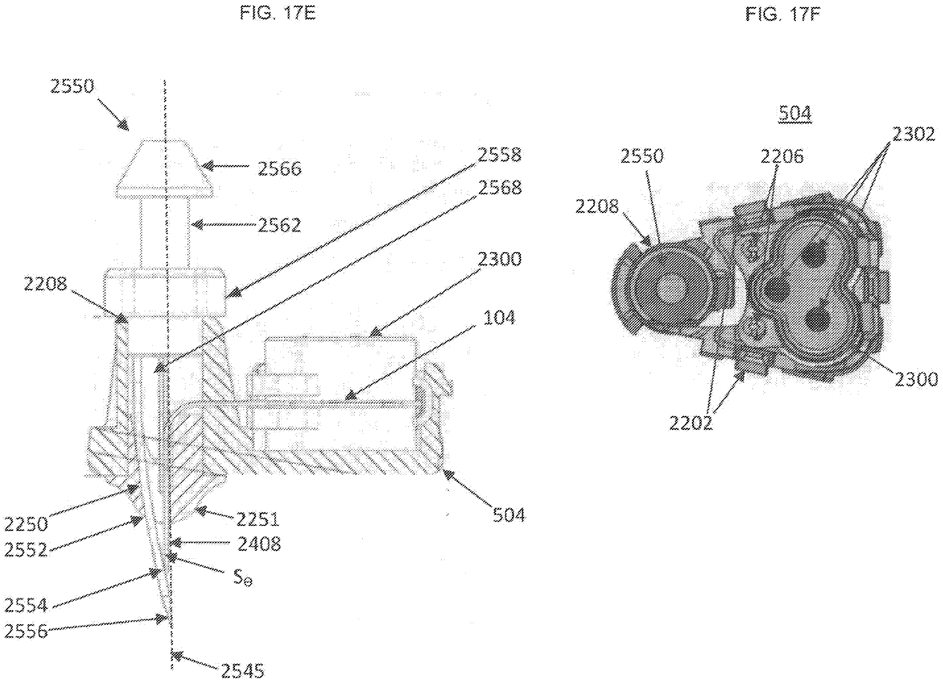

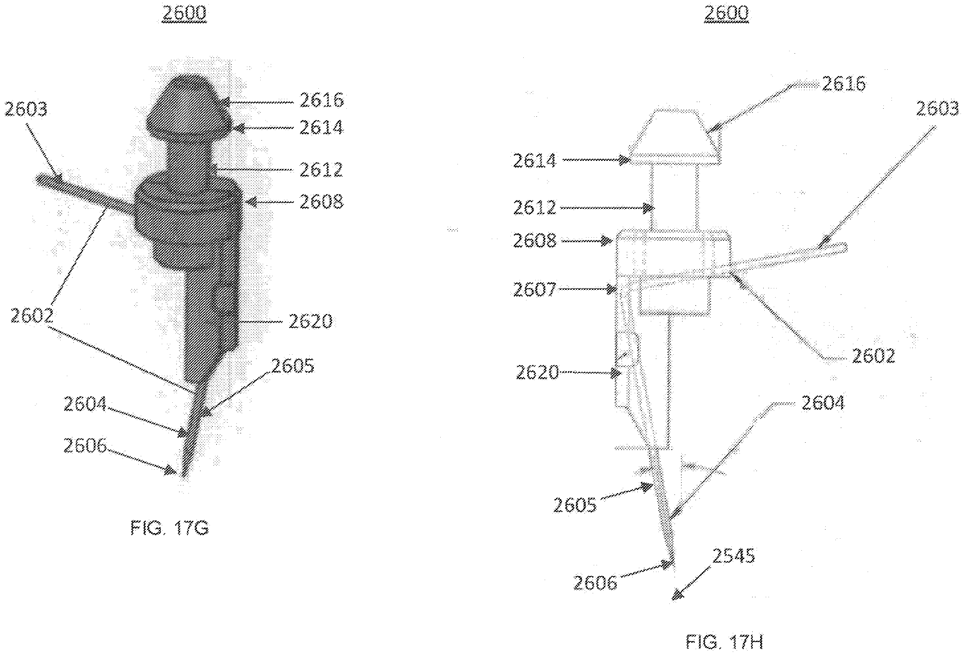

FIG. 17E depicts a side cross-sectional side view of sharp module 2550, including sharp 2552, sharp spacer 2568 and hub components (hub snap pawl 2566, hub small cylinder 2562, and hub push cylinder 2558), as assembled with sensor module 504. As can be seen in FIG. 17E, sharp 2552 is positioned within sharp slot 2208 of sensor module 504 that includes a curved interior surface 2250, located at a distal end. Curved interior surface 2250 of sensor module 504 can be in contact with a portion of sharp 2552 and cause a deflection such that sharp distal tip 2556 is oriented toward central longitudinal axis 2545. As best seen in FIG. 17H, sharp 2552 can be positioned such that the distal portion and central longitudinal axis 2545 form an acute angle, So, that can range between 5° and 20°. In some embodiments, for example, So, can range from 5° to 17°, or 7° to 15°, or 9° to 13°, e.g., 9°, 10°, 11°, 12°, or 13°

Referring still to FIG. 17E, near a distal end of sensor module 504 is protrusion 2251, which can enhance the perfusion of bodily fluid, such as dermal fluid. Although shown as a curved surface in FIG. 17E, protrusion 2251 can be shaped in any desired fashion. In addition, in some embodiments, multiple protrusions can be present. U.S. Patent Publication No. 2014/0275907, describes sensor devices having different protrusion configurations, each of which can be implemented with the embodiments described herein. Many of the embodiments described herein show the needle exiting from the protrusion, and in other embodiments, the needle can exit from the base of the sensor device adjacent the protrusion, and from that position extend over the tip of sensor 104.

Referring still to FIGS. 17E and 17F, sensor 104 can be a dermal sensor and can include sensor tail 2408, located at a distal end of sensor 104, and which can be positioned in a substantially parallel orientation to central longitudinal axis 2545. Distal end of sensor tail 2408 can be proximal to distal sharp tip 2556, either in a spaced relation with, at rest in, or at rest against a portion of sharp shaft 2554. As further depicted in FIG. 17E, sharp spacer 2568 provides a spaced relation between a proximal portion of sharp 2552 and sensor 104, such that the proximal portion of sharp 2552 and sensor 104 are not in contact. Sensor module 504 can further include sensor connector 2300 for housing a proximal portion of sensor 104 that is relatively perpendicular to a distal end of sensor 104.

FIG. 17F is a top-down cross-sectional view of sensor module 504. Sensor module 504 can include one or more sensor module snaps 2202 for coupling with a housing (not shown) of sensor control device 102. Sensor module 504 can also include sensor connector 2300, which can have sensor contacts 2302 for coupling with a proximal portion of sensor 104. Sensor connector 2300 can be made of silicone rubber that encapsulates compliant carbon impregnated polymer modules that serve as electrical conductive contacts 2302 between sensor 104 and electrical circuitry contacts for the electronics within sensor control device 102. The connector can also serve as a moisture barrier for sensor 104 when assembled in a compressed state after transfer from a container to an applicator and after application to a user's skin. Although three contacts 2302 are depicted, it should be understood that connector 2300 can have fewer contacts (e.g., two) or more contacts (e.g., four, five, six, etc.), depending on the particular type or configuration of sensor 104. Sensor connector 2300 can be further coupled with sensor module 504 by two connector posts 2206 positioned through a like number of apertures in connector 2300. Although two connector posts 2206 are depicted, it should be understood that any number of connector posts 2206 can be used to couple connector 2300 to sensor module 504.

FIGS. 17G and 17H are, respectively, a perspective view and a side view of another example embodiment of sharp module 2600 that can be used for the insertion of a dermal sensor. Sharp module 2600 is shown here prior to assembly with sensor module 504 (FIG. 6B), and can include components similar to those of the embodiments described with respect to FIGS. 17A and 17B, including sharp 2602, sharp shaft 2604, sharp distal tip 2606, hub push cylinder 2608, hub small cylinder 2612, hub snap pawl 2616 and hub snap pawl locating cylinder 2614. In some embodiments, sharp 2602 can be a "pre-bent" needle that includes a proximal portion 2603 that originates from a point external to sharp module 2600 and intersects, at an angle, a central point of the hub (e.g., through hub push cylinder 2608). Sharp 2602 can also include a distal portion 2605 that extends in a distal direction, at an angle, from a point near a distal portion of hub toward the insertion point of the user's skin. As shown in FIG. 17H, sharp 2602 can include an angled portion 2607 located external to hub push cylinder 2608, which can have a substantially 90° angle between proximal portion 2603 and distal portion 2605 of sharp 2602. Sharp module 2600 can also include a bend fin guide 2620 for maintaining "pre-bent" sharp 2602 in position during assembly and/or use, and can prevent lateral or rotational movement of sharp 2602 relative to hub components. Proximal portion 2603 of sharp 2602 can be "trimmed" from the hub after molding process is completed, and prior to assembly of sharp module 2600 with sensor module 504.

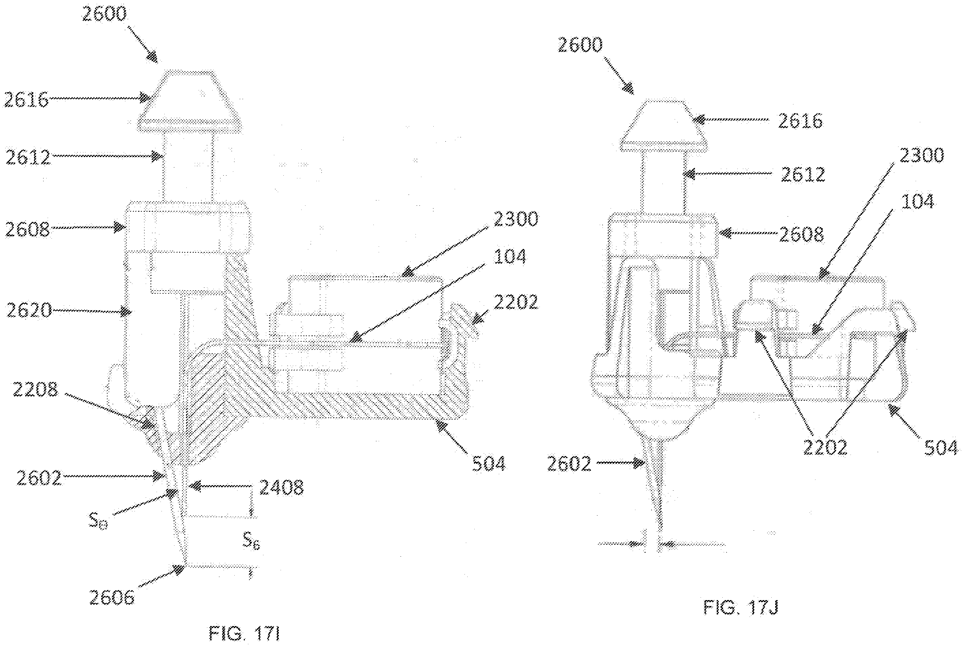

FIGS. 17I and 17J show, respectively, a side cross-sectional view and a side view of sharp module 2600 (including hub snap pawl 2616, hub small cylinder 2612, and hub push cylinder 2608), as assembled with sensor module 504. As can be seen in FIG. 17I, sensor module 504 includes sharp slot 2208, through which sharp 2602 can extend in an angled and distal direction. As described earlier, a proximal portion of sharp 2602 passes through bend fin guide 2620, which is coupled with a distal portion of sensor module 504. Sensor module 504 can also include sensor 104, which can be a dermal sensor. As seen in FIG. 17I, sharp 2602 and sensor tail 2408 can form an acute angle, So, at a point where their respective longitudinal axes converge. Angle So can range between 5° and 20°. In some embodiments, for example, So, can range from 5° to 17°, or 7° to 15°, or 9° to 13°, e.g., 9°, 10°, 11°, 12°, or 13° In some embodiments, distal sharp tip 2606 is located at a distance, S₆, that is proximal to an end of sensor tail 2408. Distance, S₆, can range between 0.02 mm to 0.10 mm, e.g., 0.05 mm, 0.06 mm or 0.07 mm.

Referring still to FIGS. 17I and 17J, sensor module 504 can also include sensor connector 2300 for housing a proximal portion of sensor 104 that is relatively perpendicular to a distal end of sensor 104. Sensor module 504 can further include one or more sensor module snaps 2202 for coupling with a housing (not shown) of sensor control device 102. Sensor connector 2300 can include the same structures described with respect to FIG. 17F.

In the above embodiments, the sharp can be made of stainless steel or a like flexible material (e.g., material used to manufacture acupuncture needles), and dimensioned such that the applicator provides for insertion of at least a portion of the dermal sensor into the dermal layer, but not through the dermal layer of the skin. According to certain embodiments, the sharp has a cross sectional diameter (width) of from 0.1 mm to 0.5 mm. For example, the sharp may have a diameter of from 0.1 mm to 0.3 mm, such as from 0.15 mm to 0.25 mm, e.g., 0.16 mm to 0.22 mm in diameter. A given sharp may have a constant, i.e., uniform, width along its entire length, or may have a varying, i.e., changing, width along at least a portion of its length, such as the tip portion used to pierce the surface of the skin. For example, with respect to the embodiment shown in FIG. 17I, width of sharp 2602 can narrow along a distal portion between bend fin guide 1620 and distal sharp tip 2606.

A sharp can also have a length to insert a dermal sensor just into the dermal layer, and no more. Insertion depth may be controlled by the length of the sharp, the configuration of the base and/or other applicator components that limit insertion depth. A sharp may have a length between 1.5 mm and 25 mm. For example, the sharp may have a length of from 1 mm to 3 mm, from 3 mm to 5 mm, from 5 mm to 7 mm, from 7 mm to 9 mm, from 9 mm to 11 mm, from 11 mm to 13 mm, from 13 mm to 15 mm, from 15 mm to 17 mm, from 17 mm to 19 mm, from 19 mm to 21 mm, from 21 mm to 23 mm, from 23 mm to 25 mm, or a length greater than 25 mm. It will be appreciated that while a sharp may have a length up to 25 mm, in certain embodiments the full length of the sharp is not inserted into the subject because it would extend beyond the dermal space. Non-inserted sharp length may provide for handling and manipulation of the sharp in an applicator set. Therefore, while a sharp may have a length up to 25 mm, the insertion depth of the sharp in the skin on a subject in those certain embodiments will be limited to the dermal layer, e.g., about 1.5 mm to 4 mm, depending on the skin location, as described in greater detail below. However, in all of the embodiments disclosed herein, the sharp can be configured to extend beyond the dermal space, such as into (or even fully through) subcutaneous tissue (e.g., 3 mm to 10 mm beneath the surface of the skin depending on the location of the skin on the body). Additionally, in some example embodiments, the sharps described herein can include hollow or partially hollow insertion needles, having an internal space or lumen. In other embodiments, however, the sharps described herein can include solid insertion needles, which do not have an internal space and/or lumen. Furthermore, a sharp of the subject applicator sets can also be bladed or non-bladed.

Likewise, in the above embodiments, a dermal sensor is sized so that at least a portion of the sensor is positioned in the dermal layer and no more, and a portion extends outside the skin in the transcutaneously positioned embodiments. That is, a dermal sensor is dimensioned such that when the dermal sensor is entirely or substantially entirely inserted into the dermal layer, the distal-most portion of the sensor (the insertion portion or insertion length) is positioned within the dermis of the subject and no portion of the sensor is inserted beyond a dermal layer of the subject when the sensor is operably dermally positioned.

The dimensions (e.g., the length) of the sensor may be selected according to the body site of the subject in which the sensor is to be inserted, as the depth and thickness of the epidermis and dermis exhibit a degree of variability depending on skin location. For example, the epidermis is only about 0.05 mm thick on the eyelids, but about 1.5 mm thick on the palms and the soles of the feet. The dermis is the thickest of the three layers of skin and ranges from about 1.5 mm to 4 mm thick, depending on the skin location. For implantation of the distal end of the sensor into, but not through, the dermal layer of the subject, the length of the inserted portion of the dermal sensor should be greater than the thickness of the epidermis, but should not exceed the combined thickness of the epidermis and dermis. Methods may include determining an insertion site on a body of a user and determining the depth of the dermal layer at the site, and selecting the appropriately-sized applicator set for the site.

In certain aspects, the sensor is an elongate sensor having a longest dimension (or "length") of from 0.25 mm to 4 mm. The length of the sensor that is inserted, in the embodiments in which only a portion of a sensor is dermally inserted, ranges from 0.5 mm to 3 mm, such as from 1 mm to 2 mm, e.g., 1.5 mm. The dimensions of the sensor may also be expressed in terms of its aspect ratio. In certain embodiments, a dermal sensor has an aspect ratio of length to width (diameter) of about 30:1 to about 6:1. For example, the aspect ratio may be from about 25:1 to about 10:1, including 20:1 and 15:1. The inserted portion of a dermal sensor has sensing chemistry.

However, all of the embodiments disclosed herein can be configured such that at least a portion of the sensor is positioned beyond the dermal layer, such as into (or through) the subcutaneous tissue (or fat). For example, the sensor can be dimensioned such that when the sensor is entirely or substantially entirely inserted into the body, the distal-most portion of the sensor (the insertion portion or insertion length) is positioned within the subcutaneous tissue (beyond the dermis of the subject) and no portion of the sensor is inserted beyond the subcutaneous tissue of the subject when the sensor is operably positioned. As mentioned, the subcutaneous tissue is typically present in the region that is 3 mm to 10 mm beneath the outer skin surface, depending on the location of the skin on the body.

### Exemplary Applicators and Sensor Control Devices for One Piece Architectures

Referring briefly again to FIGS. 1 and 3A-3G, for the two-piece architecture system, the sensor tray 202 and the sensor applicator 102 are provided to the user as separate packages, thus requiring the user to open each package and finally assemble the system. In some applications, the discrete, sealed packages allow the sensor tray 202 and the sensor applicator 102 to be sterilized in separate sterilization processes unique to the contents of each package and otherwise incompatible with the contents of the other. More specifically, the sensor tray 202, which includes the plug assembly 207, including the sensor 110 and the sharp 220, may be sterilized using radiation sterilization, such as electron beam (or "e-beam") irradiation. Radiation sterilization, however, can damage the electrical components arranged within the electronics housing of the sensor control device 102. Consequently, if the sensor applicator 102, which contains the electronics housing of the sensor control device 102, needs to be sterilized, it may be sterilized via another method, such as gaseous chemical sterilization using, for example, ethylene oxide. Gaseous chemical sterilization, however, can damage the enzymes or other chemistry and biologies included on the sensor 110. Because of this sterilization incompatibility, the sensor tray 202 and the sensor applicator 102 are commonly sterilized in separate sterilization processes and subsequently packaged separately, which requires the user to finally assemble the components for use.

According to embodiments of the present disclosure, the sensor control device 102 may be modified to provide a one-piece architecture that may be subjected to sterilization techniques specifically designed for a one-piece architecture sensor control device. A one-piece architecture allows the sensor applicator 150 and the sensor control device 102 to be shipped to the user in a single, sealed package that does not require any final user assembly steps. Rather, the user need only open one package and subsequently deliver the sensor control device 102 to the target monitoring location. The one-piece system architecture described herein may prove advantageous in eliminating component parts, various fabrication process steps, and user assembly steps. As a result, packaging and waste are reduced, and the potential for user error or contamination to the system is mitigated.

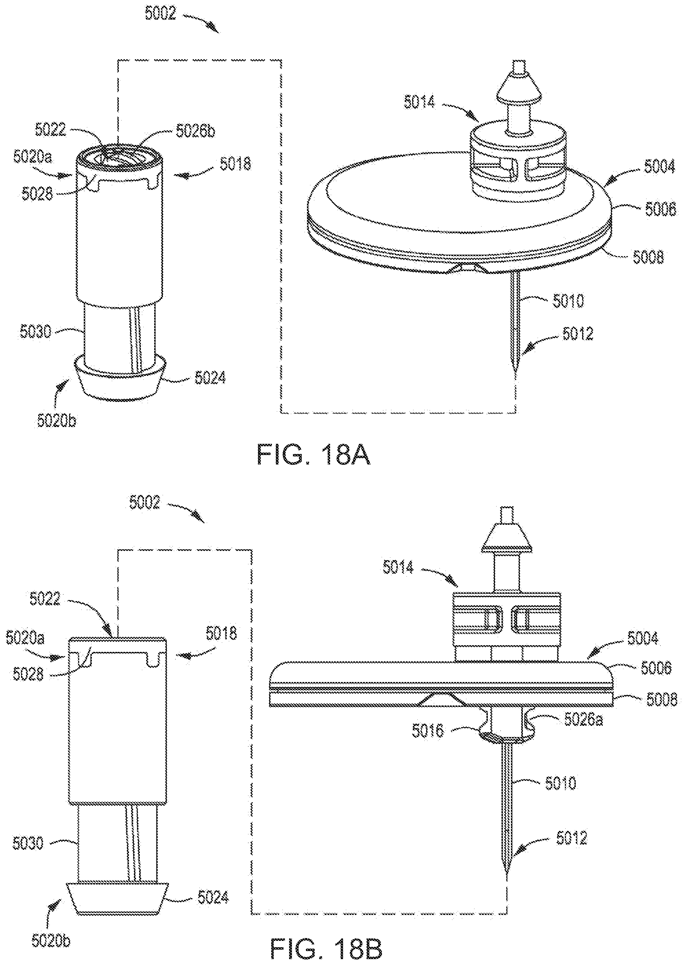

FIGS. 18A and 18B are isometric and side views, respectively, of another example sensor control device 5002, according to one or more embodiments of the present disclosure. The sensor control device 5002 may be similar in some respects to the sensor control device 102 of FIG. 1 and therefore may be best understood with reference thereto. Moreover, the sensor control device 5002 may replace the sensor control device 102 of FIG. 1 and, therefore, may be used in conjunction with the sensor applicator 102 of FIG. 1, which may deliver the sensor control device 5002 to a target monitoring location on a user's skin.

Unlike the sensor control device 102 of FIG. 1, however, the sensor control device 5002 may comprise a one-piece system architecture not requiring a user to open multiple packages and finally assemble the sensor control device 5002 prior to application. Rather, upon receipt by the user, the sensor control device 5002 may already be fully assembled and properly positioned within the sensor applicator 150 (FIG. 1). To use the sensor control device 5002, the user need only open one barrier (e.g., the applicator cap 708 of FIG. 3B) before promptly delivering the sensor control device 5002 to the target monitoring location for use.

As illustrated, the sensor control device 5002 includes an electronics housing 5004 that is generally disc-shaped and may have a circular cross-section. In other embodiments, however, the electronics housing 5004 may exhibit other cross-sectional shapes, such as ovoid or polygonal. The electronics housing 5004 may be configured to house or otherwise contain various electrical components used to operate the sensor control device 5002. In at least one embodiment, an adhesive patch (not shown) may be arranged at the bottom of the electronics housing 5004. The adhesive patch may be similar to the adhesive patch 105 of FIG. 1, and may thus help adhere the sensor control device 5002 to the user's skin for use.

As illustrated, the sensor control device 5002 includes an electronics housing 5004 that includes a shell 5006 and a mount 5008 that is matable with the shell 5006. The shell 5006 may be secured to the mount 5008 via a variety of ways, such as a snap fit engagement, an interference fit, sonic welding, one or more mechanical fasteners (e.g., screws), a gasket, an adhesive, or any combination thereof. In some cases, the shell 5006 may be secured to the mount 5008 such that a sealed interface is generated therebetween.

The sensor control device 5002 may further include a sensor 5010 (partially visible) and a sharp 5012 (partially visible), used to help deliver the sensor 5010 transcutaneously under a user's skin during application of the sensor control device 5002. As illustrated, corresponding portions of the sensor 5010 and the sharp 5012 extend distally from the bottom of the electronics housing 5004 (e.g., the mount 5008). The sharp 5012 may include a sharp hub 5014 configured to secure and carry the sharp 5012. As best seen in FIG. 18B, the sharp hub 5014 may include or otherwise define a mating member 5016. To couple the sharp 5012 to the sensor control device 5002, the sharp 5012 may be advanced axially through the electronics housing 5004 until the sharp hub 5014 engages an upper surface of the shell 5006 and the mating member 5016 extends distally from the bottom of the mount 5008. As the sharp 5012 penetrates the electronics housing 5004, the exposed portion of the sensor 5010 may be received within a hollow or recessed (arcuate) portion of the sharp 5012. The remaining portion of the sensor 5010 is arranged within the interior of the electronics housing 5004.

The sensor control device 5002 may further include a sensor cap 5018, shown exploded or detached from the electronics housing 5004 in FIGS. 18A-18B. The sensor cap 5016 may be removably coupled to the sensor control device 5002 (e.g., the electronics housing 5004) at or near the bottom of the mount 5008. The sensor cap 5018 may help provide a sealed barrier that surrounds and protects the exposed portions of the sensor 5010 and the sharp 5012 from gaseous chemical sterilization. As illustrated, the sensor cap 5018 may comprise a generally cylindrical body having a first end 5020a and a second end 5020b opposite the first end 5020a. The first end 5020a may be open to provide access into an inner chamber 5022 defined within the body. In contrast, the second end 5020b may be closed and may provide or otherwise define an engagement feature 5024. As described herein, the engagement feature 5024 may help mate the sensor cap 5018 to the cap (e.g., the applicator cap 708 of FIG. 3B) of a sensor applicator (e.g., the sensor applicator 150 of FIGS. 1 and 3A-3G), and may help remove the sensor cap 5018 from the sensor control device 5002 upon removing the cap from the sensor applicator.

The sensor cap 5018 may be removably coupled to the electronics housing 5004 at or near the bottom of the mount 5008. More specifically, the sensor cap 5018 may be removably coupled to the mating member 5016, which extends distally from the bottom of the mount 5008. In at least one embodiment, for example, the mating member 5016 may define a set of external threads 5026a (FIG. 18B) matable with a set of internal threads 5026b (FIG. 18A) defined by the sensor cap 5018. In some embodiments, the external and internal threads 5026a, b may comprise a flat thread design (e.g., lack of helical curvature), which may prove advantageous in molding the parts. Alternatively, the external and internal threads 5026a,b may comprise a helical threaded engagement. Accordingly, the sensor cap 5018 may be threadably coupled to the sensor control device 5002 at the mating member 5016 of the sharp hub 5014. In other embodiments, the sensor cap 5018 may be removably coupled to the mating member 5016 via other types of engagements including, but not limited to, an interference or friction fit, or a frangible member or substance that may be broken with minimal separation force (e.g., axial or rotational force).

In some embodiments, the sensor cap 5018 may comprise a monolithic (singular) structure extending between the first and second ends 5020a, b. In other embodiments, however, the sensor cap 5018 may comprise two or more component parts. In the illustrated embodiment, for example, the sensor cap 5018 may include a seal ring 5028 positioned at the first end 5020a and a desiccant cap 5030 arranged at the second end 5020b. The seal ring 5028 may be configured to help seal the inner chamber 5022, as described in more detail below. In at least one embodiment, the seal ring 5028 may comprise an elastomeric O-ring. The desiccant cap 5030 may house or comprise a desiccant to help maintain preferred humidity levels within the inner chamber 5022. The desiccant cap 5030 may also define or otherwise provide the engagement feature 5024 of the sensor cap 5018.

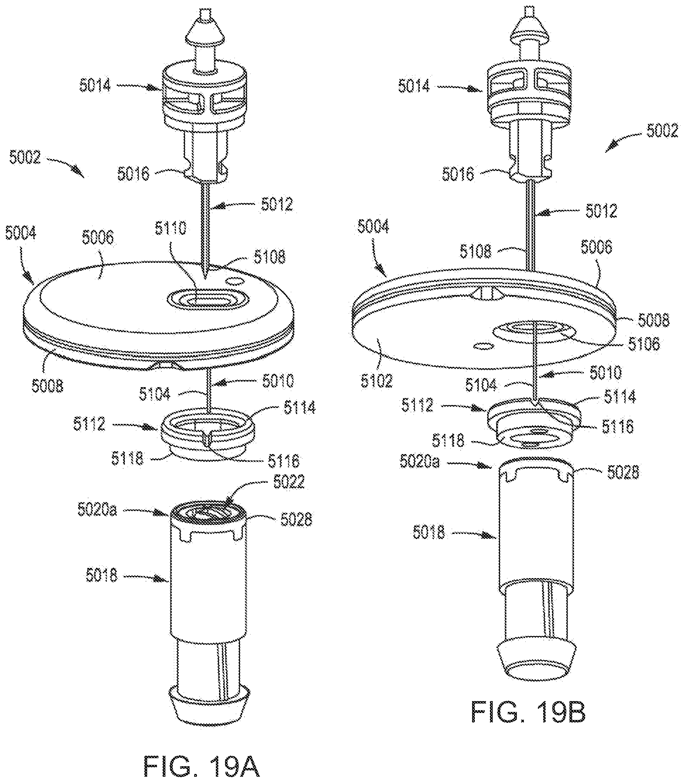

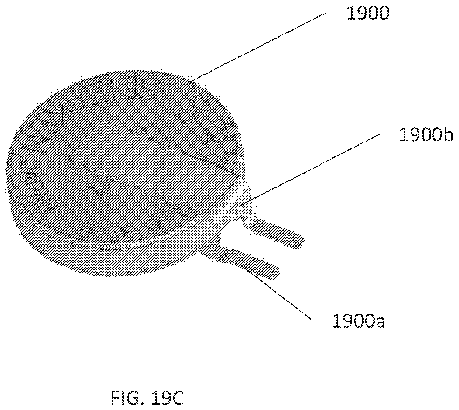

FIGS. 19A and 19B are exploded isometric top and bottom views, respectively, of the sensor control device 5002, according to one or more embodiments. The shell 5006 and the mount 5008 operate as opposing clamshell halves that enclose or otherwise substantially encapsulate various electronic components of the sensor control device 5002. More specifically, electronic components may include, but are not limited to, a printed circuit board (PCB), one or more resistors, transistors, capacitors, inductors, diodes, and switches. A data processing unit and a battery may be mounted to or otherwise interact with the PCB. The data processing unit may comprise, for example, an application specific integrated circuit (ASIC) configured to implement one or more functions or routines associated with operation of the sensor control device 5002. More specifically, the data processing unit may be configured to perform data processing functions, where such functions may include, but are not limited to, filtering and encoding of data signals, each of which corresponds to a sampled analyte level of the user. The data processing unit may also include or otherwise communicate with an antenna for communicating with the reader device 120 (FIG. 1). The battery may provide power to the sensor control device 5002 and, more particularly, to the electronic components of the PCB. For example, battery can be any battery known to a person of ordinary skill in the art, such as a coin cell battery or a button battery, as shown in FIG. 19C. In certain embodiments, battery 1900 may include silver oxide batteries. Battery 1900 may be laser welded to the PCB and located to ensure that outer diameter of battery remains within outer perimeter of PCB. In some embodiments, battery 1900 may be connected to PCB using negative battery tab 1900a and positive battery tab 1900b. For example, negative battery tab 1900a of battery 1900 may be substantially planar and in the same plane as negative terminal of battery 1900; while positive battery tab 1900b may include one or more bends so that one end of positive terminal 1900b is in contact with and in the same plane as positive battery terminal while a second end configured to couple to the PCB is in the same plane as the negative battery tab 1900a. Furthermore, battery 1900 may be positioned in a battery aperture on the PCB to eliminate electrical interference between negative battery tab 1900a and PCB. While not shown, the sensor control device 5002 may also include an adhesive patch that may be applied to the bottom 5102 (FIG. 19B) of the mount 5008, and may help adhere the sensor control device 5002 to the user's skin for use.

It will be understood by those of skill in the art that the battery assembly embodiments described herein, including the PCB and data processing unit, may be implemented in other medical devices, including sensor control devices having other types of housing and configurations relating thereto. In other words, the example embodiments of sensor control device 5002, comprising opposing clamshell halves, are intended to be non-limiting and merely illustrate one type of device that can be used with the battery assembly embodiments described herein.

The sensor control device 5002 may provide or otherwise include a sealed subassembly that includes, among other component parts, the shell 5006, the sensor 5010, the sharp 5012, and the sensor cap 5018. The sealed subassembly of the sensor control device 5002 may help isolate the sensor 5010 and the sharp 5012 within the inner chamber 5022 (FIG. 19A) of the sensor cap 5018 during a gaseous chemical sterilization process, which might otherwise adversely affect the chemistry provided on the sensor 5010.

The sensor 5010 may include a tail 5104 that extends out an aperture 5106 (FIG. 19B) defined in the mount 5008 to be transcutaneously received beneath a user's skin. The tail 5104 may have an enzyme or other chemistry included thereon to help facilitate analyte monitoring. The sharp 5012 may include a sharp tip 5108 extendable through an aperture 5110 (FIG. 19A) defined by the shell 5006, and the aperture 5110 may be coaxially aligned with the aperture 5106 of the mount 5008. As the sharp tip 5108 penetrates the electronics housing 5004, the tail 5104 of the sensor 5010 may be received within a hollow or recessed portion of the sharp tip 5108. The sharp tip 5108 may be configured to penetrate the skin while carrying the tail 5104 to put the active chemistry of the tail 5104 into contact with bodily fluids.

The sharp tip 5108 may be advanced through the electronics housing 5004 until the sharp hub 5014 engages an upper surface of the shell 5006 and the mating member 5016 extends out the aperture 5106 in the bottom 5102 of the mount 5008. In some embodiments, a seal member (not shown), such as an O-ring or seal ring, may interpose the sharp hub 5014 and the upper surface of the shell 5006 to help seal the interface between the two components. In some embodiments, the seal member may comprise a separate component part, but may alternatively form an integral part of the shell 5006, such as being a co-molded or overmolded component part.

The sealed subassembly may further include a collar 5112 that is positioned within the electronics housing 5004 and extends at least partially into the aperture 5106. The collar 5112 may be a generally annular structure that defines or otherwise provides an annular ridge 5114 on its top surface. In some embodiments, as illustrated, a groove 5116 may be defined in the annular ridge 5114 and may be configured to accommodate or otherwise receive a portion of the sensor 5010 extending laterally within the electronics housing 5004.

In assembling the sealed subassembly, a bottom 5118 of the collar 5112 may be exposed at the aperture 5106 and may sealingly engage the first end 5020a of the sensor cap 5018 and, more particularly, the seal ring 5028. In contrast, the annular ridge 5114 at the top of the collar 5112 may sealingly engage an inner surface (not shown) of the shell 5006. In at least one embodiment, a seal member (not shown) may interpose the annular ridge 5114 and the inner surface of the shell 5006 to form a sealed interface. In such embodiments, the seal member may also extend (flow) into the groove 5116 defined in the annular ridge 5114 and thereby seal about the sensor 5010 extending laterally within the electronics housing 5004. The seal member may comprise, for example, an adhesive, a gasket, or an ultrasonic weld, and may help isolate the enzymes and other chemistry included on the tail 5104.

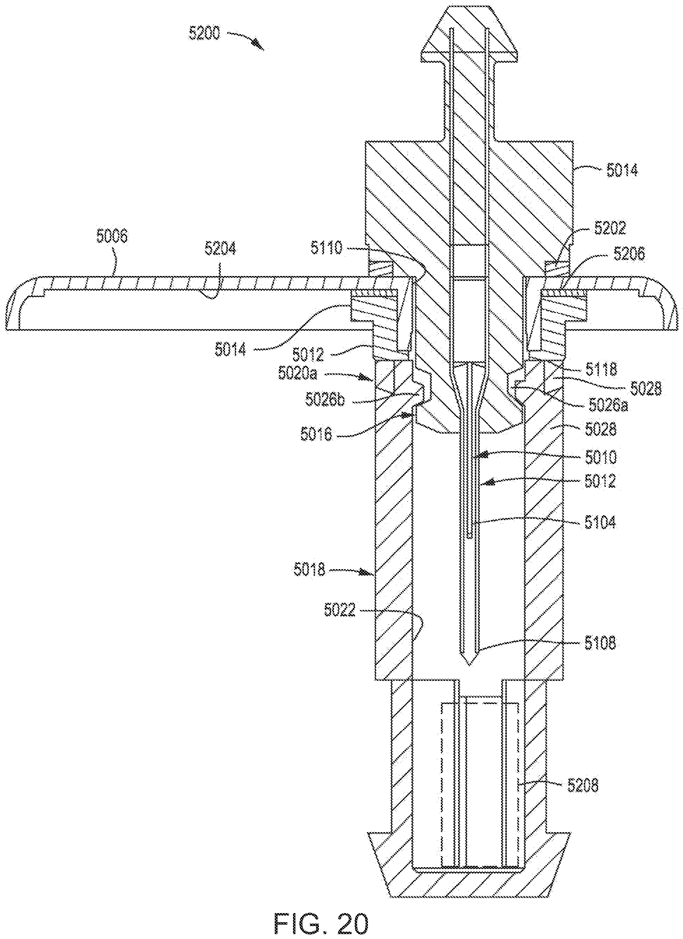

FIG. 20 is a cross-sectional side view of an assembled sealed subassembly 5200, according to one or more embodiments. The sealed subassembly 5200 may form part of the sensor control device 5002 of FIGS. 18A-18B and 19A-20B and may include portions of the shell 5006, the sensor 5010, the sharp 5012, the sensor cap 5018, and the collar 5112. The sealed subassembly 5200 may be assembled in a variety of ways. In one assembly process, the sharp 5012 may be coupled to the sensor control device 5002 by extending the sharp tip 5108 through the aperture 5110 defined in the top of the shell 5006 and advancing the sharp 5012 through the shell 5006 until the sharp hub 5014 engages the top of the shell 5006 and the mating member 5016 extends distally from the shell 5006. In some embodiments, as mentioned above, a seal member 5202 (e.g., an O- ring or seal ring) may interpose the sharp hub 5014 and the upper surface of the shell 5006 to help seal the interface between the two components.

The collar 5112 may then be received over (about) the mating member 5016 and advanced toward an inner surface 5204 of the shell 5006 to enable the annular ridge 5114 to engage the inner surface 5204. A seal member 5206 may interpose the annular ridge 5114 and the inner surface 5204 and thereby form a sealed interface. The seal member 5206 may also extend (flow) into the groove 5116 (FIGS. 19A-20B) defined in the annular ridge 5114 and thereby seal about the sensor 5010 extending laterally within the electronics housing 5004 (FIGS. 19A-20B). In other embodiments, however, the collar 5112 may first be sealed to the inner surface 5204 of the shell 5006, following which the sharp 5012 and the sharp hub 5014 may be extended through the aperture 5110, as described above.

The sensor cap 5018 may be removably coupled to the sensor control device 5002 by threadably mating the internal threads 5026b of the sensor cap 5018 with the external threads 5026a of the mating member 5016. Tightening (rotating) the mated engagement between the sensor cap 5018 and the mating member 5016 may urge the first end 5020a of the sensor cap 5018 into sealed engagement with the bottom 5118 of the collar 5112. Moreover, tightening the mated engagement between the sensor cap 5018 and the mating member 5016 may also enhance the sealed interface between the sharp hub 5014 and the top of the shell 5006, and between the annular ridge 5114 and the inner surface 5204 of the shell 5006.

The inner chamber 5022 may be sized and otherwise configured to receive the tail 5104 and the sharp tip 5108. Moreover, the inner chamber 5022 may be sealed to isolate the tail 5104 and the sharp tip 5108 from substances that might adversely interact with the chemistry of the tail 5104. In some embodiments, a desiccant 5208 (shown in dashed lines) may be present within the inner chamber 5022 to maintain proper humidity levels.

Once properly assembled, the sealed subassembly 5200 may be subjected to any of the radiation sterilization processes mentioned herein to properly sterilize the sensor 5010 and the sharp 5012. This sterilization step may be undertaken apart from the remaining portions of the sensor control device (FIGS. 18A-18B and 19A-20B) to prevent damage to sensitive electrical components. The sealed subassembly 5200 may be subjected to radiation sterilization prior to or after coupling the sensor cap 5018 to the sharp hub 5014. When sterilized after coupling the sensor cap 5018 to the sharp hub 5014, the sensor cap 5018 may be made of a material that permits the propagation of radiation therethrough. In some embodiments, the sensor cap 5018 may be transparent or translucent, but can otherwise be opaque.

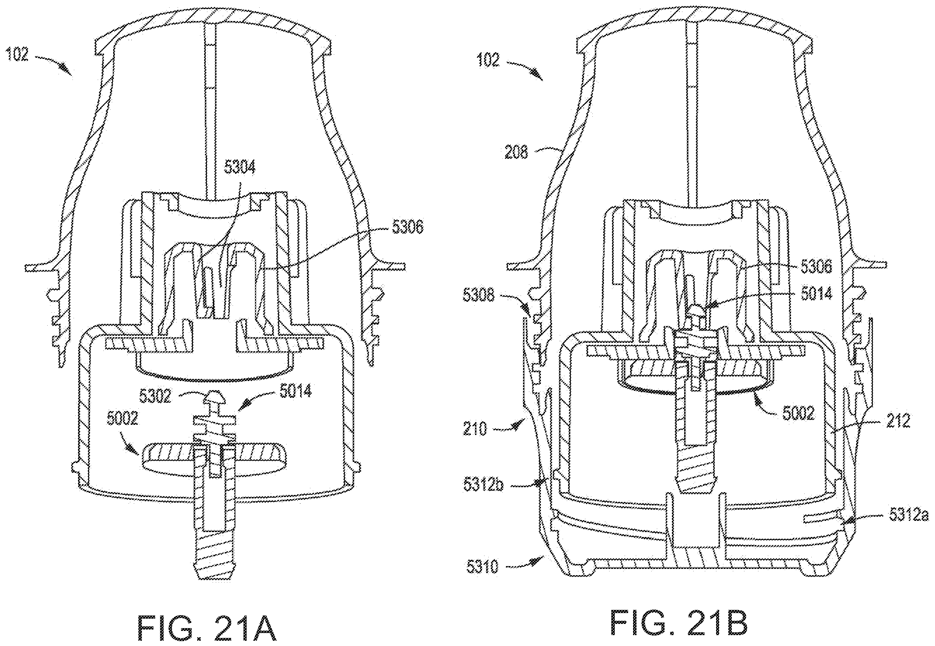

FIGS. 21A-21C are progressive cross-sectional side views showing assembly of the sensor applicator 102 with the sensor control device 5002, according to one or more embodiments. Once the sensor control device 5002 is fully assembled, it may then be loaded into the sensor applicator 102. With reference to FIG. 21A, the sharp hub 5014 may include or otherwise define a hub snap pawl 5302 configured to help couple the sensor control device 5002 to the sensor applicator 102. More specifically, the sensor control device 5002 may be advanced into the interior of the sensor applicator 102 and the hub snap pawl 5302 may be received by corresponding arms 5304 of a sharp carrier 5306 positioned within the sensor applicator 102.

In FIG. 21B, the sensor control device 5002 is shown received by the sharp carrier 5306 and, therefore, secured within the sensor applicator 102. Once the sensor control device 5002 is loaded into the sensor applicator 102, the applicator cap 210 may be coupled to the sensor applicator 102. In some embodiments, the applicator cap 210 and the housing 208 may have opposing, matable sets of threads 5308 that enable the applicator cap 210 to be screwed onto the housing 208 in a clockwise (or counter-clockwise) direction and thereby secure the applicator cap 210 to the sensor applicator 102.

As illustrated, the sheath 212 is also positioned within the sensor applicator 102, and the sensor applicator 102 may include a sheath locking mechanism 5310 configured to ensure that the sheath 212 does not prematurely collapse during a shock event. In the illustrated embodiment, the sheath locking mechanism 5310 may comprise a threaded engagement between the applicator cap 210 and the sheath 212. More specifically, one or more internal threads 5312a may be defined or otherwise provided on the inner surface of the applicator cap 210, and one or more external threads 53 l2b may be defined or otherwise provided on the sheath 212. The internal and external threads 53 l2a,b may be configured to threadably mate as the applicator cap 210 is threaded to the sensor applicator 102 at the threads 5308. The internal and external threads 53l2a,b may have the same thread pitch as the threads 5308 that enable the applicator cap 210 to be screwed onto the housing 208.

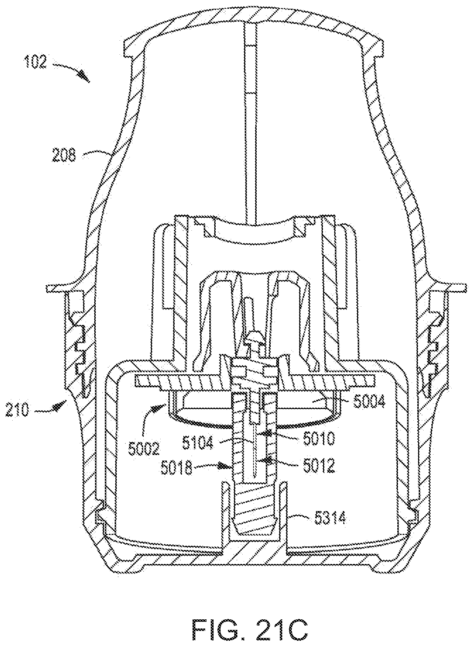

In FIG. 21C, the applicator cap 210 is shown fully threaded (coupled) to the housing 208. As illustrated, the applicator cap 210 may further provide and otherwise define a cap post 5314 centrally located within the interior of the applicator cap 210 and extending proximally from the bottom thereof. The cap post 5314 may be configured to receive at least a portion of the sensor cap 5018 as the applicator cap 210 is screwed onto the housing 208.

With the sensor control device 5002 loaded within the sensor applicator 102 and the applicator cap 210 properly secured, the sensor control device 5002 may then be subjected to a gaseous chemical sterilization configured to sterilize the electronics housing 5004 and any other exposed portions of the sensor control device 5002. Since the distal portions of the sensor 5010 and the sharp 5012 are sealed within the sensor cap 5018, the chemicals used during the gaseous chemical sterilization process are unable to interact with the enzymes, chemistry, and biologies provided on the tail 5104, and other sensor components, such as membrane coatings that regulate analyte influx.

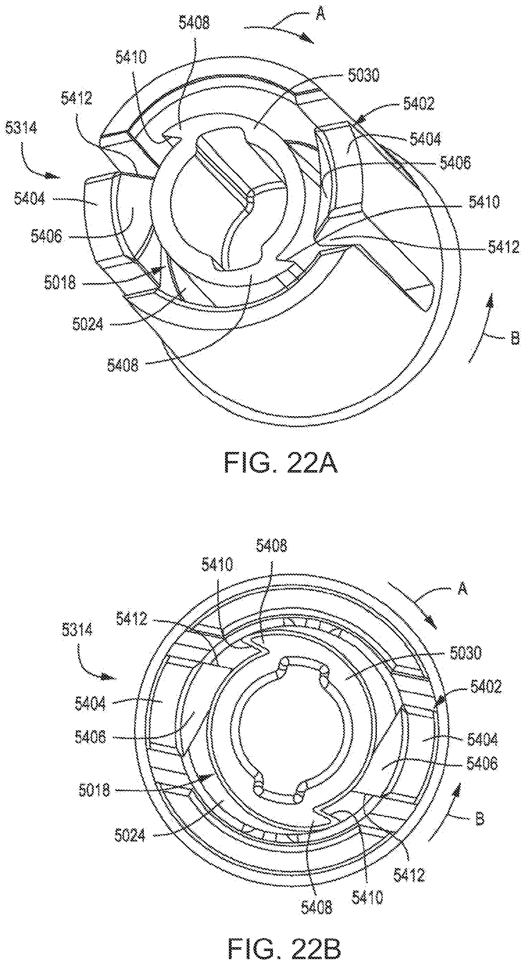

FIGS. 22A and 22B are perspective and top views, respectively, of the cap post 5314, according to one or more additional embodiments. In the illustrated depiction, a portion of the sensor cap 5018 is received within the cap post 5314 and, more specifically, the desiccant cap 5030 of the sensor cap 5018 is arranged within cap post 5314.

As illustrated, the cap post 5314 may define a receiver feature 5402 configured to receive the engagement feature 5024 of the sensor cap 5018 upon coupling (e.g., threading) the applicator cap 210 (FIG. 21C) to the sensor applicator 102 (FIGS. 21A-21C). Upon removing the applicator cap 210 from the sensor applicator 102, however, the receiver feature 5402 may prevent the engagement feature 5024 from reversing direction and thus prevent the sensor cap 5018 from separating from the cap post 5314. Instead, removing the applicator cap 210 from the sensor applicator 102 will simultaneously detach the sensor cap 5018 from the sensor control device 5002 (FIGS. 18A-18B and 21A-21C), and thereby expose the distal portions of the sensor 5010 (FIGS. 21A-21C) and the sharp 5012 (FIGS. 21A-21C).

Many design variations of the receiver feature 5402 may be employed. In the illustrated embodiment, the receiver feature 5402 includes one or more compliant members 5404 (two shown) that are expandable or flexible to receive the engagement feature 5024 (FIGS. 18A-18B). The engagement feature 5024 may comprise, for example, an enlarged head and the compliant member(s) 5404 may comprise a collet- type device that includes a plurality of compliant fingers configured to flex radially outward to receive the enlarged head.

The compliant member(s) 5404 may further provide or otherwise define corresponding ramped surfaces 5406 configured to interact with one or more opposing camming surfaces 5408 provided on the outer wall of the engagement feature 5024. The configuration and alignment of the ramped surface(s) 5406 and the opposing camming surface(s) 5408 is such that the applicator cap 210 is able to rotate relative to the sensor cap 5018 in a first direction A (e.g., clockwise), but the cap post 5314 binds against the sensor cap 5018 when the applicator cap 210 is rotated in a second direction B (e.g., counter clockwise). More particularly, as the applicator cap 210 (and thus the cap post 5314) rotates in the first direction A, the camming surfaces 5408 engage the ramped surfaces 5406, which urge the compliant members 5404 to flex or otherwise deflect radially outward and results in a ratcheting effect. Rotating the applicator cap 210 (and thus the cap post 5314) in the second direction B, however, will drive angled surfaces 5410 of the camming surfaces 5408 into opposing angled surfaces 5412 of the ramped surfaces 5406, which results in the sensor cap 5018 binding against the compliant member(s) 5404.

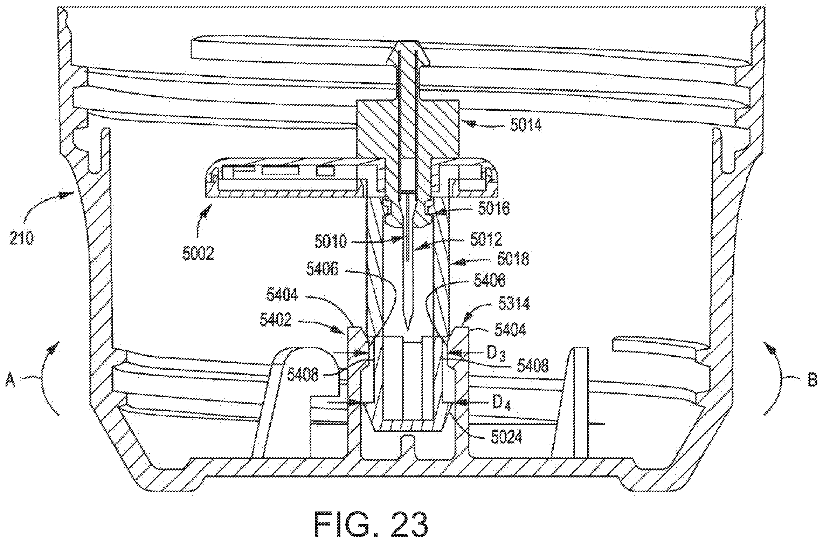

FIG. 23 is a cross-sectional side view of the sensor control device 5002 positioned within the applicator cap 210, according to one or more embodiments. As illustrated, the opening to the receiver feature 5402 exhibits a first diameter D₃, while the engagement feature 5024 of the sensor cap 5018 exhibits a second diameter D₄ that is larger than the first diameter D₃ and greater than the outer diameter of the remaining portions of the sensor cap 5018. As the sensor cap 5018 is extended into the cap post 5314, the compliant member(s) 5404 of the receiver feature 5402 may flex (expand) radially outward to receive the engagement feature 5024. In some embodiments, as illustrated, the engagement feature 5024 may provide or otherwise define an angled or frustoconical outer surface that helps bias the compliant member(s) 5404 radially outward. Once the engagement feature 5024 bypasses the receiver feature 5402, the compliant member(s) 5404 are able to flex back to (or towards) their natural state and thus lock the sensor cap 5018 within the cap post 5314.

As the applicator cap 210 is threaded to (screwed onto) the housing 208 (FIGS. 21A-21C) in the first direction A, the cap post 5314 correspondingly rotates in the same direction and the sensor cap 5018 is progressively introduced into the cap post 5314. As the cap post 5314 rotates, the ramped surfaces 5406 of the compliant members 5404 ratchet against the opposing camming surfaces 5408 of the sensor cap 5018. This continues until the applicator cap 210 is fully threaded onto (screwed onto) the housing 208. In some embodiments, the ratcheting action may occur over two full revolutions of the applicator cap 210 before the applicator cap 210 reaches its final position.

To remove the applicator cap 210, the applicator cap 210 is rotated in the second direction B, which correspondingly rotates the cap post 5314 in the same direction and causes the camming surfaces 5408 (i.e., the angled surfaces 5410 of FIGS. 22A-22B) to bind against the ramped surfaces 5406 (i.e., the angled surfaces 5412 of FIGS. 22A-22B). Consequently, continued rotation of the applicator cap 210 in the second direction B causes the sensor cap 5018 to correspondingly rotate in the same direction and thereby unthread from the mating member 5016 to allow the sensor cap 5018 to detach from the sensor control device 5002. Detaching the sensor cap 5018 from the sensor control device 5002 exposes the distal portions of the sensor 5010 and the sharp 5012, and thus places the sensor control device 5002 in position for firing (use).

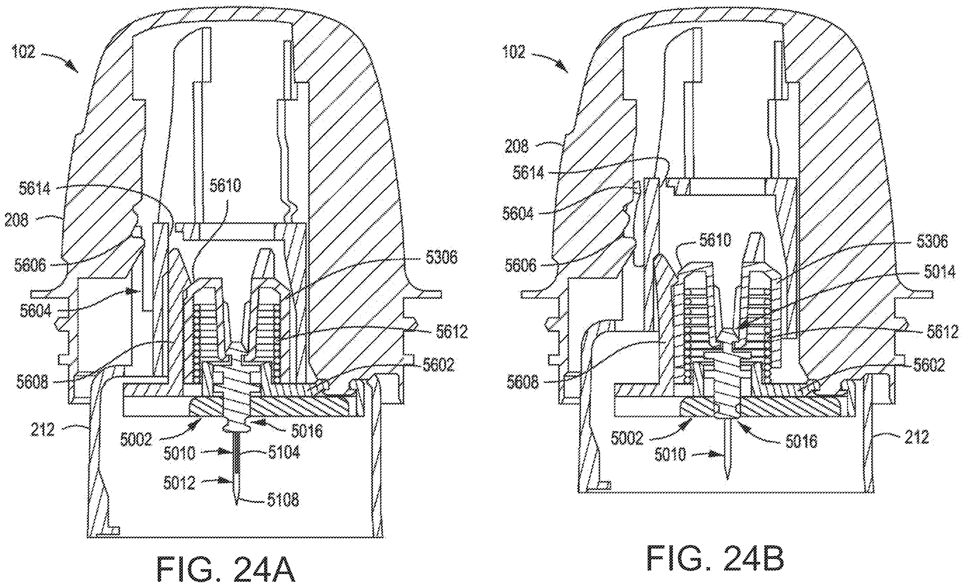

FIGS. 24A and 24B are cross-sectional side views of the sensor applicator 102 ready to deploy the sensor control device 5002 to a target monitoring location, according to one or more embodiments. More specifically, FIG. 24A depicts the sensor applicator 102 ready to deploy (fire) the sensor control device 5002, and FIG. 24B depicts the sensor applicator 102 in the process of deploying (firing) the sensor control device 5002. As illustrated, the applicator cap 210 (FIGS. 21A-21C and 23) has been removed, which correspondingly detaches (removes) the sensor cap 5018 (FIGS. 21A-21C and 23) and thereby exposes the tail 5104 of the sensor 5010 and the sharp tip 5108 of the sharp 5012, as described above. In conjunction with the sheath 212 and the sharp carrier 5306, the sensor applicator 102 also includes a sensor carrier 5602 (alternately referred to as a "puck" carrier) that helps position and secure the sensor control device 5002 within the sensor applicator 102.

Referring first to FIG. 24A, as illustrated, the sheath 212 includes one or more sheath arms 5604 (one shown) configured to interact with a corresponding one or more detents 5606 (one shown) defined within the interior of the housing 208. The detent(s) 5606 are alternately referred to as "firing" detent(s). When the sensor control device 5002 is initially installed in the sensor applicator 102, the sheath arms 5604 may be received within the detents 5606, which places the sensor applicator 102 in firing position. In the firing position, the mating member 5016 extends distally beyond the bottom of the sensor control device 5002. As discussed below, the process of firing the sensor applicator 102 causes the mating member 5016 to retract so that it does not contact the user's skin.

The sensor carrier 5602 may also include one or more carrier arms 5608 (one shown) configured to interact with a corresponding one or more grooves 5610 (one shown) defined on the sharp carrier 5306. A spring 5612 may be arranged within a cavity defined by the sharp carrier 5306 and may passively bias the sharp carrier 5306 upward within the housing 208. When the carrier arm(s) 5608 are properly received within the groove(s) 5610, however, the sharp carrier 5306 is maintained in position and prevented from moving upward. The carrier arm(s) 5608 interpose the sheath 212 and the sharp carrier 5306, and a radial shoulder 5614 defined on the sheath 212 may be sized to maintain the carrier arm(s) 5608 engaged within the groove(s) 5610 and thereby maintain the sharp carrier 5306 in position.

In FIG. 24B, the sensor applicator 102 is in the process of firing. As discussed herein with reference to FIGS. 3F-3G, this may be accomplished by advancing the sensor applicator 102 toward a target monitoring location until the sheath 212 engages the skin of the user. Continued pressure on the sensor applicator 102 against the skin may cause the sheath arm(s) 5604 to disengage from the corresponding detent(s) 5606, which allows the sheath 212 to collapse into the housing 208. As the sheath 212 starts to collapse, the radial shoulder 5614 eventually moves out of radial engagement with the carrier arm(s) 5608, which allows the carrier arm(s) 5608 to disengage from the groove(s) 5610. The passive spring force of the spring 5612 is then free to push upward on the sharp carrier 5306 and thereby force the carrier arm(s) 5608 out of engagement with the groove(s) 5610, which allows the sharp carrier 5306 to move slightly upward within the housing 208. In some embodiments, fewer coils may be incorporated into the design of the spring 5612 to increase the spring force necessary to overcome the engagement between carrier arm(s) 5608 and the groove(s) 5610. In at least one embodiment, one or both of the carrier arm(s) 5608 and the groove(s) 5610 may be angled to help ease disengagement.

As the sharp carrier 5306 moves upward within the housing 208, the sharp hub 5014 may correspondingly move in the same direction, which may cause partial retraction of the mating member 5016 such that it becomes flush, substantially flush, or sub-flush with the bottom of the sensor control device 5002. As will be appreciated, this ensures that the mating member 5016 does not come into contact with the user's skin, which might otherwise adversely impact sensor insertion, cause excessive pain, or prevent the adhesive patch (not shown) positioned on the bottom of the sensor control device 5002 from properly adhering to the skin.

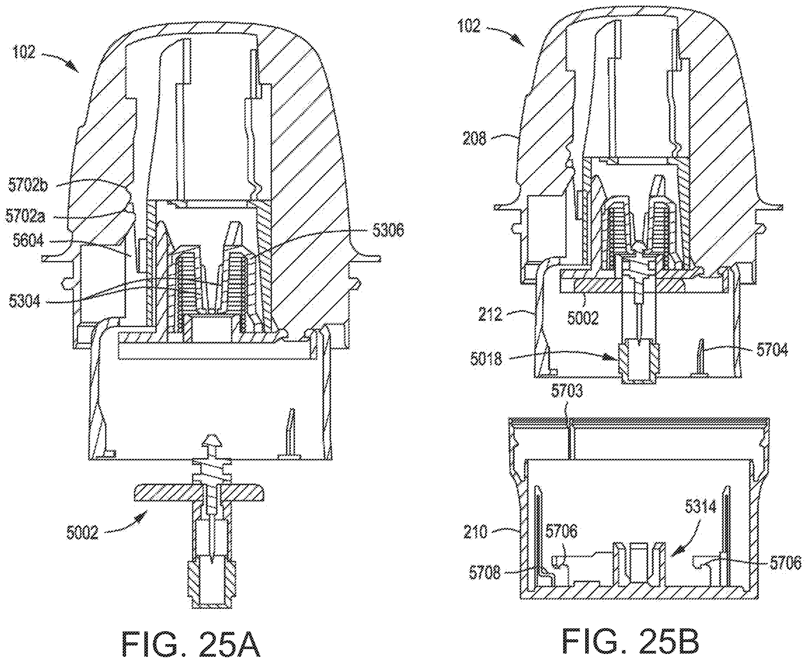

FIGS. 25A-25C are progressive cross-sectional side views showing assembly and disassembly of an alternative embodiment of the sensor applicator 102 with the sensor control device 5002, according to one or more additional embodiments. A fully assembled sensor control device 5002 may be loaded into the sensor applicator 102 by coupling the hub snap pawl 5302 into the arms 5304 of the sharp carrier 5306 positioned within the sensor applicator 102, as generally described above.

In the illustrated embodiment, the sheath arms 5604 of the sheath 212 may be configured to interact with a first detent 5702a and a second detent 5702b defined within the interior of the housing 208. The first detent 5702a may alternately be referred to a "locking" detent, and the second detent 5702b may alternately be referred to as a "firing" detent. When the sensor control device 5002 is initially installed in the sensor applicator 102, the sheath arms 5604 may be received within the first detent 5702a. As discussed below, the sheath 212 may be actuated to move the sheath arms 5604 to the second detent 5702b, which places the sensor applicator 102 in firing position.

In FIG. 25B, the applicator cap 210 is aligned with the housing 208 and advanced toward the housing 208 so that the sheath 212 is received within the applicator cap 210. Instead of rotating the applicator cap 210 relative to the housing 208, the threads of the applicator cap 210 may be snapped onto the corresponding threads of the housing 208 to couple the applicator cap 210 to the housing 208. Axial cuts or slots 5703 (one shown) defined in the applicator cap 210 may allow portions of the applicator cap 210 near its threading to flex outward to be snapped into engagement with the threading of the housing 208. As the applicator cap 210 is snapped to the housing 208, the sensor cap 5018 may correspondingly be snapped into the cap post 5314.

Similar to the embodiment of FIGS. 21A-21C, the sensor applicator 102 may include a sheath locking mechanism configured to ensure that the sheath 212 does not prematurely collapse during a shock event. In the illustrated embodiment, the sheath locking mechanism includes one or more ribs 5704 (one shown) defined near the base of the sheath 212 and configured to interact with one or more ribs 5706 (two shown) and a shoulder 5708 defined near the base of the applicator cap 210. The ribs 5704 may be configured to inter-lock between the ribs 5706 and the shoulder 5708 while attaching the applicator cap 210 to the housing 208. More specifically, once the applicator cap 210 is snapped onto the housing 208, the applicator cap 210 may be rotated (e.g., clockwise), which locates the ribs 5704 of the sheath 212 between the ribs 5706 and the shoulder 5708 of the applicator cap 210 and thereby "locks" the applicator cap 210 in place until the user reverse rotates the applicator cap 210 to remove the applicator cap 210 for use. Engagement of the ribs 5704 between the ribs 5706 and the shoulder 5708 of the applicator cap 210 may also prevent the sheath 212 from collapsing prematurely.

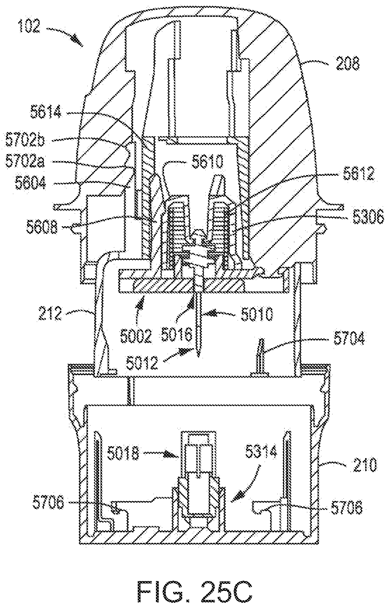

In FIG. 25C, the applicator cap 210 is removed from the housing 208. As with the embodiment of FIGS. 21A-21C, the applicator cap 210 can be removed by reverse rotating the applicator cap 210, which correspondingly rotates the cap post 5314 in the same direction and causes sensor cap 5018 to unthread from the mating member 5016, as generally described above. Moreover, detaching the sensor cap 5018 from the sensor control device 5002 exposes the distal portions of the sensor 5010 and the sharp 5012.

As the applicator cap 210 is unscrewed from the housing 208, the ribs 5704 defined on the sheath 212 may slidingly engage the tops of the ribs 5706 defined on the applicator cap 210. The tops of the ribs 5706 may provide corresponding ramped surfaces that result in an upward displacement of the sheath 212 as the applicator cap 210 is rotated, and moving the sheath 212 upward causes the sheath arms 5604 to flex out of engagement with the first detent 5702a to be received within the second detent 5702b. As the sheath 212 moves to the second detent 5702b, the radial shoulder 5614 moves out of radial engagement with the carrier arm(s) 5608, which allows the passive spring force of the spring 5612 to push upward on the sharp carrier 5306 and force the carrier arm(s) 5608 out of engagement with the groove(s) 5610. As the sharp carrier 5306 moves upward within the housing 208, the mating member 5016 may correspondingly retract until it becomes flush, substantially flush, or sub-flush with the bottom of the sensor control device 5002. At this point, the sensor applicator 102 in firing position. Accordingly, in this embodiment, removing the applicator cap 210 correspondingly causes the mating member 5016 to retract.

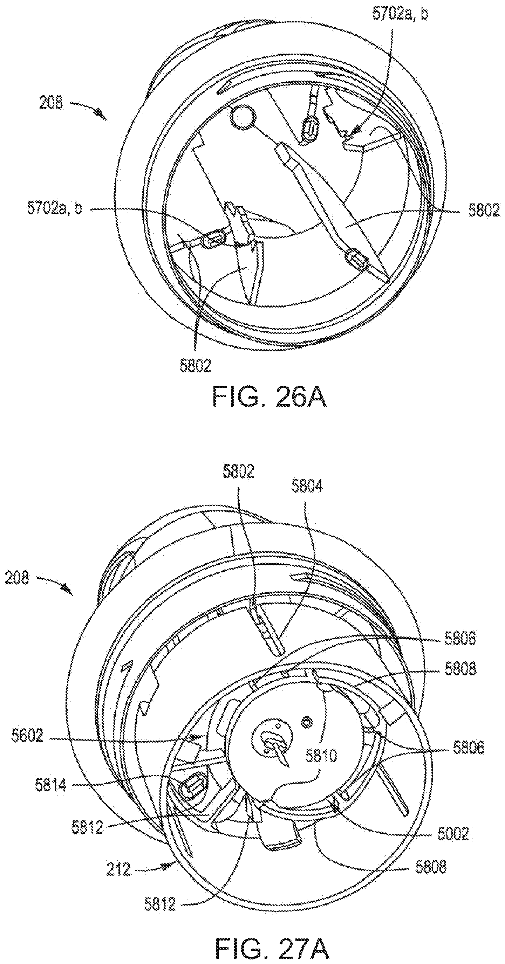

FIG. 26A is an isometric bottom view of the housing 208, according to one or more embodiments. As illustrated, one or more longitudinal ribs 5802 (four shown) may be defined within the interior of the housing 208. The ribs 5802 may be equidistantly or non-equidistantly spaced from each other and extend substantially parallel to centerline of the housing 208. The first and second detents 5702a, b may be defined on one or more of the longitudinal ribs 5802.

FIG. 27A is an isometric bottom view of the housing 208 with the sheath 212 and other components at least partially positioned within the housing 208. As illustrated, the sheath 212 may provide or otherwise define one or more longitudinal slots 5804 configured to mate with the longitudinal ribs 5802 of the housing 208. As the sheath 212 collapses into the housing 208, as generally described above, the ribs 5802 may be received within the slots 5804 to help maintain the sheath 212 aligned with the housing during its movement. As will be appreciated, this may result in tighter circumferential and radial alignment within the same dimensional and tolerance restrictions of the housing 208.

In the illustrated embodiment, the sensor carrier 5602 may be configured to hold the sensor control device 5002 in place both axially (e.g., once the sensor cap 5018 is removed) and circumferentially. To accomplish this, the sensor carrier 5602 may include or otherwise define one or more support ribs 5806 and one or more flexible arms 5808. The support ribs 5806 extend radially inward to provide radial support to the sensor control device 5002. The flexible arms 5808 extend partially about the circumference of the sensor control device 5002 and the ends of the flexible arms 5808 may be received within corresponding grooves 5810 defined in the side of the sensor control device 5002. Accordingly, the flexible arms 5808 may be able to provide both axial and radial support to the sensor control device 5002. In at least one embodiment, the ends of the flexible arms 5808 may be biased into the grooves 5810 of the sensor control device 5002 and otherwise locked in place with corresponding sheath locking ribs 5812 provided by the sheath 212.

In some embodiments, the sensor carrier 5602 may be ultrasonically welded to the housing 208 at one or more points 5814. In other embodiments, however, the sensor carrier 5602 may alternatively be coupled to the housing 208 via a snap-fit engagement. This may help hold the sensor control device 5002 in place during transport and firing.

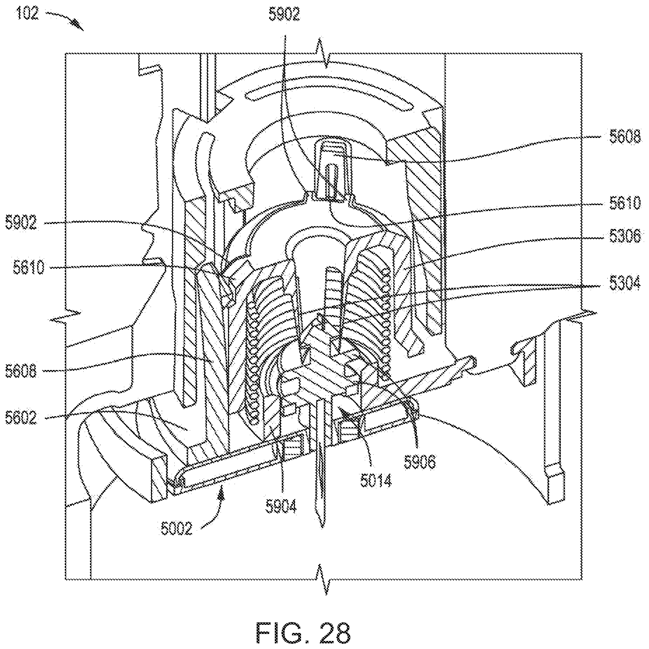

FIG. 28 is an enlarged cross-sectional side view of the sensor applicator 102 with the sensor control device 5002 installed therein, according to one or more embodiments. As discussed above, the sensor carrier 5602 may include one or more carrier arms 5608 (two shown) engageable with the sharp carrier 5306 at corresponding grooves 5610. In at least one embodiment, the grooves 5610 may be defined by pairs of protrusions 5902 defined on the sharp carrier 5306. Receiving the carrier arms 5608 within the grooves 5610 may help stabilize the sharp carrier 5306 from unwanted tilting during all stages of retraction (firing).

In the illustrated embodiment, the arms 5304 of the sharp carrier 5306 may be stiff enough to control, with greater refinement, radial and bi-axial motion of the sharp hub 5014. In some embodiments, for example, clearances between the sharp hub 5014 and the arms 5304 may be more restrictive in both axial directions as the relative control of the height of the sharp hub 5014 may be more critical to the design.

In the illustrated embodiment, the sensor carrier 5602 defines or otherwise provides a central boss 5904 sized to receive the sharp hub 5014. In some embodiments, as illustrated, the sharp hub 5014 may provide one or more radial ribs 5906 (two shown). In at least one embodiment, the inner diameter of the central boss 5904 helps provide radial and tilt support to the sharp hub 5014 during the life of sensor applicator 102 and through all phases of operation and assembly. Moreover, having multiple radial ribs 5906 increases the length-to-width ratio of the sharp hub 5014, which also improves support against tilting.

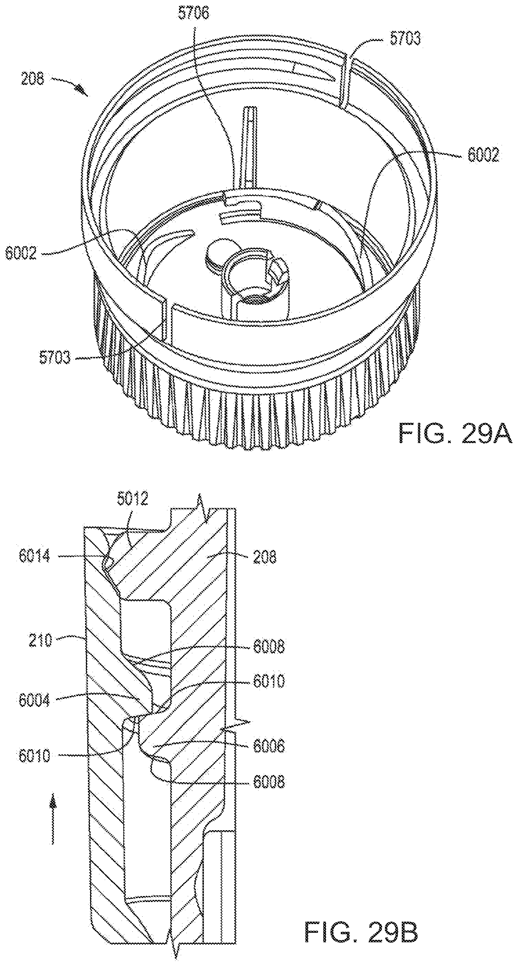

FIG. 29A is an isometric top view of the applicator cap 210, according to one or more embodiments. In the illustrated embodiment, two axial slots 5703 are depicted that separate upper portions of the applicator cap 210 near its threading. As mentioned above, the slots 5703 may help the applicator cap 210 flex outward to be snapped into engagement with the housing 208 (FIG. 25B). In contrast, the applicator cap 210 may be twisted (unthreaded) off the housing 208 by an end user.

FIG. 29A also depicts the ribs 5706 (one visible) defined by the applicator cap 210. By interlocking with the ribs 5704 (FIG. 25C) defined on the sheath 212 (FIG. 25C), the ribs 5706 may help lock the sheath 212 in all directions to prevent premature collapse during a shock or drop event. The sheath 212 may be unlocked when the user unscrews the applicator cap 210 from the housing, as generally described above. As mentioned herein, the top of each rib 5706 may provide a corresponding ramped surface 6002, and as the applicator cap 210 is rotated to unthread from the housing 208, the ribs 5704 defined on the sheath 212 may slidingly engage the ramped surfaces 6002, which results in the upward displacement of the sheath 212 into the housing 208.

In some embodiments, additional features may be provided within the interior of the applicator cap 210 to hold a desiccant component that maintains proper moisture levels through shelf life. Such additional features may be snaps, posts for press-fitting, heat-staking, ultrasonic welding, etc.

FIG. 29B is an enlarged cross-sectional view of the engagement between the applicator cap 210 and the housing 208, according to one or more embodiments. As illustrated, the applicator cap 210 may define a set of inner threads 6004 and the housing 208 may define a set of outer threads 6006 engageable with the inner threads 6004. As mentioned herein, the applicator cap 210 may be snapped onto the housing 208, which may be accomplished by advancing the inner threads 6004 axially past the outer threads 6006 in the direction indicated by the arrow, which causes the applicator cap 210 to flex outward. To help ease this transition, as illustrated, corresponding surfaces 6008 of the inner and outer threads 6004, 6006 may be curved, angled, or chamfered. Corresponding flat surfaces 6010 may be provided on each thread 6004, 6006 and configured to matingly engage once the applicator cap 210 is properly snapped into place on the housing 208. The flat surfaces 6010 may slidingly engage one another as the user unthreads the applicator cap 210 from the housing 208.

The threaded engagement between the applicator cap 210 and the housing 208 results in a sealed engagement that protects the inner components against moisture, dust, etc. In some embodiments, the housing 208 may define or otherwise provide a stabilizing feature 6012 configured to be received within a corresponding groove 1914 defined on the applicator cap 210. The stabilizing feature 6012 may help stabilize and stiffen the applicator cap 210 once the applicator cap 210 is snapped onto the housing 208. This may prove advantageous in providing additional drop robustness to the sensor applicator 102. This may also help increase the removal torque of the applicator cap 210.

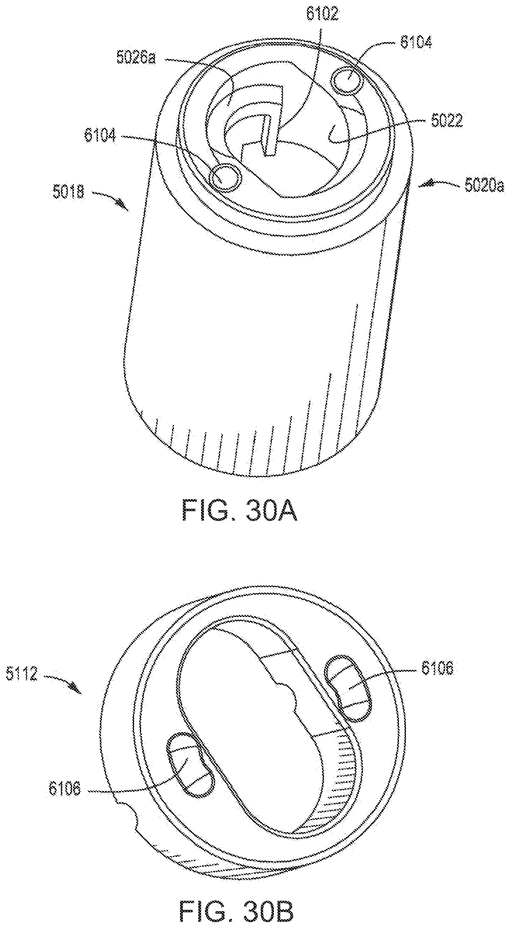

FIGS. 30A and 30B are isometric views of the sensor cap 5018 and the collar 5112, respectively, according to one or more embodiments. Referring to FIG. 30A, in some embodiments, the sensor cap 5018 may comprise an injection molded part. This may prove advantageous in molding the internal threads 5026a defined within the inner chamber 5022, as opposed to installing a threaded core or threading the inner chamber 5022. In some embodiments, one or more stop ribs 6102 (on visible) may be defined within the inner chamber 5022 to prevent over travel relative to mating member 5016 of the sharp hub 5014 (FIGS. 18A-18B).

Referring to both FIGS. 30A and 30B, in some embodiments, one or more protrusions 6104 (two shown) may be defined on the first end 5020a of the sensor cap 5018 and configured to mate with one or more corresponding indentations 6106 (two shown) defined on the collar 5112. In other embodiments, however, the protrusions 6104 may instead be defined on the collar 5112 and the indentations 6106 may be defined on the sensor cap 5018.

The matable protrusions 6104 and indentations 6106 may prove advantageous in rotationally locking the sensor cap 5018 to prevent unintended unscrewing of the sensor cap 5018 from the collar 5112 (and thus the sensor control device 5002) during the life of the sensor applicator 102 and through all phases of operation/assembly. In some embodiments, as illustrated, the indentations 6106 may be formed or otherwise defined in the general shape of a kidney bean. This may prove advantageous in allowing for some over-rotation of the sensor cap 5018 relative to the collar 5112. Alternatively, the same benefit may be achieved via a flat end threaded engagement between the two parts.

Embodiments disclosed herein include:
A. A sensor control device that includes an electronics housing, a sensor arranged within the electronics housing and having a tail extending from a bottom of the electronics housing, a sharp extending through the electronics housing and having a sharp tip extending from the bottom of the electronics housing, and a sensor cap removably coupled at the bottom of the electronics housing and defining a sealed inner chamber that receives the tail and the sharp.
B. An analyte monitoring system that includes a sensor applicator, a sensor control device positioned within the sensor applicator and including an electronics housing, a sensor arranged within the electronics housing and having a tail extending from a bottom of the electronics housing, a sharp extending through the electronics housing and having a sharp tip extending from the bottom of the electronics housing, and a sensor cap removably coupled at the bottom of the electronics housing and defining an engagement feature and a sealed inner chamber that receives the tail and the sharp. The analyte monitoring system may further include a cap coupled to the sensor applicator and providing a cap post defining a receiver feature that receives the engagement feature upon coupling the cap to the sensor applicator, wherein removing the cap from the sensor applicator detaches the sensor cap from the electronics housing and thereby exposes the tail and the sharp tip.
C. A method of preparing an analyte monitoring system that includes loading a sensor control device into a sensor applicator, the sensor control device including an electronics housing, a sensor arranged within the electronics housing and having a tail extending from a bottom of the electronics housing, a sharp extending through the electronics housing and having a sharp tip extending from the bottom of the electronics housing, and a sensor cap removably coupled at the bottom of the electronics housing and defining a sealed inner chamber that receives the tail and the sharp. The method further including securing a cap to the sensor applicator, sterilizing the sensor control device with gaseous chemical sterilization while the sensor control device is positioned within the sensor applicator, and isolating the tail and the sharp tip within the inner chamber from the gaseous chemical sterilization.

Each of embodiments A, B, and C may have one or more of the following additional elements in any combination: Element 1 : wherein the sensor cap comprises a cylindrical body having a first end that is open to access the inner chamber, and a second end opposite the first end and providing an engagement feature engageable with a cap of a sensor applicator, wherein removing the cap from the sensor applicator correspondingly removes the sensor cap from the electronics housing and thereby exposes the tail and the sharp tip. Element 2: wherein the electronics housing includes a shell matable with a mount, the sensor control device further comprising a sharp and sensor locator defined on an inner surface of the shell, and a collar received about the sharp and sensor locator, wherein the sensor cap is removably coupled to the collar. Element 3 : wherein the sensor cap is removably coupled to the collar by one or more of an interference fit, a threaded engagement, a frangible member, and a frangible substance. Element 4: wherein an annular ridge circumscribes the sharp and sensor locator and the collar provides a column and an annular shoulder extending radially outward from the column, and wherein a seal member interposes the annular shoulder and the annular ridge to form a sealed interface. Element 5: wherein the annular ridge defines a groove and a portion of the sensor is seated within the groove, and wherein the seal member extends into the groove to seal about the portion of the sensor. Element 6: wherein the seal member is a first seal member, the sensor control device further comprising a second seal member interposing the annular shoulder and a portion of the mount to form a sealed interface. Element 7: wherein the electronics housing includes a shell matable with a mount, the sensor control device further comprising a sharp hub that carries the sharp and is engageable with a top surface of the shell, and a mating member defined by the sharp hub and extending from the bottom of the electronics housing, wherein the sensor cap is removably coupled to the mating member. Element 8: further comprising a collar at least partially receivable within an aperture defined in the mount and sealingly engaging the sensor cap and an inner surface of the shell. Element 9: wherein a seal member interposes the collar and the inner surface of the shell to form a sealed interface. Element 10: wherein the collar defines a groove and a portion of the sensor is seated within the groove, and wherein the seal member extends into the groove to seal about the portion of the sensor.

Element 11 : wherein the receiver feature comprises one or more compliant members that flex to receive the engagement feature, and wherein the one or more compliant members prevent the engagement feature from exiting the cap post upon removing the cap from the sensor applicator. Element 12: further comprising a ramped surface defined on at least one of the one or more compliant members, and one or more camming surfaces provided by the engagement feature and engageable with the ramped surface, wherein the ramped surface and the one or more camming surfaces allow the cap and the cap post to rotate relative to the sensor cap in a first direction, but prevent the cap and the cap post from rotating relative to the sensor cap in a second direction opposite the first direction. Element 13 : wherein the electronics housing includes a shell matable with a mount, the sensor control device further comprising a sharp hub that carries the sharp and is engageable with a top surface of the shell, and a mating member defined by the sharp hub and extending from the bottom of the electronics housing, wherein the sensor cap is removably coupled to the mating member and rotating the cap in the second direction detaches the sensor cap from the mating member. Element 14: wherein the electronics housing includes a shell matable with a mount and the sensor control device further includes a sharp and sensor locator defined on an inner surface of the shell, and a collar received about the sharp and sensor locator, wherein the sensor cap is removably coupled to the collar.

Element 15: wherein the cap provides a cap post defining a receiver feature and the sensor cap defines an engagement feature, the method further comprising receiving the engagement feature with the receiver feature as the cap is secured to the sensor applicator. Element 16: further comprising removing the cap from the sensor applicator, and engaging the engagement feature on the receiver feature as the cap is being removed and thereby detaching the sensor cap from the electronics housing and exposing the tail and the sharp tip. Element 17: wherein loading the sensor control device into a sensor applicator is preceded by sterilizing the tail and the sharp tip with radiation sterilization, and sealing the tail and the sharp tip within the inner chamber.

By way of non-limiting example, exemplary combinations applicable to A, B, and C include: Element 2 with Element 3; Element 2 with Element 4; Element 4 with Element 5; Element 4 with Element 6; Element 7 with Element 8; Element 8 with Element 9; Element 9 with Element 10; Element 11 with Element 12; and Element 15 with Element 16.

### Example Embodiments of Seal Arrangement for Analyte Monitoring Systems

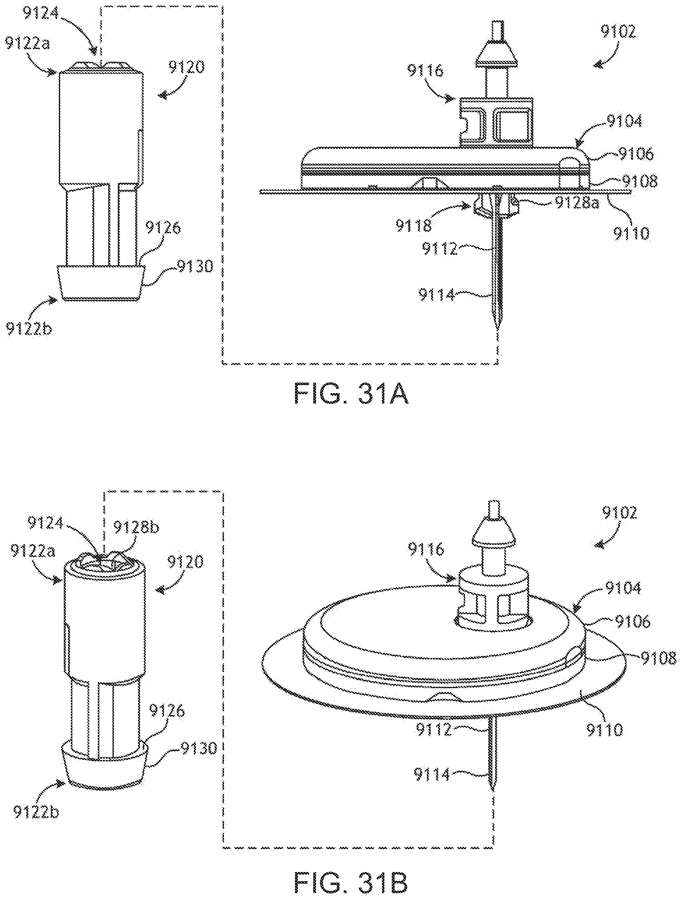

FIGS. 31A and 31B are side and isometric views, respectively, of an example sensor control device 9102, according to one or more embodiments of the present disclosure. The sensor control device 9102 may be similar in some respects to the sensor control device 102 of FIG. 1 and therefore may be best understood with reference thereto. Moreover, the sensor control device 9102 may replace the sensor control device 102 of FIG. 1 and, therefore, may be used in conjunction with the sensor applicator 102 of FIG. 1, which may deliver the sensor control device 9102 to a target monitoring location on a user's skin.

As illustrated, the sensor control device 9102 includes an electronics housing 9104, which may be generally disc-shaped and have a circular cross-section. In other embodiments, however, the electronics housing 9104 may exhibit other cross-sectional shapes, such as ovoid, oval, or polygonal The electronics housing 9104 includes a shell 9106 and a mount 9108 that is matable with the shell 9106. The shell 9106 may be secured to the mount 9108 via a variety of ways, such as a snap fit engagement, an interference fit, sonic welding, laser welding, one or more mechanical fasteners (e.g., screws), a gasket, an adhesive, or any combination thereof. In some cases, the shell 9106 may be secured to the mount 9108 such that a sealed interface is generated therebetween. An adhesive patch 9110 may be positioned on and otherwise attached to the underside of the mount 9108. Similar to the adhesive patch 108 of FIG. 1, the adhesive patch 9110 may be configured to secure and maintain the sensor control device 9102 in position on the user's skin during operation.

The sensor control device 9102 may further include a sensor 9112 and a sharp 9114 used to help deliver the sensor 9112 transcutaneously under a user's skin during application of the sensor control device 9102. Corresponding portions of the sensor 9112 and the sharp 9114 extend distally from the bottom of the electronics housing 9104 (e.g., the mount 9108). A sharp hub 9116 may be overmolded onto the sharp 9114 and configured to secure and carry the sharp 9114. As best seen in FIG. 31A, the sharp hub 9116 may include or otherwise define a mating member 9118. In assembling the sharp 9114 to the sensor control device 9102, the sharp 9114 may be advanced axially through the electronics housing 9104 until the sharp hub 9116 engages an upper surface of the electronics housing 9104 or an internal component thereof and the mating member 9118 extends distally from the bottom of the mount 9108. As described herein below, in at least one embodiment, the sharp hub 9116 may sealingly engage an upper portion of a seal overmolded onto the mount 9108. As the sharp 9114 penetrates the electronics housing 9104, the exposed portion of the sensor 9112 may be received within a hollow or recessed (arcuate) portion of the sharp 9114. The remaining portion of the sensor 9112 is arranged within the interior of the electronics housing 9104.

The sensor control device 9102 may further include a sensor cap 9120, shown detached from the electronics housing 9104 in FIGS. 31A-31B. The sensor cap 9120 may help provide a sealed barrier that surrounds and protects exposed portions of the sensor 9112 and the sharp 9114. As illustrated, the sensor cap 9120 may comprise a generally cylindrical body having a first end 9122a and a second end 9122b opposite the first end 9122a. The first end 9122a may be open to provide access into an inner chamber 9124 defined within the body. In contrast, the second end 9l22b may be closed and may provide or otherwise define an engagement feature 9126. As described in more detail below, the engagement feature 9126 may help mate the sensor cap 9120 to an applicator cap of a sensor applicator (e.g., the sensor applicator 102 of FIG. 1), and may help remove the sensor cap 9120 from the sensor control device 9102 upon removing the sensor cap from the sensor applicator.

The sensor cap 9120 may be removably coupled to the electronics housing 9104 at or near the bottom of the mount 9108. More specifically, the sensor cap 9120 may be removably coupled to the mating member 9118, which extends distally from the bottom of the mount 9108. In at least one embodiment, for example, the mating member 9118 may define a set of external threads 9l28a (FIG. 31A) matable with a set of internal threads 9l28b (FIG. 31B) defined within the inner chamber 9124 of the sensor cap 9120. In some embodiments, the external and internal threads 9l28a,b may comprise a flat thread design (e.g., lack of helical curvature), but may alternatively comprise a helical threaded engagement. Accordingly, in at least one embodiment, the sensor cap 9120 may be threadably coupled to the sensor control device 9102 at the mating member 9118 of the sharp hub 9116. In other embodiments, the sensor cap 9120 may be removably coupled to the mating member 9118 via other types of engagements including, but not limited to, an interference or friction fit, or a frangible member or substance (e.g., wax, an adhesive, etc.) that may be broken with minimal separation force (e.g., axial or rotational force).

In some embodiments, the sensor cap 9120 may comprise a monolithic (singular) structure extending between the first and second ends 9l22a,b. In other embodiments, however, the sensor cap 9120 may comprise two or more component parts. In the illustrated embodiment, for example, the body of the sensor cap 9120 may include a desiccant cap 9130 arranged at the second end 9l22b. The desiccant cap 9130 may house or comprise a desiccant to help maintain preferred humidity levels within the inner chamber 9124. Moreover, the desiccant cap 9130 may also define or otherwise provide the engagement feature 9126 of the sensor cap 9120. In at least one embodiment, the desiccant cap 9130 may comprise an elastomeric plug inserted into the bottom end of the sensor cap 9120.

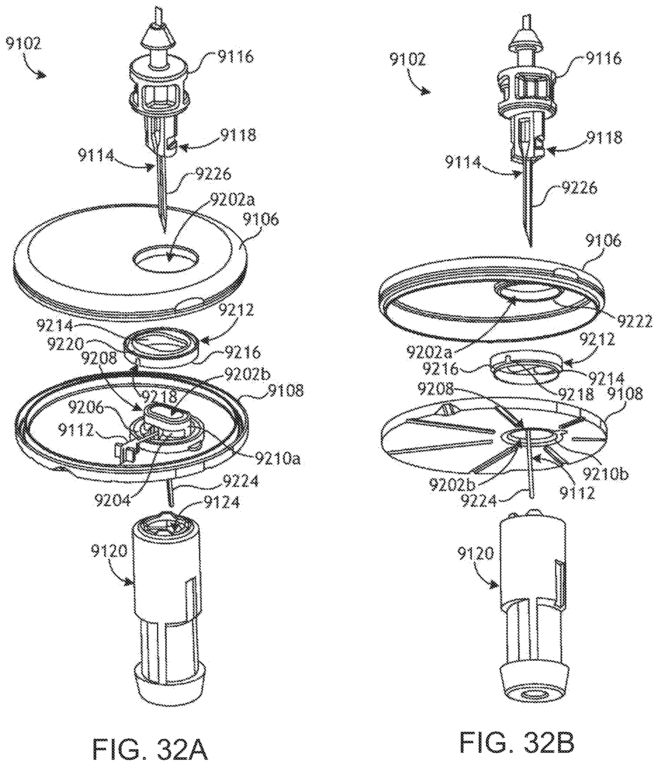

FIGS. 32A and 32B are exploded, isometric top and bottom views, respectively, of the sensor control device 9102, according to one or more embodiments. The shell 9106 and the mount 9108 operate as opposing clamshell halves that enclose or otherwise substantially encapsulate various electronic components (not shown) of the sensor control device 9102. Example electronic components that may be arranged between the shell 9106 and the mount 9108 include, but are not limited to, a battery, resistors, transistors, capacitors, inductors, diodes, and switches.

The shell 9106 may define a first aperture 9202a and the mount 9108 may define a second aperture 9202b, and the apertures 9202a, b may align when the shell 9106 is properly mounted to the mount 9108. As best seen in FIG. 32A, the mount 9108 may provide or otherwise define a pedestal 9204 that protrudes from the inner surface of the mount 9108 at the second aperture 9202b. The pedestal 9204 may define at least a portion of the second aperture 9202b. Moreover, a channel 9206 may be defined on the inner surface of the mount 9108 and may circumscribe the pedestal 9202. In the illustrated embodiment, the channel 9206 is circular in shape, but could alternatively be another shape, such as oval, ovoid, or polygonal.

The mount 9108 may comprise a molded part made of a rigid material, such as plastic or metal. In some embodiments, a seal 9208 may be overmolded onto the mount 9108 and may be made of an elastomer, rubber, a -polymer, or another pliable material suitable for facilitating a sealed interface. In embodiments where the mount 9108 is made of a plastic, the mount 9108 may be molded in a first "shot" of injection molding, and the seal 9208 may be overmolded onto the mount 9108 in a second "shot" of injection molding. Accordingly, the mount 9108 may be referred to or otherwise characterized as a "two-shot mount."

In the illustrated embodiment, the seal 9208 may be overmolded onto the mount 9108 at the pedestal 9204 and also on the bottom of the mount 9108. More specifically, the seal 9208 may define or otherwise provide a first seal element 92l0a overmolded onto the pedestal 9204, and a second seal element 9210b (FIG. 32B) interconnected to (with) the first seal element 92l0a and overmolded onto the mount 9108 at the bottom of the mount 9108. In some embodiments, one or both of the seal elements 92l0a,b may help form corresponding portions (sections) of the second aperture 9202b. While the seal 9208 is described herein as being overmolded onto the mount 9108, it is also contemplated herein that one or both of the seal elements 92l0a,b may comprise an elastomeric component part independent of the mount 9208, such as an O-ring or a gasket.

The sensor control device 9102 may further include a collar 9212 disposed between shell 9106 and mount 9208 and may be a generally annular structure that defines a central aperture 9214. The central aperture 9214 may be sized to receive the first seal element 92l0a and may align with both the first and second apertures 9202a, b when the sensor control device 9102 is properly assembled. The shape of the central aperture 9214 may generally match the shape of the second aperture 9202b and the first seal element 92l0a.

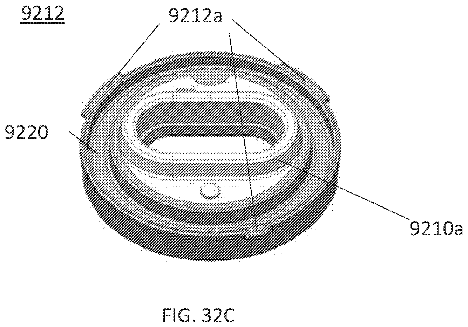

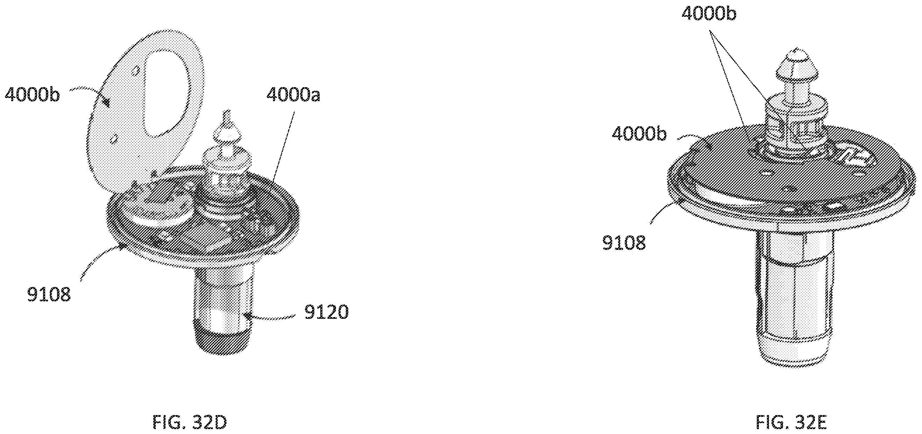

In some embodiments, the collar 9212 may define or otherwise provide an annular lip 9216 on its bottom surface. The annular lip 9216 may be sized and otherwise configured to mate with or be received into the channel 9206 defined on the inner surface of the mount 9108. In some embodiments, a groove 9218 may be defined on the annular lip 9216 and may be configured to accommodate or otherwise receive a portion of the sensor 9112 extending laterally within the mount 9108. In some embodiments, the collar 9212 may further define or otherwise provide a collar channel 9220 (FIG. 32A) on its upper surface sized to receive and otherwise mate with an annular ridge 9222 (FIG. 32B) defined on the inner surface of the shell 9106 when the sensor control device 9102 is properly assembled. In some embodiments, collar 9212 may further include a plurality of tabs 9212a (FIG. 32C) on its outer periphery. Tabs 9212a may be located on the same plane as the top surface of collar 9212 and create a shelf for a circuit board. In some embodiments, the circuit board can be foldable and/or a flexible PCB, as described in U.S. Provisional Application No. 63/081,223, filed September 21, 2020. For example, PCB 4000 can be mounted within the electronics housing on the plurality of tabs 9212a. As can be seen in FIG. 32D, after PCB 4000 has been loaded onto the sensor mount 9108, first portion 4000a of PCB may be located under tabs 9212a (not shown in FIG. 32D) and rest on the sensor mount 9108. Thereafter, second portion 4000b of PCB 4000 may be folded over first portion 4000a and mounted on the plurality of tabs 9212a. In some embodiments, tabs 9212a can be of the same size or of different sizes and may be equally spaced along the collar 9212's outer periphery. In some embodiments, tabs 9212a can extend from the top surface of the collar 9212, the bottom surface of the collar 9212, or an intermediate location of the collar 9212 therebetween. As shown in FIG. 32C, collar 9212 can include three tabs 9212a and tabs 9212a can be of different sizes, for example, with two tabs being the same size as each other and longer (i.e., as measured along the outer periphery of collar 9212) than a third tab. Additionally, tabs 9212a can be the same or different widths (i.e., measured as distance extending radially outwards). In some embodiments, as can be seen in FIG. 32E, PCB 4000 can include one or more tabs 4000c corresponding to tabs 9212a and mated with and fixed to tabs 9212a.

The sensor 9112 may include a tail 9224 that extends through the second aperture 9202b defined in the mount 9108 to be transcutaneously received beneath a user's skin. The tail 9224 may have an enzyme or other chemistry included thereon to help facilitate analyte monitoring. The sharp 9114 may include a sharp tip 9226 extendable through the first aperture 9202a defined by the shell 9106. As the sharp tip 9226 penetrates the electronics housing 9104, the tail 9224 of the sensor 9112 may be received within a hollow or recessed portion of the sharp tip 9226. The sharp tip 9226 may be configured to penetrate the skin while carrying the tail 9224 to put the active chemistry of the tail 9224 into contact with bodily fluids.

The sensor control device 9102 may provide a sealed subassembly that includes, among other component parts, portions of the shell 9106, the sensor 9112, the sharp 9114, the seal 9208, the collar 9212, and the sensor cap 9120. The sealed subassembly may help isolate the sensor 9112 and the sharp 9114 within the inner chamber 9124 (FIG. 32A) of the sensor cap 9120. In assembling the sealed subassembly, the sharp tip 9226 is advanced through the electronics housing 9104 until the sharp hub 9116 engages the seal 9208 and, more particularly, the first seal element 92l0a. The mating member 9118 provided at the bottom of the sharp hub 9116 may extend out the second aperture 9202b in the bottom of the mount 9108, and the sensor cap 9120 may be coupled to the sharp hub 9116 at the mating member 9118. Coupling the sensor cap 9120 to the sharp hub 9116 at the mating member 9118 may urge the first end 9122a of the sensor cap 9120 into sealed engagement with the seal 9208 and, more particularly, into sealed engagement with the second seal element 9210b on the bottom of the mount 9108. In some embodiments, as the sensor cap 9120 is coupled to the sharp hub 9116, a portion of the first end 9l22a of the sensor cap 9120 may bottom out (engage) against the bottom of the mount 9108, and the sealed engagement between the sensor hub 9116 and the first seal element 92l0a may be able to assume any tolerance variation between features.

FIG. 33 is a cross-sectional side view of the sensor control device 9102, according to one or more embodiments. As indicated above, the sensor control device 9102 may include or otherwise incorporate a sealed subassembly 9302, which may be useful in isolating the sensor 9112 and the sharp 9114 within the inner chamber 9124 of the sensor cap 9120. To assemble the sealed subassembly 9302, the sensor 9112 may be located within the mount 9108 such that the tail 9224 extends through the second aperture 9202b at the bottom of the mount 9108. In at least one embodiment, a locating feature 9304 may be defined on the inner surface of the mount 9108, and the sensor 9112 may define a groove 9306 that is matable with the locating feature 9304 to properly locate the sensor 9112 within the mount 9108.

Once the sensor 9112 is properly located, the collar 9212 may be installed on the mount 9108. More specifically, the collar 9212 may be positioned such that the first seal element 92l0a of the seal 9208 is received within the central aperture 9214 defined by the collar 9212 and the first seal element 92l0a generates a radial seal against the collar 9212 at the central aperture 9214. Moreover, the annular lip 9216 defined on the collar 9212 may be received within the channel 9206 defined on the mount 9108, and the groove 9218 defined through the annular lip 9216 may be aligned to receive the portion of the sensor 9112 that traverses the channel 9206 laterally within the mount 9108. In some embodiments, an adhesive may be injected into the channel 9206 to secure the collar 9212 to the mount 9108. The adhesive may also facilitate a sealed interface between the two components and generate a seal around the sensor 9112 at the groove 9218, which may isolate the tail 9224 from the interior of the electronics housing 9104.

The shell 9106 may then be mated with or otherwise coupled to the mount 9108. In some embodiments, as illustrated, the shell 9106 may mate with the mount 9108 via a tongue-and-groove engagement 9308 at the outer periphery of the electronics housing 9104. An adhesive may be injected (applied) into the groove portion of the engagement 9308 to secure the shell 9106 to the mount 9108, and also to create a sealed engagement interface. Mating the shell 9106 to the mount 9108 may also cause the annular ridge 9222 defined on the inner surface of the shell 9106 to be received within the collar channel 9220 defined on the upper surface of the collar 9212. In some embodiments, an adhesive may be injected into the collar channel 9220 to secure the shell 9106 to the collar 9212, and also to facilitate a sealed interface between the two components at that location. When the shell 9106 mates with the mount 9108, the first seal element 92l0a may extend at least partially through (into) the first aperture 9202a defined in the shell 9106.

The sharp 9114 may then be coupled to the sensor control device 9102 by extending the sharp tip 9226 through the aligned first and second apertures 9202a, b defined in the shell 9106 and the mount 9108, respectively. The sharp 9114 may be advanced until the sharp hub 9116 engages the seal 9208 and, more particularly, engages the first seal element 92l0a. The mating member 9118 may extend (protrude) out the second aperture 9202b at the bottom of the mount 9108 when the sharp hub 9116 engages the first seal element 92l0a.

The sensor cap 9120 may then be removably coupled to the sensor control device 9102 by threadably mating the internal threads 9128b of the sensor cap 9120 with the external threads 9l28a of the mating member 9118. The inner chamber 9124 may be sized and otherwise configured to receive the tail 9224 and the sharp tip 9226 extending from the bottom of the mount 9108. Moreover, the inner chamber 9124 may be sealed to isolate the tail 9224 and the sharp tip 9226 from substances that might adversely interact with the chemistry of the tail 9224. In some embodiments, a desiccant (not shown) may be present within the inner chamber 9124 to maintain proper humidity levels.

Tightening (rotating) the mated engagement between the sensor cap 9120 and the mating member 9118 may urge the first end 9122a of the sensor cap 9120 into sealed engagement with the second seal element 9210b in an axial direction (e.g., along the centerline of the apertures 9202a, b), and may further enhance the sealed interface between the sharp hub 9116 and the first seal element 92l0a in the axial direction. Moreover, tightening the mated engagement between the sensor cap 9120 and the mating member 9118 may compress the first seal element 92l0a, which may result in an enhanced radial sealed engagement between the first seal element 92l0a and the collar 9212 at the central aperture 9214. Accordingly, in at least one embodiment, the first seal element 92l0a may help facilitate axial and radial sealed engagements.

As mentioned above, the first and second seal elements 92l0a,b may be overmolded onto the mount 9108 and may be physically linked or otherwise interconnected. Consequently, a single injection molding shot may flow through the second aperture 9202b of the mount 9108 to create both ends of the seal 9208. This may prove advantageous in being able to generate multiple sealed interfaces with only a single injection molded shot. An additional advantage of a two-shot molded design, as opposed to using separate elastomeric components (e.g., O-rings, gaskets, etc.), is that the interface between the first and second shots is a reliable bond rather than a mechanical seal. Hence, the effective number of mechanical sealing barriers is effectively cut in half. Moreover, a two-shot component with a single elastomeric shot also has implications to minimizing the number of two-shot components needed to achieve all the necessary sterile barriers. Once properly assembled, the sealed subassembly 9302 may be subjected to a radiation sterilization process to sterilize the sensor 9112 and the sharp 9114. The sealed subassembly 9302 may be subjected to the radiation sterilization prior to or after coupling the sensor cap 9120 to the sharp hub 9116. When sterilized after coupling the sensor cap 9120 to the sharp hub 9116, the sensor cap 9120 may be made of a material that permits the propagation of radiation therethrough. In some embodiments, the sensor cap 9120 may be transparent or translucent, but can otherwise be opaque.

FIG. 33A is an exploded isometric view of a portion of another embodiment of the sensor control device 9102 of FIGS. 31A-31B and 32A-32B. Embodiments included above describe the mount 9108 and the seal 9208 being manufactured via a two-shot injection molding process. In other embodiments, however, as briefly mentioned above, one or both of the seal elements 92l0a,b of the seal 9208 may comprise an elastomeric component part independent of the mount 9208. In the illustrated embodiment, for example, the first seal element 92l0a may be overmolded onto the collar 9212 and the second seal element 92l0b may be overmolded onto the sensor cap 9120. Alternatively, the first and second seal elements 92l0a,b may comprise a separate component part, such as a gasket or O-ring 9210a positioned on the upper surface of the collar 9212 (FIGS. 32C and 33A) and the sensor cap 9120, respectively. Tightening (rotating) the mated engagement between the sensor cap 9120 and the mating member 9118 may urge the second seal element 9210b into sealed engagement with the bottom of the mount 9108 in an axial direction, and may enhance a sealed interface between the sharp hub 9116 and the first seal element 92l0a in the axial direction. In some embodiments, as shown in FIG. 33B, sharp hub 9116 may include a raised mating surface 9116a (e.g., a ledge) configured to mate with seal element 9210a. As such, tightening (rotating) the mated engagement between the sensor cap 9120 and the mating member 9118 may enhance a sealed interface between the mating surface 9116a and the first seal element 92l0a in the axial direction.

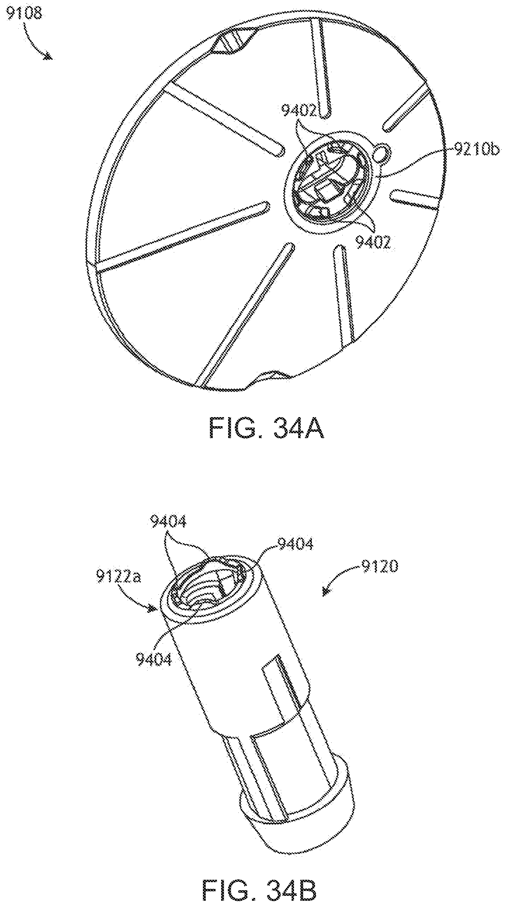

FIG. 34A is an isometric bottom view of the mount 9108, and FIG. 34B is an isometric top view of the sensor cap 9120, according to one or more embodiments. As shown in FIG. 34A, the mount 9108 may provide or otherwise define one or more indentations or pockets 9402 at or near the opening to the second aperture 9202b. As shown in FIG. 34B, the sensor cap 9120 may provide or otherwise define one or more projections 9404 at or near the first end 9l22a of the sensor cap 9120. The projections 9404 may be received within the pockets 9402 when the sensor cap 9120 is coupled to the sharp hub 9116 (FIGS. 32A-32B and 93). More specifically, as described above, as the sensor cap 9120 is coupled to the mating member 9118 (FIGS. 32A-32B and 93) of the sensor hub 9116, the first end 9l22a of the sensor cap 9120 is brought into sealed engagement with the second seal element 92l0b. In this process, the projections 9404 may also be received within the pockets 9402, which may help prevent premature unthreading of the sensor cap 9120 from the sharp hub 9116.

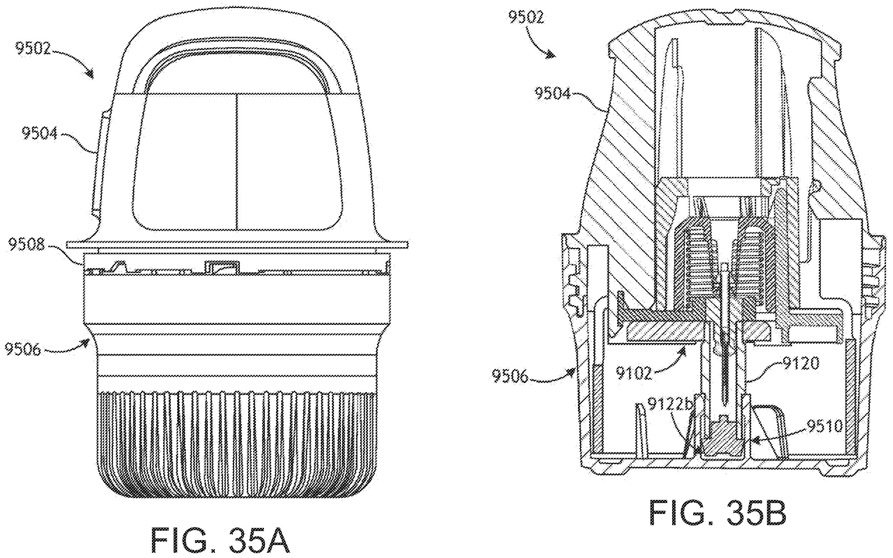

FIGS. 35A and 35B are side and cross-sectional side views, respectively, of an example sensor applicator 9502, according to one or more embodiments. The sensor applicator 9502 may be similar in some respects to the sensor applicator 102 of FIG. 1 and, therefore, may be designed to deliver (fire) a sensor control device, such as the sensor control device 9102. FIG. 35A depicts how the sensor applicator 9502 might be shipped to and received by a user, and FIG. 35B depicts the sensor control device 9102 arranged within the interior of the sensor applicator 9502.

As shown in FIG. 35A, the sensor applicator 9502 includes a housing 9504 and an applicator cap 9506 removably coupled to the housing 9504. In some embodiments, the applicator cap 9506 may be threaded to the housing 9504 and include a tamper ring 9508. Upon rotating (e.g., unscrewing) the applicator cap 9506 relative to the housing 9504, the tamper ring 9508 may shear and thereby free the applicator cap 9506 from the sensor applicator 9502.

In FIG. 35B, the sensor control device 9102 is positioned within the sensor applicator 9502. Once the sensor control device 9102 is fully assembled, it may then be loaded into the sensor applicator 9502 and the applicator cap 9506 may be coupled to the sensor applicator 9502. In some embodiments, the applicator cap 9506 and the housing 9504 may have opposing, matable sets of threads that enable the applicator cap 9506 to be screwed onto the housing 9504 in a clockwise (or counter-clockwise) direction and thereby secure the applicator cap 9506 to the sensor applicator 9502.

Securing the applicator cap 9506 to the housing 9504 may also cause the second end 9l22b of the sensor cap 9120 to be received within a cap post 9510 located within the interior of the applicator cap 9506 and extending proximally from the bottom thereof. The cap post 9510 may be configured to receive at least a portion of the sensor cap 9120 as the applicator cap 9506 is coupled to the housing 9504.

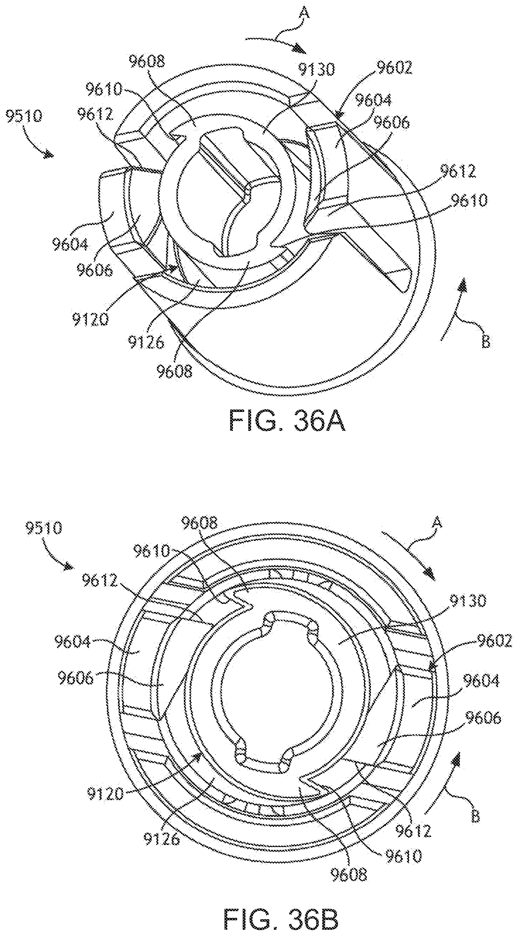

FIGS. 36A and 36B are perspective and top views, respectively, of the cap post 9510, according to one or more additional embodiments. In the illustrated depiction, a portion of the sensor cap 9120 is received within the cap post 9510 and, more specifically, the desiccant cap 9130 of the sensor cap 9120 is arranged within cap post 9510. The cap post 9510 may define a receiver feature 9602 configured to receive the engagement feature 9126 of the sensor cap 9120 upon coupling (e.g., threading) the applicator cap 9506 (FIG. 35B) to the sensor applicator 9502 (FIGS. 35A-35B). Upon removing the applicator cap 9506 from the sensor applicator 9502, however, the receiver feature 9602 may prevent the engagement feature 9126 from reversing direction and thus prevent the sensor cap 9120 from separating from the cap post 9510. Instead, removing the applicator cap 9506 from the sensor applicator 9502 will simultaneously detach the sensor cap 9120 from the sensor control device 9102 (FIGS. 31A-31B and 32A-32B), and thereby expose the distal portions of the sensor 9112 (FIGS. 32A-32B) and the sharp 9114 (FIGS. 32A-32B).

Many design variations of the receiver feature 9602 may be employed. In the illustrated embodiment, the receiver feature 9602 includes one or more compliant members 9604 (two shown) that are expandable or flexible to receive the engagement feature 9126. The engagement feature 9126 may comprise, for example, an enlarged head and the compliant member(s) 9604 may comprise a collet-type device that includes a plurality of compliant fingers configured to flex radially outward to receive the enlarged head.

The compliant member(s) 9604 may further provide or otherwise define corresponding ramped surfaces 9606 configured to interact with one or more opposing camming surfaces 9608 provided on the outer wall of the engagement feature 9126. The configuration and alignment of the ramped surface(s) 9606 and the opposing camming surface(s) 9608 is such that the applicator cap 9506 is able to rotate relative to the sensor cap 9120 in a first direction A (e.g., clockwise), but the cap post 9510 binds against the sensor cap 9120 when the applicator cap 9506 is rotated in a second direction B (e.g., counter clockwise). More particularly, as the applicator cap 9506 (and thus the cap post 9510) rotates in the first direction A, the camming surfaces 9608 engage the ramped surfaces 9606, which urge the compliant members 9604 to flex or otherwise deflect radially outward and results in a ratcheting effect. Rotating the applicator cap 9506 (and thus the cap post 9510) in the second direction B, however, will drive angled surfaces 9610 of the camming surfaces 9608 into opposing angled surfaces 9612 of the ramped surfaces 9606, which results in the sensor cap 9120 binding against the compliant member(s) 9604.

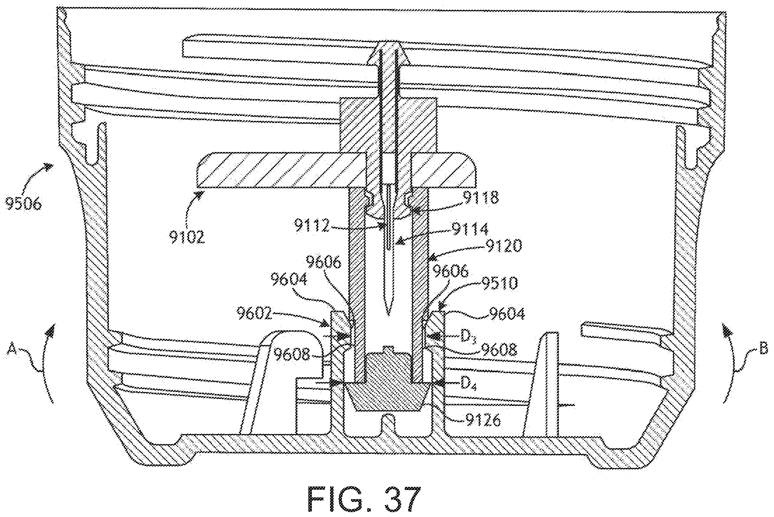

FIG. 37 is a cross-sectional side view of the sensor control device 9102 positioned within the applicator cap 9506, according to one or more embodiments. As illustrated, the opening to the receiver feature 9602 exhibits a first diameter D₃, while the engagement feature 9126 of the sensor cap 9120 exhibits a second diameter D₄ that is larger than the first diameter D₃ and greater than the outer diameter of the remaining portions of the sensor cap 9120. As the sensor cap 9120 is extended into the cap post 9510, the compliant member(s) 9604 of the receiver feature 9602 may flex (expand) radially outward to receive the engagement feature 9126. In some embodiments, as illustrated, the engagement feature 9126 may provide or otherwise define an angled outer surface that helps bias the compliant member(s) 9604 radially outward. Once the engagement feature 9126 bypasses the receiver feature 9602, the compliant member(s) 9604 are able to flex back to (or towards) their natural state and thus lock the sensor cap 9120 within the cap post 9510.

As the applicator cap 9506 is threaded to (screwed onto) the housing 9504 (FIGS. 35A-35B) in the first direction A, the cap post 9510 correspondingly rotates in the same direction and the sensor cap 9120 is progressively introduced into the cap post 9510. As the cap post 9510 rotates, the ramped surfaces 9606 of the compliant members 9604 ratchet against the opposing camming surfaces 9608 of the sensor cap 9120. This continues until the applicator cap 9506 is fully threaded onto (screwed onto) the housing 9504. In some embodiments, the ratcheting action may occur over two full revolutions of the applicator cap 9506 before the applicator cap 9506 reaches its final position.

To remove the applicator cap 9506, the applicator cap 9506 is rotated in the second direction B, which correspondingly rotates the cap post 9510 in the same direction and causes the camming surfaces 9608 (i.e., the angled surfaces 9610 of FIGS. 36A-36B) to bind against the ramped surfaces 9606 (i.e., the angled surfaces 9612 of FIGS. 36A-36B). Consequently, continued rotation of the applicator cap 9506 in the second direction B causes the sensor cap 9120 to correspondingly rotate in the same direction and thereby unthread from the mating member 9118 to allow the sensor cap 9120 to detach from the sensor control device 9102. Detaching the sensor cap 9120 from the sensor control device 9102 exposes the distal portions of the sensor 9112 and the sharp 9114, and thus places the sensor control device 9102 in position for firing (use).

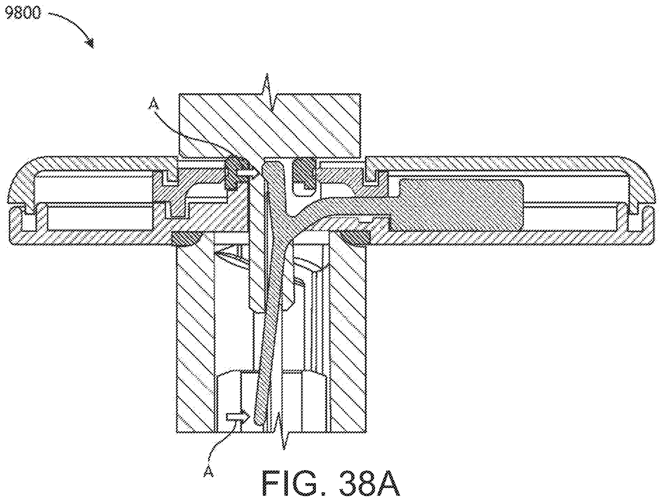

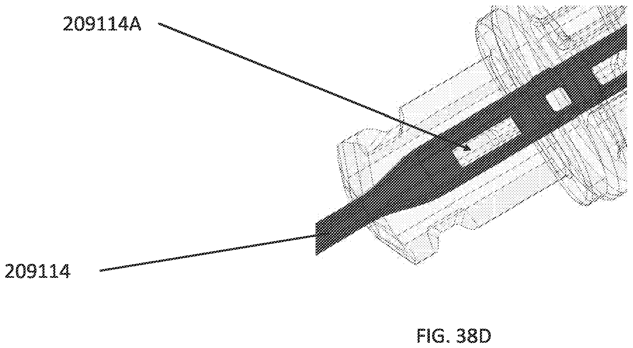

FIG. 38A is a cross-sectional view of a sensor control device 9800 showing example interaction between the sensor and the sharp. After assembly of the sharp, the sensor should sit in a channel defined by the sharp. The sensor control device in FIG. 9 does not show the sensor deflected inwards and otherwise aligned fully with the sharp, but such may be the case upon full assembly as slight bias forces may be assumed by the sensor at the locations indicated by the two arrows A. Biasing the sensor against the sharp may be advantageous so that any relative motion between the sensor and the sharp during subcutaneous insertion does not expose the sensor tip (i.e., the tail) outside the sharp channel, which could potentially cause an insertion failure.

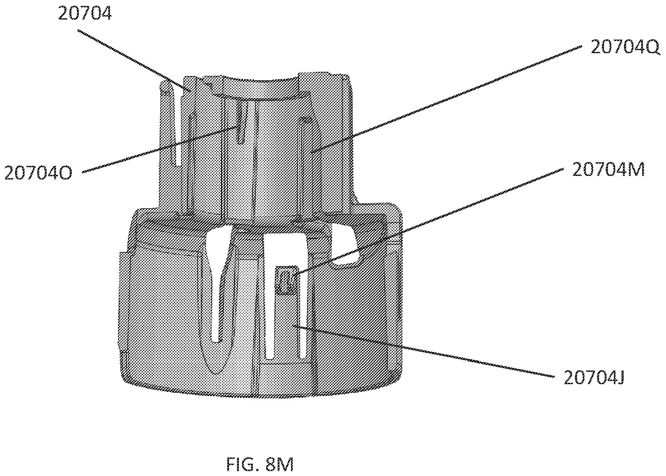

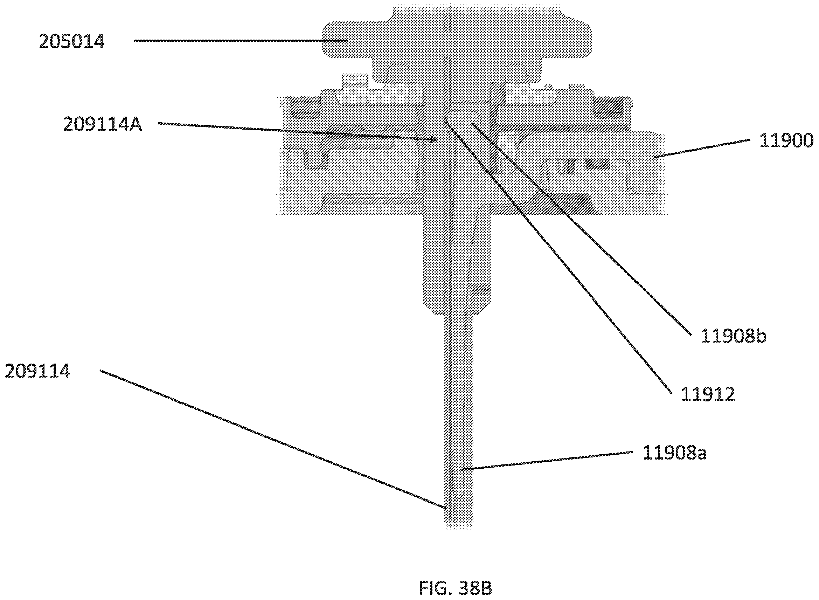

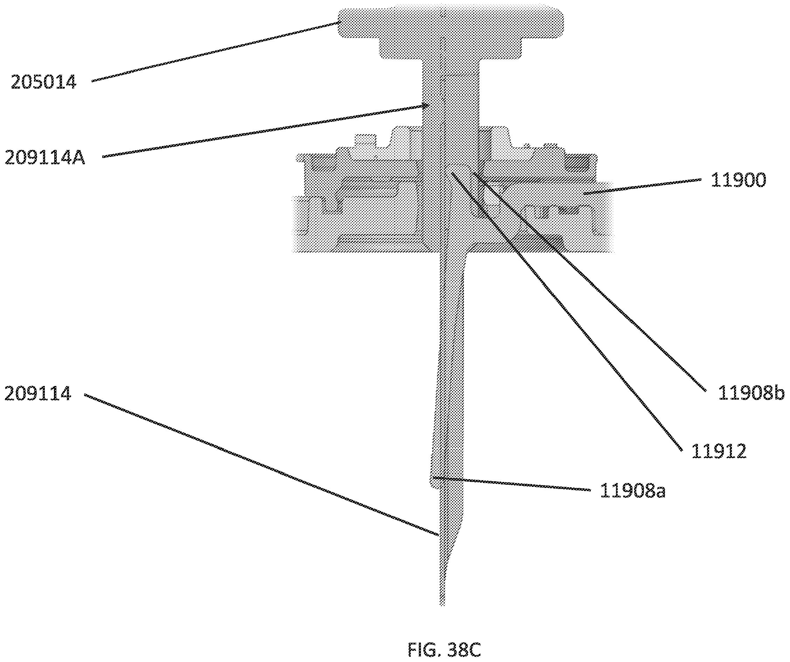

FIGs. 38B-38D illustrates an exemplary sharp hub 205014 and sharp 209114 configured to not bias the sensor 11900 prior to delivery, for example, during shipping and storage (FIG. 15B) and bias the sensor 11900 during delivery of the sensor (FIG. 38C). By storing and shipping the sensor in the unbiased (relaxed or unstressed) position, the sensor can have increased shelf life and lower overall stress. Furthermore, by storing and shipping the sensor in the unbiased position, stress relaxation over shelf life can be reduced and therefore loss in bias force due to stress relaxation can be limited. Accordingly, bias forces during delivery of the sensor can more predictable and biasing during delivery can be as designed. The sharp 209114 can include a window 209114A. Prior to use, window 209114A can be aligned with protrusion 11912 on top end 11908b of the sensor 11900, and protrusion 11912 can extend through window 209114. In such a configuration, bottom end 11908a is not biased toward the sharp, and accordingly, sensor 11900 can be in a relaxed state. During firing, needle carrier 201102 can be partially retracted, thereby pulling sharp 209114 into a partially retracted position. Partial retraction can occur as the sheath 20704 initially moves proximally relative the sensor carrier 20710 during firing. Each sharp carrier lock arm 20710K of the sensor carrier 20710 (see FIG. 9D) can extend radially outward as the rib 20710M of the retention arm 20710L engages a respective slot 20704Q of sheath 20704 (see FIG. 8M) which can allow sharp carrier retention feature 20710L to clear the pre-partial retraction retention face 201102A and engage the post-partial retraction retention face 201102B of the sharp carrier 201102 (see FIG. 10C). In the partially retracted position, window 209114A no longer receives protrusion 11912, and sharp 209114 engages protrusion 11912 to thereby biases the bottom end 11908a toward the sharp 209114 and into a proper position for delivery, as described above.

Embodiments disclosed herein include:
D. A sensor control device that includes an electronics housing including a shell that defines a first aperture and a mount that defines a second aperture alignable with the first aperture when the shell is coupled to the mount, a seal overmolded onto the mount at the second aperture and comprising a first seal element overmolded onto a pedestal protruding from an inner surface of the mount, and a second seal element interconnected with the first seal element and overmolded onto a bottom of the mount, a sensor arranged within the electronics housing and having a tail extending through the second aperture and past the bottom of the mount, and a sharp that extends through the first and second apertures and past the bottom of the electronics housing.
E. An assembly that includes a sensor applicator, a sensor control device positioned within the sensor applicator and including an electronics housing including a shell that defines a first aperture and a mount that defines a second aperture alignable with the first aperture when the shell is mated to the mount, a seal overmolded onto the mount at the second aperture and comprising a first seal element overmolded onto a pedestal protruding from an inner surface of the mount, and a second seal element interconnected with the first seal element and overmolded onto a bottom of the mount, a sensor arranged within the electronics housing and having a tail extending through the second aperture and past the bottom of the mount, and a sharp that extends through the first and second apertures and past the bottom of the electronics housing. The assembly further including a sensor cap removably coupled to the sensor control device at the bottom of the mount and defining a sealed inner chamber that receives the tail and the sharp, and an applicator cap coupled to the sensor applicator.

Each of embodiments D and E may have one or more of the following additional elements in any combination: Element 1 : wherein the mount comprises a first injection molded part molded in a first shot, and the seal comprises a second injection molded part overmolded onto the first injection molded part in a second shot. Element 2: further comprising a sharp hub that carries the sharp and sealingly engages the first seal element, and a sensor cap removably coupled to the sharp hub at the bottom of the mount and sealingly engaging the second seal element, wherein the sensor cap defines an inner chamber that receives the tail and the sharp. Element 3 : wherein the sharp hub provides a mating member that extends past the bottom of the mount and the sensor cap is removably coupled to the mating member. Element 4: further comprising one or more pockets defined on the bottom of the mount at the second aperture, and one or more projections defined on an end of the sensor cap and receivable within the one or more pockets when the sensor cap is coupled to the sharp hub. Element 5: further comprising a collar positioned within the electronics housing and defining a central aperture that receives and sealingly engages the first seal element in a radial direction. Element 6: further comprising a channel defined on the inner surface of the mount and circumscribing the pedestal, an annular lip defined on an underside of the collar and matable with the channel, and an adhesive provided in the channel to secure and seal the collar to the mount at the channel. Element 7 : further comprising a groove defined through the annular lip to accommodate a portion of the sensor extending laterally within the mount, wherein the adhesive seals about the sensor at the groove. Element 8: further comprising a collar channel defined on an upper surface of the collar, an annular ridge defined on an inner surface of the shell and matable with the collar channel, and an adhesive provided in the collar channel to secure and seal the shell to the collar. Element 9: wherein one or both of the first and second seal elements define at least a portion of the second aperture. Element 10: wherein the first seal element extends at least partially through the first aperture when the shell is coupled to the mount.

Element 11 : wherein the sensor control device further includes a sharp hub that carries the sharp and sealingly engages the first seal element, and wherein the sensor cap is removably coupled to the sharp hub at the bottom of the mount and sealingly engages the second seal element. Element 12: wherein the sensor control device further includes one or more pockets defined on the bottom of the mount at the second aperture, and one or more projections defined on an end of the sensor cap and receivable within the one or more pockets when the sensor cap is coupled to the sharp hub. Element 13 : wherein the sensor control device further includes a collar positioned within the electronics housing and defining a central aperture that receives and sealingly engages the first seal element in a radial direction. Element 14: wherein the sensor control device further includes a channel defined on the inner surface of the mount and circumscribing the pedestal, an annular lip defined on an underside of the collar and matable with the channel, and an adhesive provided in the channel to secure and seal the collar to the mount at the channel. Element 15: wherein the sensor control device further includes a groove defined through the annular lip to accommodate a portion of the sensor extending laterally within the mount, and wherein the adhesive seals about the sensor at the groove. Element 16: wherein the sensor control device further includes a collar channel defined on an upper surface of the collar, an annular ridge defined on an inner surface of the shell and matable with the collar channel, and an adhesive provided in the collar channel to secure and seal the shell to the collar. Element 17: wherein one or both of the first and second seal elements define at least a portion of the second aperture. Element 18: wherein the first seal element extends at least partially through the first aperture.

By way of non-limiting example, exemplary combinations applicable to D and E include: Element 2 with Element 3; Element 2 with Element 4; Element 5 with Element 6; Element 6 with Element 7; Element 5 with Element 8; Element 11 with Element 12; Element 13 with Element 14; Element 14 with Element 15; and Element 13 with Element 16.

### Exemplary Firing Mechanism of One-Piece and Two-Piece Applicators

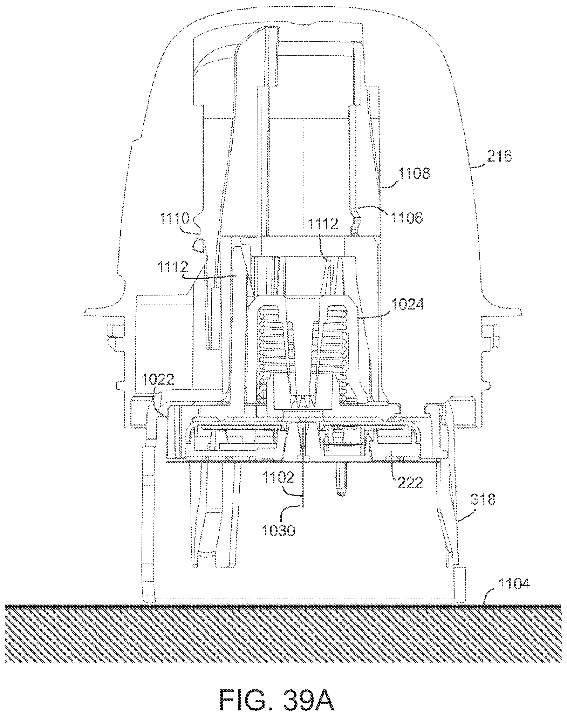

FIGS. 39A-39F illustrate example details of embodiments of the internal device mechanics of "firing" the applicator 216 to apply sensor control device 222 to a user and including retracting sharp 1030 safely back into used applicator 216. All together, these drawings represent an example sequence of driving sharp 1030 (supporting a sensor coupled to sensor control device 222) into the skin of a user, withdrawing the sharp while leaving the sensor behind in operative contact with interstitial fluid of the user, and adhering the sensor control device to the skin of the user with an adhesive. Modification of such activity for use with the alternative applicator assembly embodiments and components can be appreciated in reference to the same by those with skill in the art. Moreover, applicator 216 may be a sensor applicator having one-piece architecture or a two-piece architecture as disclosed herein.

Turning now to FIG. 39A, a sensor 1102 is supported within sharp 1030, just above the skin 1104 of the user. Rails 1106 (optionally three of them) of an upper guide section 1108 may be provided to control applicator 216 motion relative to sheath 318. The sheath 318 is held by detent features 1110 within the applicator 216 such that appropriate downward force along the longitudinal axis of the applicator 216 will cause the resistance provided by the detent features 1110 to be overcome so that sharp 1030 and sensor control device 222 can translate along the longitudinal axis into (and onto) skin 1104 of the user. In addition, catch arms 1112 of sensor carrier 1022 engage the sharp retraction assembly 1024 to maintain the sharp 1030 in a position relative to the sensor control device 222.

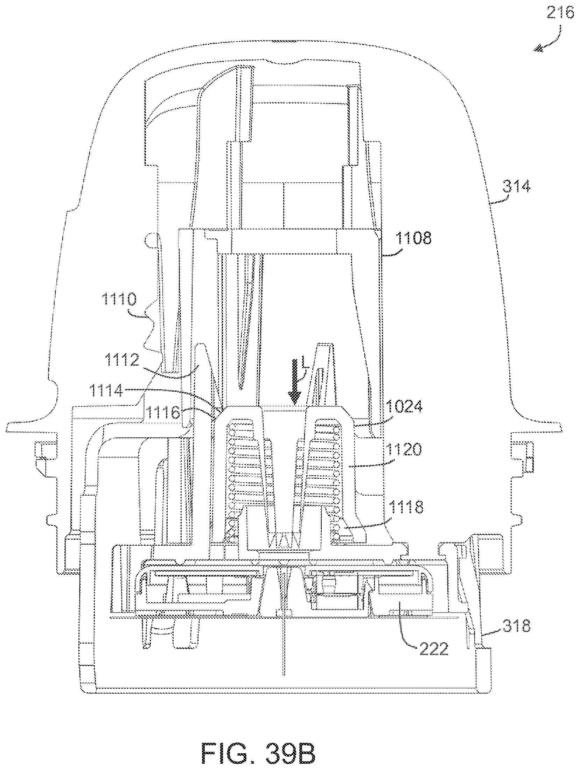

In FIG. 39B, user force is applied to overcome or override detent features 1110 and sheath 318 collapses into housing 314 driving the sensor control device 222 (with associated parts) to translate down as indicated by the arrow L along the longitudinal axis. An inner diameter of the upper guide section 1108 of the sheath 318 constrains the position of carrier arms 1112 through the full stroke of the sensor/sharp insertion process. The retention of the stop surfaces 1114 of carrier arms 1112 against the complimentary faces 1116 of the sharp retraction assembly 1024 maintains the position of the members with return spring 1118 fully energized.

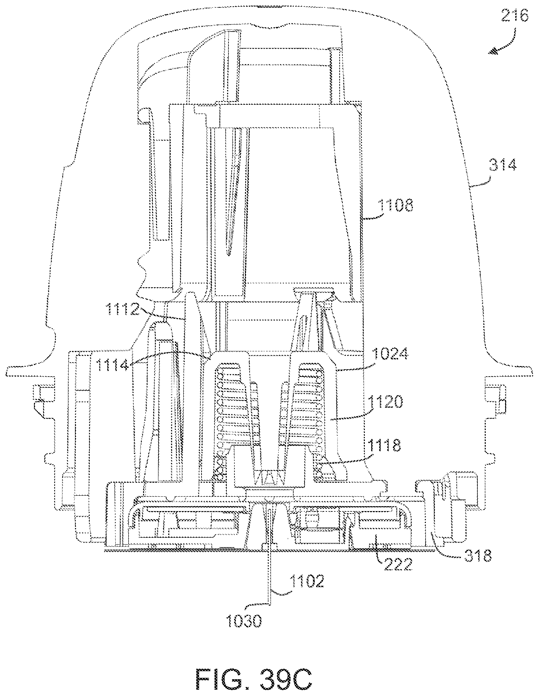

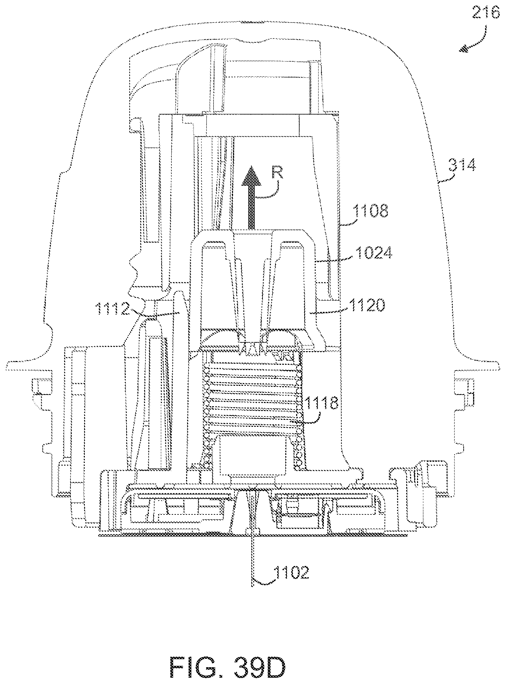

In FIG. 39C, sensor 1102 and sharp 1030 have reached full insertion depth. In so doing, the carrier arms 1112 clear the upper guide section 1108 inner diameter. Then, the compressed force of the coil return spring 1118 drives angled stop surfaces 1114 radially outward, releasing force to drive the sharp carrier 1102 of the sharp retraction assembly 1024 to pull the (slotted or otherwise configured) sharp 1030 out of the user and off of the sensor 1102 as indicated by the arrow R in FIG. 39D.

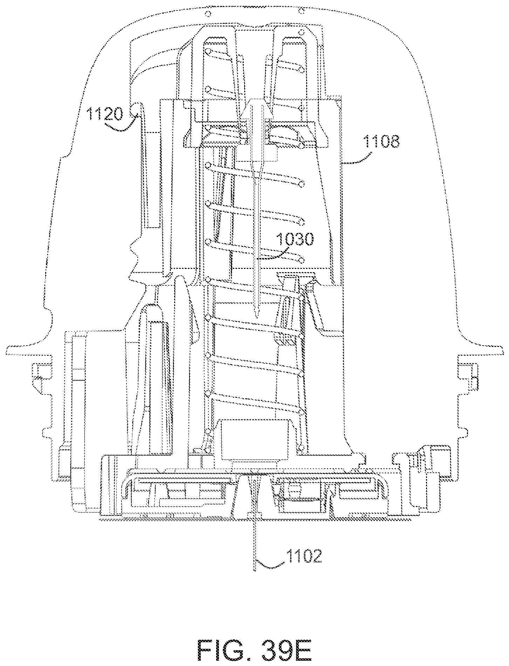

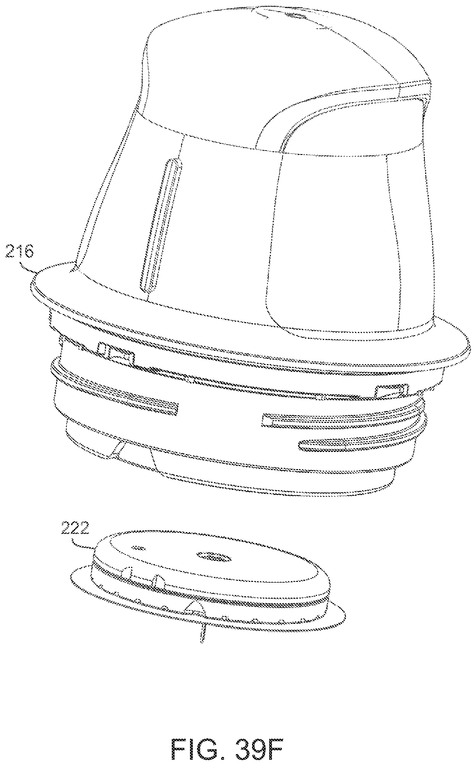

With the sharp 1030 fully retracted as shown in FIG. 39E, the upper guide section 1108 of the sheath 318 is set with a final locking feature 1120. As shown in FIG. 39F, the spent applicator assembly 216 is removed from the insertion site, leaving behind the sensor control device 222, and with the sharp 1030 secured safely inside the applicator assembly 216. The spent applicator assembly 216 is now ready for disposal.

Operation of the applicator 216 when applying the sensor control device 222 is designed to provide the user with a sensation that both the insertion and retraction of the sharp 1030 is performed automatically by the internal mechanisms of the applicator 216. In other words, the present invention avoids the user experiencing the sensation that he is manually driving the sharp 1030 into his skin. Thus, once the user applies sufficient force to overcome the resistance from the detent features of the applicator 216, the resulting actions of the applicator 216 are perceived to be an automated response to the applicator being "triggered." The user does not perceive that he is supplying additional force to drive the sharp 1030 to pierce his skin despite that all the driving force is provided by the user and no additional biasing/driving means are used to insert the sharp 1030. As detailed above in FIG. 39C, the retraction of the sharp 1030 is automated by the coil return spring 1118 of the applicator 216.

With respect to any of the applicator embodiments described herein, as well as any of the components thereof, including but not limited to the sharp, sharp module and sensor module embodiments, those of skill in the art will understand that said embodiments can be dimensioned and configured for use with sensors configured to sense an analyte level in a bodily fluid in the epidermis, dermis, or subcutaneous tissue of a subject. In some embodiments, for example, sharps and distal portions of analyte sensors disclosed herein can both be dimensioned and configured to be positioned at a particular end-depth (i.e., the furthest point of penetration in a tissue or layer of the subject's body, e.g., in the epidermis, dermis, or subcutaneous tissue). With respect to some applicator embodiments, those of skill in the art will appreciate that certain embodiments of sharps can be dimensioned and configured to be positioned at a different end-depth in the subject's body relative to the final end-depth of the analyte sensor. In some embodiments, for example, a sharp can be positioned at a first end-depth in the subject's epidermis prior to retraction, while a distal portion of an analyte sensor can be positioned at a second end-depth in the subject's dermis. In other embodiments, a sharp can be positioned at a first end-depth in the subject's dermis prior to retraction, while a distal portion of an analyte sensor can be positioned at a second end-depth in the subject's subcutaneous tissue. In still other embodiments, a sharp can be positioned at a first end-depth prior to retraction and the analyte sensor can be positioned at a second end-depth, wherein the first end-depth and second end-depths are both in the same layer or tissue of the subject's body.

Additionally, with respect to any of the applicator embodiments described herein, those of skill in the art will understand that an analyte sensor, as well as one or more structural components coupled thereto, including but not limited to one or more spring-mechanisms, can be disposed within the applicator in an off-center position relative to one or more axes of the applicator. In some applicator embodiments, for example, an analyte sensor and a spring mechanism can be disposed in a first off-center position relative to an axis of the applicator on a first side of the applicator, and the sensor electronics can be disposed in a second off-center position relative to the axis of the applicator on a second side of the applicator. In other applicator embodiments, the analyte sensor, spring mechanism, and sensor electronics can be disposed in an off-center position relative to an axis of the applicator on the same side. Those of skill in the art will appreciate that other permutations and configurations in which any or all of the analyte sensor, spring mechanism, sensor electronics, and other components of the applicator are disposed in a centered or off-centered position relative to one or more axes of the applicator are possible and fully within the scope of the present disclosure.

A number of deflectable structures are described herein, including but not limited to deflectable detent snaps 1402, deflectable locking arms 1412, sharp carrier lock arms 1524, sharp retention arms 1618, and module snaps 2202. These deflectable structures are composed of a resilient material such as plastic or metal (or others) and operate in a manner well known to those of ordinary skill in the art. The deflectable structures each has a resting state or position that the resilient material is biased towards. If a force is applied that causes the structure to deflect or move from this resting state or position, then the bias of the resilient material will cause the structure to return to the resting state or position once the force is removed (or lessened). In many instances these structures are configured as arms with detents, or snaps, but other structures or configurations can be used that retain the same characteristics of deflectability and ability to return to a resting position, including but not limited to a leg, a clip, a catch, an abutment on a deflectable member, and the like.

Additional details of suitable devices, systems, methods, components and the operation thereof along with related features are set forth in International Publication No. WO2018/136898 to Rao et. al., International Publication No. WO2019/236850 to Thomas et. al., International Publication No. WO2019/236859 to Thomas et. al., International Publication No. WO2019/236876 to Thomas et. al., and U.S. Patent Publication No. 2020/0196919, filed June 6, 2019. Further details regarding embodiments of applicators, their components, and variants thereof, are described in U.S. Patent Publication Nos. 2013/0150691, 2016/0331283, and 2018/0235520. Further details regarding embodiments of sharp modules, sharps, their components, and variants thereof, are described in U.S. Patent Publication No. 2014/0171771, which is incorporated by reference herein in its entirety and for all purposes.

In summary, an analyte measurement device including an electronics housing including a shell having an upper surface with a first aperture defined therein; a mount mated to the shell and having an underside with a second aperture defined therein aligned with the first aperture; a collar disposed between the shell and the mount and including: a third aperture aligned with the first aperture and the second aperture, and a plurality of tabs on an outer periphery of the collar; a circuit board disposed within the electronics housing and including a plurality of electronic modules, wherein the circuit board is mounted within the electronics housing on the plurality of tabs on the outer periphery; and an analyte sensor including a tail portion extending through the first aperture and the second aperture and configured to measure an analyte level and a flag portion including a plurality of electrical contacts coupled with the circuit board, and a neck portion interconnecting the tail portion and the flag portion. An assembly for delivery of an analyte sensor is also disclosed.

It should be noted that all features, elements, components, functions, and steps described with respect to any embodiment provided herein are intended to be freely combinable and substitutable with those from any other embodiment. If a certain feature, element, component, function, or step is described with respect to only one embodiment, then it should be understood that that feature, element, component, function, or step can be used with every other embodiment described herein unless explicitly stated otherwise. This paragraph therefore serves as antecedent basis and written support for the introduction of claims, at any time, that combine features, elements, components, functions, and steps from different embodiments, or that substitute features, elements, components, functions, and steps from one embodiment with those of another, even if the following description does not explicitly state, in a particular instance, that such combinations or substitutions are possible. Thus, the foregoing description of specific embodiments of the disclosed subject matter has been presented for purposes of illustration and description.

## Claims

1. An analyte measurement device, comprising:
an electronics housing including:
a shell (9106) having an upper surface with a first aperture defined therein;
a mount (9108) mated to the shell (9106) and having an underside with a second aperture defined therein aligned with the first aperture;
a collar (9212) disposed between the shell (9106) and the mount and including:
a third aperture aligned with the first aperture and the second aperture, and
a plurality of tabs (9212a) on an outer periphery of the collar (9212);
a circuit board (4000) disposed within the electronics housing and including a plurality of electronic modules, wherein the circuit board is mounted within the electronics housing on the plurality of tabs on the outer periphery of the collar,
wherein the circuit board includes a first portion (4000b) foldable over a second portion (4000a), the second portion mounted on the mount and the first portion mounted on the plurality of tabs on the outer periphery of the collar; and
an analyte sensor (9112) including a tail portion (9224) extending through an aperture in the mount and configured to measure an analyte level, a flag portion including a plurality of electrical contacts coupled with the circuit board, and a neck portion interconnecting the tail portion and the flag portion.

2. The device of claim 1, wherein the plurality of tabs (9212a) includes three tabs.

3. The device of claim 1, wherein the collar (9212) further includes:
a collar channel (9220) on an upper surface mated with an annular ridge (9222) defined on an inner surface of the shell (9106),
an annular lip (9216) on a bottom surface received within a channel (9206) defined on an inner surface of the mount, and
a seal (9210a) on the upper surface of the collar (9212).

4. The device of claim 3, wherein the seal (9210a) comprises an elastomeric seal.

5. The device of claim 3, further comprising a sharp hub (9116) including a sharp (9114) extending through the first, second, and third apertures and a mating surface (9116a) mated with the seal (9210a).

6. The device of claim 3, wherein the mating surface (9116a) includes a ledge.

7. The device of claim 1, wherein the circuit board (4000) further includes a battery.

8. An assembly for delivery of an analyte sensor comprising:
an analyte measurement device according to claim 1, the analyte measurement device including:
a sensor cap (9120) having a first end (9122a), a second end (9122b), and an inner chamber (9124), wherein the inner chamber receives the tail portion (9224) of the analyte sensor and wherein the first end is removably coupled to the mount (9108) to seal the inner chamber; and
an applicator (9502) for delivery of the analyte sensor including:
a housing (9504) including a sensor carrier configured to secure the analyte measurement device within an interior of the applicator;
an applicator cap (9506) removably coupled to the housing to seal the interior of the applicator and including:
a desiccant (20502) including a plurality of retention snaps (20502A),
wherein the plurality of retention snaps is disposed on the desiccant,
one or more retention clips (20708H) engaged to the plurality of retention snaps to retain the desiccant in the cap (9506) and to limit rotation of the desiccant, and
a cap post (9510) engaged with an engagement feature (9126) on the second end of the sensor cap.

9. The assembly of claim 8, wherein the desiccant (20502) includes an internal bore (20502B) and the engagement feature (9126) on the second end (9122b) of the sensor cap (9120) passes through the internal bore of desiccant and engages the applicator cap (9506).

10. The assembly of claim 8, wherein the desiccant (20502) is substantially cylindrical in shape.

11. The assembly of claim 8, wherein the first end (9122a) is removably coupled to the mount (9108) to seal the inner chamber (9124).

12. The assembly of claim 8, wherein the analyte measurement device further comprises an elastomeric plug (9130A) arranged at the second end (9122b) of the sensor cap (9120).

13. The assembly of claim 12, wherein the elastomeric plug (9130A) includes a seal bead (9130Ab) in an interference fit arrangement with the sensor cap (9120).

14. The assembly of claim 12, wherein the elastomeric plug (9130A) defines the engagement feature (9126) of the sensor cap (9120).

15. The assembly of claim 8, wherein the applicator (9502) for delivery of the analyte sensor (9112) further includes:
a sharp carrier coupled to a sharp (9114) extending through the aperture in the mount (9108), the sharp configured to penetrate a user's skin to position the analyte sensor into contact with bodily fluid;
a sensor carrier to releasably hold the analyte measurement device within the interior space; and
a spring (1118) biasing the sharp carrier to move upward within the housing (9504).

16. The assembly of claim 15, wherein the spring (1118) includes a spring having a spring constant of about 0.12 N/m.

17. The assembly of claim 15, wherein the spring (1118) includes a spring having a wire diameter of about 0.65 millimeters, an inner diameter of about 9.6 millimeter, and an outer diameter of about 11.1 millimeters.

## Patentansprüche

1. Analytmessvorrichtung, umfassend:
ein Elektronikgehäuse, beinhaltend:
eine Schale (9106) mit einer oberen Fläche mit einer darin definierten ersten Öffnung;
eine Halterung (9108), die mit der Schale (9106) zusammenpasst und eine Unterseite mit einer darin definierten zweiten Öffnung, die auf die erste Öffnung ausgerichtet ist, aufweist;
einen Kragen (9212), die zwischen der Schale (9106) und der Halterung angeordnet ist und beinhaltet:
eine dritte Öffnung, die auf die erste Öffnung und die zweite Öffnung ausgerichtet ist; und
eine Vielzahl von Laschen (9212a) auf einem Außenumfang des Kragens (9212);
eine Leiterplatte (4000), die innerhalb des Elektronikgehäuses angeordnet ist und eine Vielzahl von Elektronikmodulen beinhaltet, wobei die Leiterplatte innerhalb des Elektronikgehäuses auf der Vielzahl von Laschen auf dem Außenumfang des Kragens montiert ist,
wobei die Leiterplatte einen ersten Abschnitt (4000b) beinhaltet, der über einen zweiten Abschnitt (4000a) klappbar ist, wobei der zweite Abschnitt auf der Halterung montiert ist und der erste Abschnitt auf der Vielzahl von Laschen auf dem Außenumfang des Kragens montiert ist; und
einen Analytsensor (9112), beinhaltend einen Ausläuferabschnitt (9224), der sich durch eine Öffnung in der Halterung erstreckt und dazu konfiguriert ist, eine Analytkonzentration zu messen, einen Fahnenabschnitt, beinhaltend eine Vielzahl von elektrischen Kontakten, die mit der Leiterplatte gekoppelt sind, und einen Halsabschnitt, der den Ausläuferabschnitt und den Fahnenabschnitt miteinander verbindet.

2. Vorrichtung nach Anspruch 1, wobei die Vielzahl von Laschen (9212a) drei Laschen beinhaltet.

3. Vorrichtung nach Anspruch 1, wobei der Kragen (9212) weiterhin beinhaltet:
einen Kragenkanal (9220) auf einer oberen Fläche, der mit einem ringförmigen Grat (9222) zusammenpasst, deer auf einer inneren Fläche der Schale (9106) definiert ist,
eine ringförmige Lippe (9216) auf einer unteren Fläche, die innerhalb eines Kanals (9206) aufgenommen wird, der auf einer inneren Fläche der Halterung definiert ist, und
eine Dichtung (9210a) auf der oberen Fläche des Kragens (9212).

4. Vorrichtung nach Anspruch 3, wobei die Dichtung (9210a) eine elastomere Dichtung umfasst.

5. Vorrichtung nach Anspruch 3, weiterhin umfassend einen Nadelverteiler (9116), beinhaltend eine Nadel (9114), die sich durch die erste, die zweite und die dritte Öffnung erstreckt, und eine zusammenpassende Fläche (9116a), die mit der Dichtung (9210a) zusammenpasst.

6. Vorrichtung nach Anspruch 3, wobei die zusammenpassende Fläche (9116a) einen Absatz beinhaltet.

7. Vorrichtung nach Anspruch 1, wobei die Leiterplatte (4000) weiterhin eine Batterie beinhaltet.

8. Baugruppe zur Abgabe eines Analytsensors, umfassend:
eine Analytmessvorrichtung nach Anspruch 1, wobei die Analytmessvorrichtung beinhaltet:
eine Sensorkappe (9120) mit einem ersten Ende (9122a), einem zweiten Ende (9122b) und einer inneren Kammer (9124), wobei die innere Kammer den Ausläuferabschnitt (9224) des Analytsensors aufnimmt und wobei das erste Ende abnehmbar an die Halterung (9108) gekoppelt ist, um die innere Kammer abzudichten; und
einen Applikator (9502) zur Abgabe des Analytsensors, beinhaltend:
ein Gehäuse (9504), beinhaltend einen Sensorträger, der dazu konfiguriert ist, die Analytmessvorrichtung innerhalb eines Inneren des Applikators zu sichern;
eine Applikatorkappe (9506), die abnehmbar an das Gehäuse gekoppelt ist, um das Innere des Applikators abzudichten, und beinhaltet:
einen Luftentfeuchter (20502), beinhaltend eine Vielzahl von Rückhalteschnappem (20502A), wobei die Vielzahl von Rückhalteschnappern auf dem Luftentfeuchter angeordnet ist,
einen oder mehrere Rückhalteklammern (20708H), die mit der Vielzahl von Rückhalteschnappern im Eingriff stehen, um den Luftentfeuchter in der Kappe (9506) zurückzuhalten und eine Drehung des Luftentfeuchters einzuschränken, und
einen Kappenstift (9510), der mit einem Eingriffsmerkmal (9126) auf dem zweiten Ende der Sensorkappe im Eingriff steht.

9. Baugruppe nach Anspruch 8, wobei der Luftentfeuchter (20502) eine Innenbohrung (20502B) beinhaltet und das Eingriffsmerkmal (9126) auf dem zweiten Ende (9122b) der Sensorkappe (9120) durch die Luftentfeuchter-Innenbohrung verläuft und die Applikatorkappe (9506) in Eingriff nimmt.

10. Baugruppe nach Anspruch 8, wobei der Luftentfeuchter (20502) eine im Wesentlichen zylindrische Form aufweist.

11. Baugruppe nach Anspruch 8, wobei das erste Ende (9122a) abnehmbar an die Halterung (9108) gekoppelt ist, um die innere Kammer (9124) abzudichten.

12. Baugruppe nach Anspruch 8, wobei die Analytmessvorrichtung weiterhin einen elastomeren Stopfen (9130A) umfasst, der an dem zweiten Ende (9122b) der Sensorkappe (9120) eingerichtet ist.

13. Baugruppe nach Anspruch 12, wobei der elastomere Stopfen (9130A) einen Dichtungsraupe (9130Ab) in einer Presspassungsanordnung mit der Sensorkappe (9120) beinhaltet.

14. Baugruppe nach Anspruch 12, wobei der elastomere Stopfen (9130A) das Eingriffsmerkmal (9126) der Sensorkappe (9120) definiert.

15. Baugruppe nach Anspruch 8, wobei der Applikator (9502) zur Abgabe des Analytsensors (9112) weiterhin beinhaltet:
einen Nadelträger, der an eine Nadel (9114) gekoppelt ist, die sich durch die Öffnung in der Halterung (9108) erstreckt, wobei die Nadel dazu konfiguriert ist, eine Haut eines Benutzers zu penetrieren, um den Analytsensor in Kontakt mit Körperflüssigkeit zu positionieren;
einen Sensorträger, um die Analytmessvorrichtung lösbar innerhalb des Innenraums zu halten; und
eine Feder (1118), die den Nadelträger vorspannt, um sich innerhalb des Gehäuses (9504) nach oben zu bewegen.

16. Baugruppe nach Anspruch 15, wobei die Feder (1118) eine Feder mit einer Federkonstante von etwa 0,12 N/m beinhaltet.

17. Baugruppe nach Anspruch 15, wobei die Feder (1118) eine Feder mit einem Drahtdurchmesser von etwa 0,65 Millimeter, einem Innendurchmesser von etwa 9,6 Millimeter und einem Außendurchmesser von etwa 11,1 Millimeter beinhaltet.

## Revendications

1. Dispositif de mesure d'analyte, comprenant:
un boîtier électronique comprenant:
une coque (9106) comportant une surface supérieure dans laquelle est délimitée une première ouverture;
un support (9108) accouplé à la coque (9106) et comportant un dessous dans lequel est délimitée une deuxième ouverture alignée avec la première ouverture;
un collier (9212) disposé entre la coque (9106) et le support et comprenant:
une troisième ouverture alignée avec la première ouverture et la deuxième ouverture, et
une pluralité de languettes (9212a) sur une périphérie extérieure du collier (9212);
une carte de circuit imprimé (4000) disposée à l'intérieur du boîtier électronique et comprenant une pluralité de modules électroniques, la carte de circuit imprimé étant montée à l'intérieur du boîtier électronique sur la pluralité de languettes sur la périphérie extérieure du collier,
dans lequel la carte de circuit imprimé comprend une première partie (4000b) qui peut être repliée par-dessus une deuxième partie (4000a), la deuxième partie étant montée sur le support et la première partie étant montée sur la pluralité de languettes sur la périphérie extérieure du collier; et
un capteur d'analyte (9112) comprenant une partie queue (9224) s'étendant à travers une ouverture dans le support et configurée pour mesurer un niveau d'analyte, une partie plane comprenant une pluralité de contacts électriques accouplés à la carte de circuit imprimé, et une partie col raccordant la partie queue et la partie plane.

2. Dispositif selon la revendication 1, dans lequel la pluralité de languettes (9212a) comprend trois languettes.

3. Dispositif selon la revendication 1, dans lequel le collier (9212) comprend en outre:
un canal de collier (9220) sur une surface supérieure accouplé à une crête annulaire (9222) délimitée sur une surface intérieure de la coque (9106),
une lèvre annulaire (9216) sur une surface inférieure logée à l'intérieur d'un canal (9206) délimité sur une surface intérieure du support, et
un joint d'étanchéité (9210a) sur la surface supérieure du collier (9212).

4. Dispositif selon la revendication 3, dans lequel le joint d'étanchéité (9210a) comprend un joint d'étanchéité élastomère.

5. Dispositif selon la revendication 3, comprenant en outre une embase de pointe (9116) comprenant une pointe (9114) s'étendant à travers les première, deuxième et troisième ouvertures et une surface de contact (9116a) accouplée avec le joint d'étanchéité (9210a).

6. Dispositif selon la revendication 3, dans lequel la surface de contact (9116a) comprend un rebord.

7. Dispositif selon la revendication 1, dans lequel la carte de circuit imprimé (4000) comprend en outre une pile.

8. Ensemble d'application d'un capteur d'analyte comprenant:
un dispositif de mesure d'analyte selon la revendication 1, le dispositif de mesure d'analyte comprenant:
un capuchon de capteur (9120) comportant une première extrémité (9122a), une deuxième extrémité (9122b) et une chambre intérieure (9124), la chambre intérieure recevant la partie queue (9224) du capteur d'analyte et la première extrémité étant accouplée de manière amovible au support (9108) pour une fermeture hermétique de la chambre intérieure; et
un applicateur (9502) pour l'application du capteur d'analyte comprenant:
un boîtier (9504) comprenant un support de capteur configuré pour fixer le dispositif de mesure d'analyte dans un intérieur de l'applicateur;
un capuchon d'applicateur (9506) accouplé de manière amovible au boîtier pour une fermeture hermétique de l'intérieur de l'applicateur et comprenant:
un déshydratant (20502) comprenant une pluralité d'éléments de rétention à pression (20502A), la pluralité d'éléments de rétention à pression étant disposée sur le déshydratant,
une ou plusieurs fermetures de rétention (20708H) fixées à la pluralité d'éléments de rétention à pression pour retenir le déshydratant dans le capuchon (9506) et pour limiter la rotation du déshydratant, et
un montant de capuchon (9510) coopérant avec un élément d'engagement (9126) sur la deuxième extrémité du capuchon de capteur.

9. Ensemble selon la revendication 8, dans lequel le déshydratant (20502) comprend un alésage interne (20502B) et l'élément d'engagement (9126) sur la deuxième extrémité (9122b) du capuchon de capteur (9120) passe dans l'alésage interne du déshydratant et s'engage dans le capuchon d'applicateur (9506).

10. Ensemble selon la revendication 8, dans lequel le déshydratant (20502) est de forme sensiblement cylindrique.

11. Ensemble selon la revendication 8, dans lequel la première extrémité (9122a) est accouplée de manière amovible au support (9108) pour une fermeture hermétique de la chambre intérieure (9124).

12. Ensemble selon la revendication 8, dans lequel le dispositif de mesure d'analyte comprend en outre un bouchon élastomère (9130A) disposé à la deuxième extrémité (9122b) du capuchon de capteur (9120).

13. Ensemble selon la revendication 12, dans lequel le bouchon élastomère (9130A) comprend un cordon d'étanchéité (9130Ab) dans une configuration d'ajustement serré avec le capuchon de capteur (9120).

14. Ensemble selon la revendication 12, dans lequel le bouchon élastomère (9130A) délimite l'élément d'engagement (9126) du capuchon de capteur (9120).

15. Ensemble selon la revendication 8, dans lequel l'applicateur (9502) pour l'application du capteur d'analyte (9112) comprend en outre:
un porte-pointe accouplé à une pointe (9114) s'étendant à travers l'ouverture dans le support (9108), la pointe étant configurée pour pénétrer dans la peau d'un utilisateur afin de positionner le capteur d'analyte en contact avec un fluide corporel;
un support de capteur conçu pour maintenir de manière amovible le dispositif de mesure d'analyte dans l'espace intérieur; et
un ressort (1118) sollicitant le porte-pointe pour qu'il se déplace vers le haut à l'intérieur du boîtier (9504).

16. Ensemble selon la revendication 15, dans lequel le ressort (1118) comprend un ressort ayant une constante de rappel d'environ 0,12 N/m.

17. Ensemble selon la revendication 15, dans lequel le ressort (1118) comprend un ressort ayant un diamètre de fil d'environ 0,65 millimètre, un diamètre intérieur d'environ 9,6 millimètres, et un diamètre extérieur d'environ 11,1 millimètres.
